# EUROPEAN PATENT APPLICATION

(11) **EP 4 497 750 A1**
(43) Date of publication of application: **29.01.2025**
(21) Application number: 23775330.6
(22) Date of filing: 23.03.2023
(51) Int. Cl.: C07D 401/14, C07D 495/04, C07D 491/048, C07D 471/04, C07D 405/14, C07D 495/14, C07D 401/04, H10K 85/60

(54) **ORGANIC COMPOUND AND ORGANIC ELECTROLUMINESCENT ELEMENT USING SAME**

(30) Priority: 23.03.2022 KR 20220036267
(71) Applicant: Solus Advanced Materials Co., Ltd., Iksan-si, Jeollabuk-do 54584 (KR)
(72) Inventor: LEE, Uigeon, Yongin-si, Gyeonggi-do 16858 (KR); SON, Hyosuk, Yongin-si, Gyeonggi-do 16858 (KR); PARK, Woojae, Yongin-si, Gyeonggi-do 16858 (KR); JUNG, Donggi, Yongin-si, Gyeonggi-do 16858 (KR); SIM, Jaeyi, Yongin-si, Gyeonggi-do 16858 (KR)
(74) Representative: Office Freylinger
(86) International application number: PCT/KR2023/003875
(87) International publication number: WO 2023/182835

(57) **Abstract**

The present invention relates to a novel organic compound and an organic electroluminescent element using same, and more specifically, to a compound having excellent electron injection and transport capabilities, and an organic electroluminescent element that comprises same in at least one organic layer, and thus is improved in terms of progressive driving voltage, as well as properties such as luminous efficiency, driving voltage, lifespan, and the like.

## Description

### Technical Field

The present disclosure relates to a novel organic compound and an organic electroluminescent device using same. More specifically, the present disclosure relates to a compound with excellent electron transport capability and an organic electroluminescent device that includes the compound in one or more organic layers, thereby exhibiting an improvement in characteristics such as luminous efficiency, driving voltage, and lifespan.

### Background Art

In an organic electroluminescent device (hereinafter referred to as "organic EL device"), when a voltage is applied to two electrodes, holes and electrons are injected from the anode and cathode, respectively, into an organic layer where the injected holes and electrons meet to form an exciton. The exciton falls to the ground state, emitting light. In this regard, the materials used for the organic layer can be classified according to their functions into emission materials, hole injection materials, hole transport materials, electron transport materials, and electron injection materials.

The light-emitting layer materials of organic EL devices can be categorized into blue, green, and red emitting materials based on the emission color. Additionally, yellow and orange emitting materials are used to achieve better natural color implementation. To increase color purity and luminous efficiency through energy transfer, host/dopant systems may be employed as an emitting material. Dopant materials can be divided into fluorescent dopants, which use organic substances, and phosphorescent dopants, which use metal complex compounds bearing heavy atoms such as Ir and Pt. The development of these phosphorescent materials can theoretically improve luminous efficiency up to four times compared to fluorescence, drawing attention to both phosphorescent dopants and host materials.

To date, NPB, BCP, and Alq3 are widely known for use in hole injection layers, hole transport layers, hole blocking layers, and electron transport layers. Anthracene derivatives have been reported as fluorescent dopant/host materials for emitting materials. Among luminous materials, phosphorescent materials, which have significant advantages in efficiency improvement, include Ir-bearing metal complex compounds such as Firpic, Ir(ppy)₃, and (acac)Ir(btp)₂, used as blue, green, and red dopant materials. Currently, CBP shows excellent characteristics as a phosphorescent host material.

However, existing materials, while advantageous in terms of luminous properties, have low glass transition temperatures and poor thermal stability, leading to unsatisfactory lifespan levels in organic EL devices.

### Disclosure of Invention

### Technical Problem

The present disclosure aims to provide a novel organic compound with excellent electron injection and transport capabilities, electrochemical stability, and thermal stability, which can be used as an organic layer material in organic EL devices, specifically as an electron transport layer material or an N-type charge generation layer material.

Additionally, the present disclosure is to provide an organic EL device that includes the aforementioned novel organic compound and exhibits low driving voltage, high luminous efficiency, and improved lifespan.

### Solution to Problem

To achieve the goals, the present disclosure provides an organic compound represented by the following Chemical Formula 1: 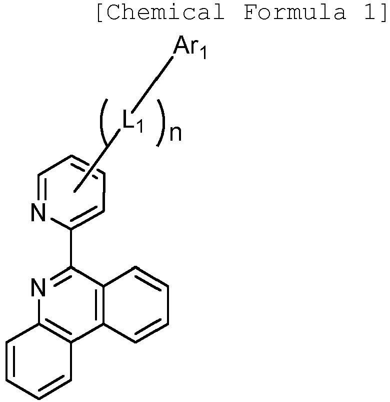 (wherein,
n is an integer of 0 to 3,
L₁ is a direct bond (single bond) or is selected from the group consisting of an arylene of C₆-C₆₀ and a heteroarylene of 5 to 60 nuclear atoms,
Ar₁ is selected from the group consisting of a hydrogen atom, a deuterium atom (D), a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, an arylamine of C₆-C₆₀, and an aryl of C₆-C₆₀ substituted with an alkenyl of C₂-C₄₀, or bind to an adjacent group to form a fused ring,
the arylene and heteroarylene groups of L₁, and the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, arylphosphine oxide, arylamine, alkenyl-substituted aryl groups, and fused ring of Ar₁ each being unsubstituted or substituted with at least one substituent selected from the group consisting of a deuterium atom, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₁-C₄₀, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀, wherein two or more substituents, if present, are same or different).

Also, the present disclosure provides an organic EL device including: an anode; a cathode; and one or more organic layers interposed between the anode and the cathode, wherein at least one of the one or more organic layers includes the organic compound described above. In this regard, the organic layer including the compound may be an electron transport layer.

Furthermore, the present disclosure provides an organic EL device including: an anode and a cathode spaced apart from each other; a plurality of lighting units interposed between the anode and the cathode; and an N-type charge generation layer and a P-type charge generation layer interposed between adjacent light units, wherein each light unit includes a hole transport layer, a light-emitting layer, and an electron transport layer, and the N-type charge generation includes the compound described above.

### Advantageous Effects of Invention

With excellent electron transport ability, luminescent ability, electrochemical stability, and thermal stability, the compound of the present disclosure can be used as a material for an organic layer in organic EL devices. Particularly, when used as material for at least one of an electron transport layer, an electron transport auxiliary layer, and an N-type charge generation layer, the compound of the present disclosure allows for the fabrication of organic EL with superior luminous performance, low driving voltage, high efficiency, and long lifespan compared to conventional materials, and makes it possible to manufacture full-color display panels with improved performance and lifespan.

### Brief Description of Drawings

FIG. 1 is a schematic cross-sectional view of an organic EL device according to the first embodiment of the present disclosure.
FIG. 2 is a schematic cross-sectional view of an organic EL device according to the second embodiment of the present disclosure.
FIG. 3 is a schematic cross-sectional view of an organic EL device according to the third embodiment of the present disclosure.
FIG. 4 is a schematic cross-sectional view of an organic EL device according to the fourth embodiment of the present disclosure.

### ** Description of reference numbers **

100: anode, 200: cathode,
300: organic layer, 310: hole injection layer,
320: hole transport layer, 330: light-emitting layer,
340: electron transport layer, 350: electron injection layer,
360: electron transport auxiliary layer, 400: 1^{st} lighting unit,
410: 1^{st} hole transport layer, 420: 1^{st} 1light-emitting layer,
430: 1^{st} electron transport layer, 440: hole injection layer,
500: 2^{nd} lighting unit, 510: 2^{nd} hole transport layer,
520: 2^{nd} light-emitting layer, 530: 2^{nd} electron transport,
600: charge generation layer, 610: N-type charge generation layer,
620: P-type charge generation layer

### Best Mode for Carrying out the Invention

Below, a detailed description will be given of the present disclosure.

### <Novel Compound>

The compound according to the present disclosure has the basic structure in which a phenanthridine moiety has a pyridine moiety bonded to the carbon at position 5 thereof, with various substituents (particularly, EWG), such as aryl, heteroaryl, etc., linked to the pyridine moiety, as illustrated in Chemical Formula 1. With excellent electron injection and transport capabilities, electrochemical stability, and thermal stability, the novel compound of Chemical Formula 1 according to the present disclosure can be used as a material for an electron transport layer, an electron transport auxiliary layer, or an N-type charge generation layer in organic EL devices, whereby an improvement can be brought about in luminous efficiency, lifespan, driving voltage, and progressive driving voltage characteristics in the organic EL devices. The carbon/nitrogen position numbers in the phenanthridine moiety can be represented as follows.

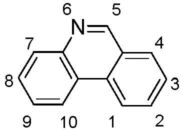

Specifically, the compound represented by Chemical Formula 1 has a pyridine moiety bonded to the carbon at position 5 of the phenanthridine moiety. In this case, the binding position of the pyridine moiety to the phenanthridine moiety is the ortho position of the nitrogen (N). The compound of the present disclosure with such a structure can bind to metals. Consequently, the compound of the present disclosure can form a gap state by binding with metals such as alkali metals and alkaline earth metals, which are dopants in the N-type charge generation layer. When used as an N-type charge generation layer material, the compound of the present disclosure improves properties of the electron transfer from an N-type charge generation layer to an electron transport layer. Additionally, when used as an N-type charge generation layer material, the compound of the present disclosure binds with alkali metals or alkaline earth metals in the N-type charge generation layer, preventing the alkali metal or alkaline earth metal from diffusing into the p-type charge generation layer. Therefore, using the compound of the present disclosure as an N-type charge generation layer material can improve the performance and lifespan of organic EL devices.

As mentioned in the foregoing, the compound represented by Chemical Formula 1 in the present disclosure exhibits excellent electron injection and transport capabilities. Therefore, the compound of the present disclosure may be used as a material for an organic layer material in organic EL devices, preferably as a material for an electron transport layer material. Furthermore, the compound of the present disclosure may be used as a material for an N-type charge generation layer material in tandemstructured organic EL devices. Applying the compound represented by Chemical Formula 1 as an electron transport layer material or an n-type charge generation layer material in organic EL devices can improve characteristics such as driving voltage, luminous efficiency, and lifespan, as well as prevent the increase of progressive driving voltage. Moreover, the compound can maximize the performance of full-color organic light-emitting panels to which the organic EL devices are applied.

In the compound represented by Chemical Formula 1, n may be an integer of 0 to 3 and particularly an integer of 0 to 2.

Here, when n is 0, L₁ is meant to be a direct bond (single bond). When n is an integer of 1 to 3, L₁ is a divalent linker and may be selected from the group consisting of an arylene of C₆-C₃₀ and a heteroarylene of 5 to 30 nuclear atoms, and specifically from the group consisting of an arylene of C₆-C₁₈ and a heteroarylene of 5 to 18 nuclear atoms. Here, the plurality of L₁ may be same or different.

The arylene and heteroarylene groups for L₁ may each be unsubstituted or substituted with a substituent selected from the group consisting of a deuterium atom, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₁-C₄₀, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀, and specifically from the group consisting of a deuterium atom, a halogen, a cyano, a nitro, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, and a heteroaryl of 5 to 60 nuclear atoms. In this regard, if present, two or more substituents may be same or different.

According to an embodiment, 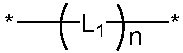 may be a linker represented by the following Chemical Formulas L1 to L5:

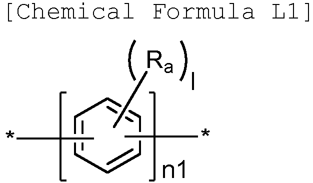

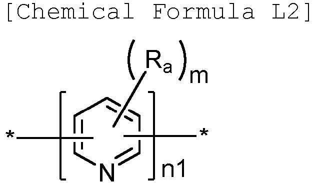

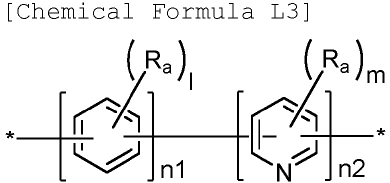

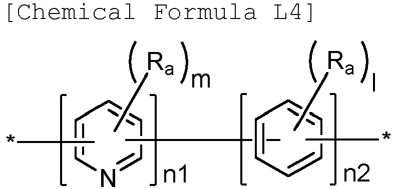

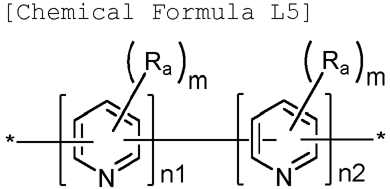

wherein,
n1 and n2 are each an integer of 0 to 3, with a proviso of 0≤n1+n2≤3,
l is an integer of 0 to 4,
m is an integer of 0 to 3, and
two or more Rₐ's, if present, are same or different,
Rₐ being selected from the group consisting of a hydrogen atom, a deuterium atom, a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀, and specifically from the group consisting of a hydrogen atom, a deuterium atom, a cyano, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, and an arylamine of C₆-C₆₀.

According to an embodiment, L₁ may be a direct bond or any one of the following linkers L-1 to L-51: 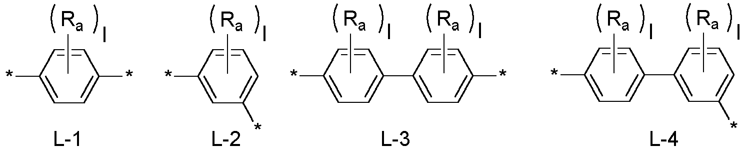 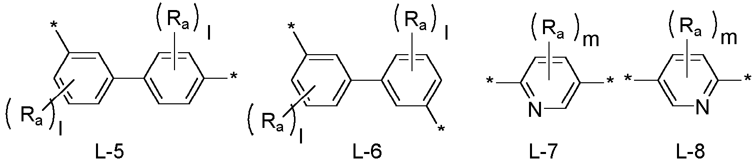 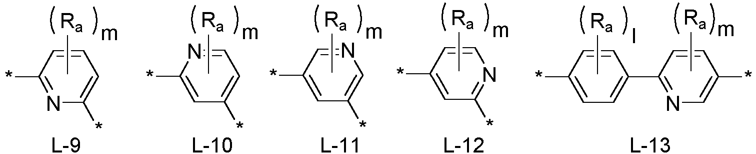 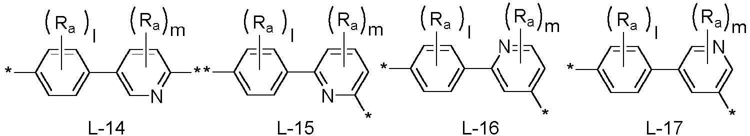 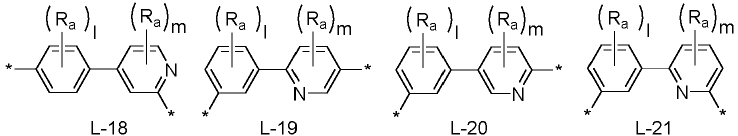 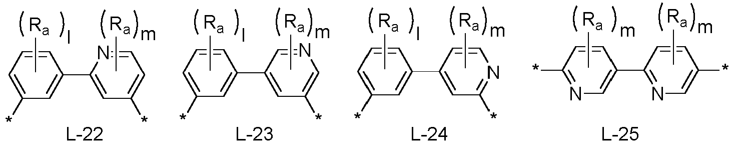 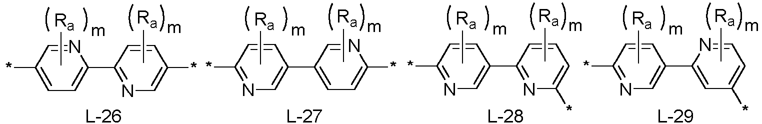 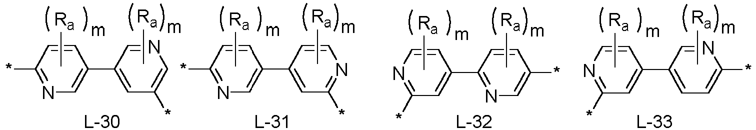 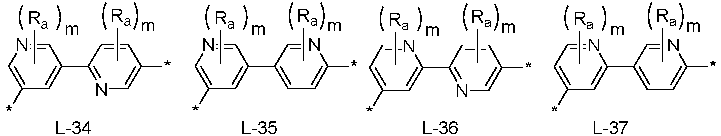 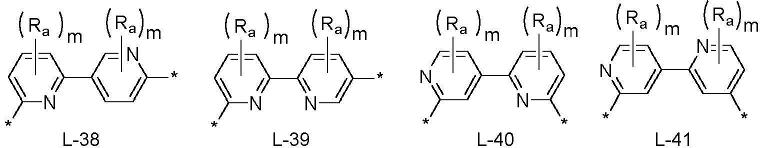 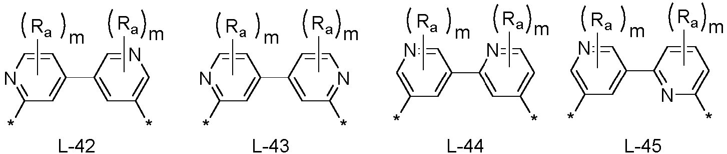 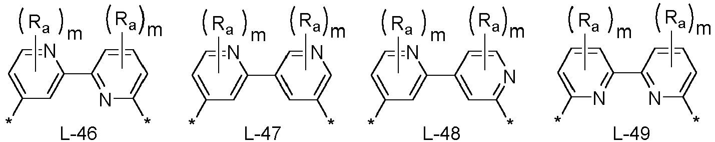 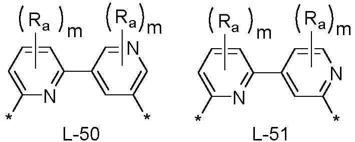 wherein,
* is a site connecting to Chemical Formula 1,
plural l's are same or different,
plural m's are same or different,
l is an integer of 0 to 4,
m is an integer of 0 to 3, and
two or more Rₐ's, if present, are same or different,
Rₐ being selected from the group consisting of a hydrogen atom, a deuterium atom, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₁-C₄₀, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀ and specifically from the group consisting of a hydrogen atom, a deuterium atom (D), a halogen, a cyano, a nitro, an alkyl of C₁-C₁₂, an aryl of C₆-C₁₀, and a heteroaryl of 5-10 nuclear atoms.

Depending on the bonding position of L₁, the compound represented by Chemical Formula 1 may be compounds represented by the following Chemical Formulas 2 to 5, but with no limitations thereto: 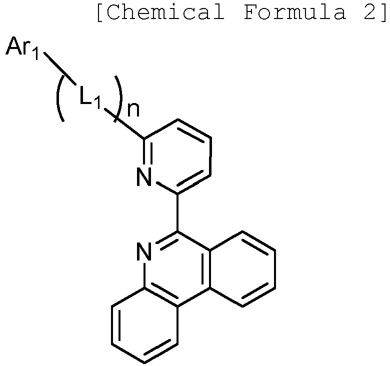 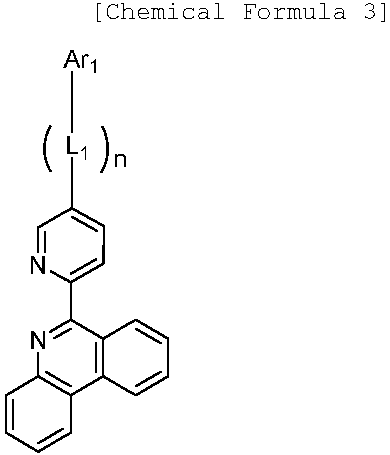 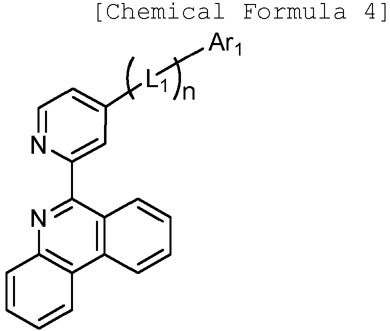 wherein,
n, L₁, and Ar₁ are as defined in Chemical Formula 1.

In the compound represented by Chemical Formula 1, Ar₁ may be selected from the group consisting of a hydrogen atom, a deuterium atom (D), a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, an arylamine of C₆-C₆₀, and an aryl of C₆-C₆₀ substituted with an alkenyl of C₂-C₄₀, or may bond to an adjacent group (e.g., Ar₁-L₁) to form a fused ring. Specifically, Ar₁ may be selected from the group consisting of a hydrogen atom, a deuterium atom, a halogen, a cyano, a nitro, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, and an aryl of C₆-C₆₀ substituted with an alkenyl of C₂-C₄₀, or may bond to an adjacent group (e.g., Ar₁-L₁) to form a fused ring. More specifically, Ar₁ may be selected from the group consisting of a deuterium atom, a cyano, an alkyl of C₂-C₂₀, a cycloalkyl of C₃-C₂₀, a heterocyclic alkyl of 3 to 20 nuclear atoms, an aryl of C₆-C₃₀, a heteroaryl of 5 to 30 nuclear atoms, and an aryl of C₆-C₃₀ substituted with an alkenyl of C₂-C₂₀, or may bond to an adjacent group (e.g., Ar₁-L₁) to form a fused ring. Here, the fused ring may be at least one selected from the group consisting of a fused aliphatic ring of C₃-C₆₀ (specifically, fused aliphatic ring of C₃-C₃₀), a fused aromatic ring of C₆-C₆₀ (specifically, fused aromatic ring of C₆-C₃₀), a 5- to 60-membered, fused heteroaromatic ring containing one or more heteroatoms (e.g., N, O, S, Se, etc.) (specifically, 5- to 30-membered fused heteroaromatic ring containing one or more heteroatoms), a spiro ring of C₃-C₆₀, and a combination thereof.

According to an embodiment, Ar₁ may be a substituent represented by any one selected from the group consisting of the following Chemical Formulas S1 to S12, but is not limited thereto. Particularly, when Ar₁ is an electron withdrawing group (EWG) having high electronic affinity (electron absorptivity) such as the following S1, S2, etc., the compound of the present disclosure may be used as an electron transport layer material because the LUMO level may be adjusted similarly to other electron transport layer materials. Also, when the compound of the present disclosure, in which Ar₁ is an EWG, is used as the N-type charge generation layer material, a reduction can be made a difference in LUMO level from adjacent electron transport layer, thus greatly improving the performance of the organic EL device: 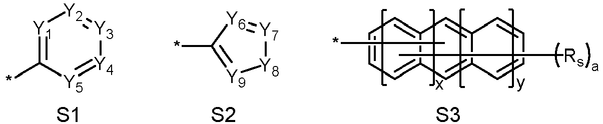 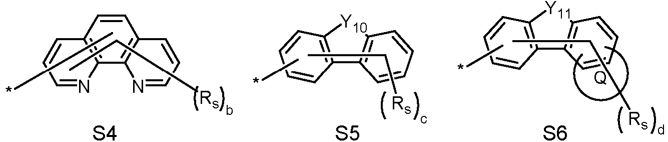 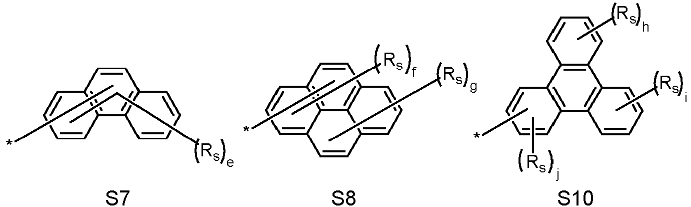 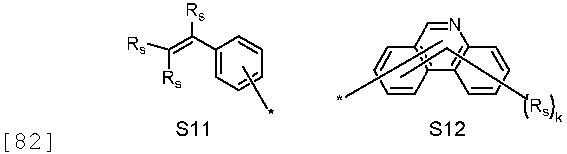 wherein,
* is a site bonding to the compound of Chemical Formula 1,
Y₁ to Y₅, which are same or different, are each independently N or C(R₁) wherein at least one of Y₁ to Y₅ is N and when two or more C(R₁) are present, the corresponding R₁'s are same or different,
Y₆ to Y₉, which are same or different, are each independently N, N(R₂), C(R₃), or C(R₄)(R₅), wherein at least one of Y₆ to Y₉ is N or N(R₂), and when two or more C(R₂) are present, the corresponding R₂'s are same or different, when two or more C(R₂) are present, the corresponding R₃'s are same or different, and when two or more C(R₄)(R₅)are present, the corresponding R₄'s are same or different and the corresponding R₅'s are same or different, with sameness or difference between R₄ and R₅,
Y₁₀ and Y₁₁ are each independently O or S,
cyclic Q is a benzene ring,
x and y are each independently 0 or 1,
a, d, and e are each independently an integer of 0 to 9,
b and c are each independently an integer of 0 to 7,
f, h, and i are each independently an integer of 0 to 4,
g is an integer of 0 to 5,
j is an integer of 0 to 3,
k is an integer of 0 to 8,
R₁ to R₅ and Rₛ, which are same or different, are each independently selected from the group consisting of a hydrogen atom, a deuterium atom, a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀ or each independently bond to an adjacent group (e.g., R₁-R₁, R₂-R₂, R₂-R₃, R₃-R₃, R₄-R₅, and so on) to form a fused ring, specifically are each independently selected from the group consisting of a hydrogen atom, a deuterium atom, a halogen, a cyano, a nitro, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, and a heteroaryl of 5 to 60 nuclear atoms or each independently bond to an adjacent group (e.g., R₂-R₁, R₂-R₂, R₂-R₃, R₃-R₃, R₄-R₅, and so on) to form a fused ring, and more specifically are each independently selected from the group consisting of a deuterium atom, a cyano, an alkyl of C₁-C₂₀, a cycloalkyl of C₃-C₂₀, a heterocyclic alkyl of 3 to 20 nuclear atoms, an aryl of C₆-C₃₀, and a heteroaryl of 5 to 30 nuclear atoms or each independently bond to an adjacent group (e.g., R₁-R₁, R₂-R₂, R₂-R₃, R₃-R₃, R₄-R₅, and so on) to form a fused ring,
the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, arylphosphineoxide, arylamine, and fused ring in R₁ to R₅ and Rₛ each being independently unsubstituted or substituted with at least one substituent selected from the group consisting of a deuterium hydrogen, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₁-C₄₀, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀ and an arylamine of C₆-C₆₀ and specifically with at least one substituent selected from the group consisting of a deuterium atom, a halogen, a cyano, a nitro, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, and a heteroaryl of 5 to 60 nuclear atoms wherein two or more substituents, if present, are same or different.

Specifically, the substituent represented by Chemical Formula S1 may be a substituent represented by any one of the following Chemical Formulas S1-A to S1-D, but with no limitations thereto: 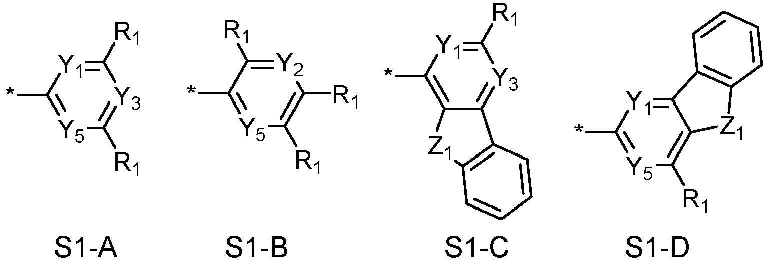 wherein,
Y₁, Y₃, and Y₅, which are same or different, are each independently N or C(R₁), wherein at least one of Y₁, Y₃, and Y₅ is N and when two or more C(R₁) are present, the corresponding R₁'s are same or different,
Y₂ and Y₅, which are same or different, are each independently N or C(R₁) wherein at least one of Y₂ and Y₅ is N and when two or more C(R₁) are present, the corresponding R₁'s are same or different,
Y₁ and Y₃, which are same or different, are each independently N or C(R₁) wherein at least one of Y₁ and Y₃ is N and when two or more C(R₁) are present, the corresponding R₁'s are same or different,
Y₁ and Y₅ 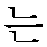 , which are same or different, are each independently N or C(R₁) wherein at least one of Y₁ and Y₅ is N and when two or more C(R₁) are present, the corresponding R₁'s are same or different,
Z₁ is O or S,
R₁ is selected from the group consisting of a hydrogen atom, a deuterium atom, a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀, or binds to an adjacent group (e.g., R₁-R₁) to form a fused ring,
the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, arylphosphineoxide, arylamine, and fused ring in R₁ each being independently unsubstituted or substituted with at least one substituent selected from the group consisting of a deuterium atom, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₁-C₄₀, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀ wherein two or more substituents, if present, are same or different.

In addition, the compound represented by Chemical Formula S2 may be a substituent represented by the following Chemical Formula S2-A: 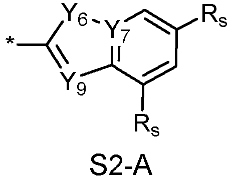 wherein,
Y₆, Y₇, and Y₉, which are same or different, are each independently N, N(R₂), C(R₃), or C(R₄)(R₅)wherein at least one of Y₆, Y₇, and Y₉ is N or N(R₂) and when two or more C(R₁) are present, the corresponding R₃'s are same or different and when two or more C(R₄)(R₅)are present, the corresponding R₄'s are same or different and the corresponding R₅'s are same or different,
R₂ to R₅, and Rₛ, which are same or different, are each independently selected from the group consisting of a hydrogen atom, a deuterium atom, a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀, or each independently bond to an adjacent group (e.g., R₂-R₃, R₄-R₅, R₂-R₄, R₃-R₄) to form a fused ring,
the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, arylphosphineoxide, arylamine, and fused ring in R₂ to R₅ and Rₛ each being independently unsubstituted or substituted with a substituent selected from the group consisting of a deuterium atom, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₁-C₄₀, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀ wherein two or more substituents, if present, are same or different.

In another embodiment, the Ar₁ may be a substituent selected from the group consisting of the following substituents S2-1 to S2-68, but with no limitations thereto: 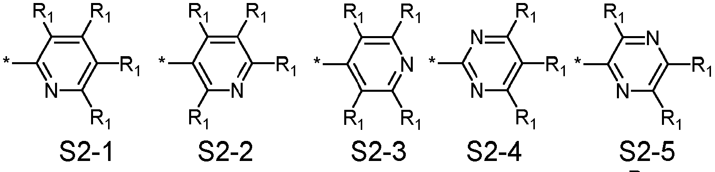 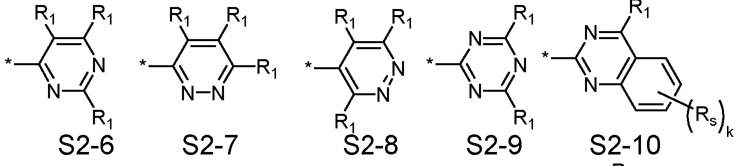 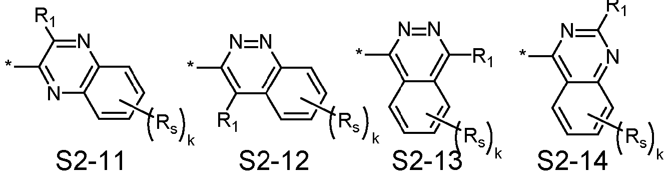 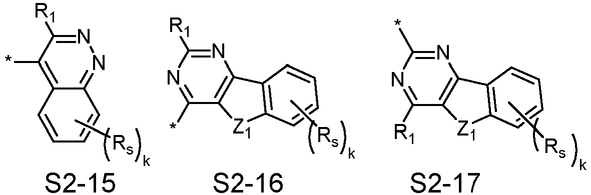 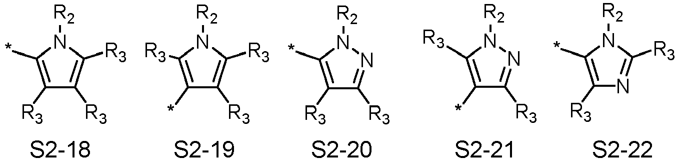 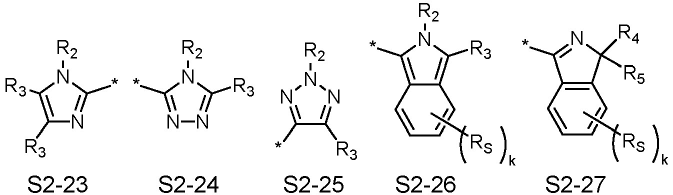 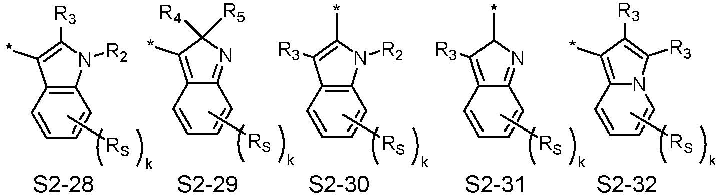 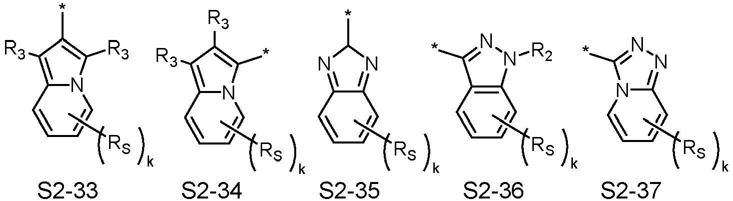 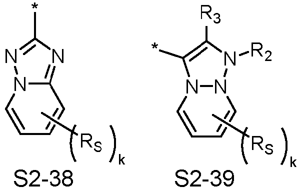 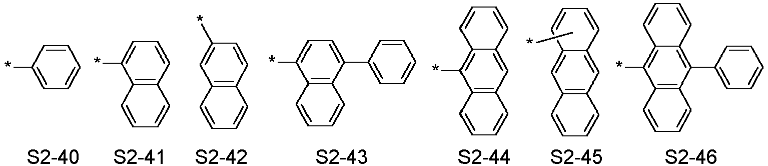 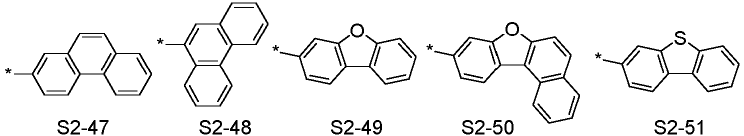 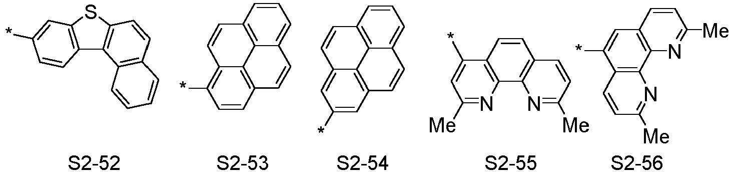 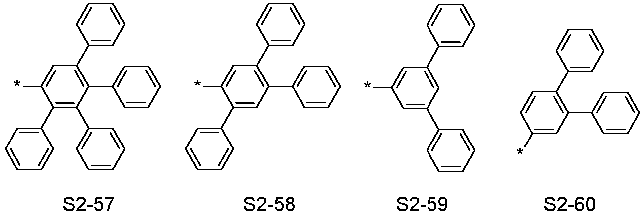 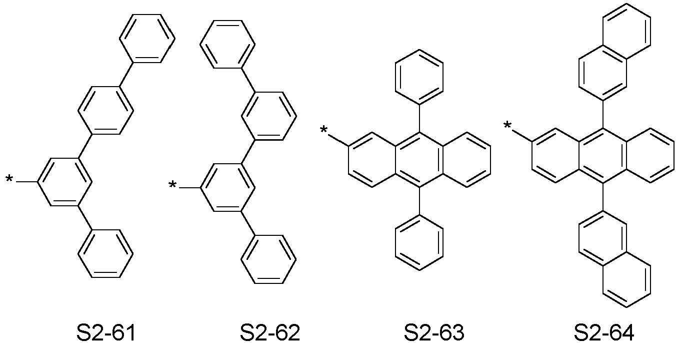 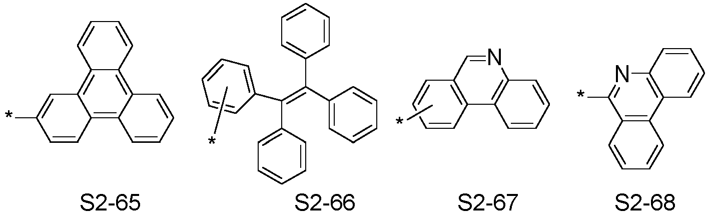 wherein,
* is a bonding site to the compound of Chemical Formula 1,
R₁ to R₅ and Rₛ are each as defined in Chemical Formulas S1 to S10,
k is an integer of 0 to 4,
Me is a methyl.

Depending on L₁ and Ar₁ defined above, the compound represented by Chemical Formula 1 may be a compound represented by any one of Chemical Formula 5 to 17, but with no limitations thereto: 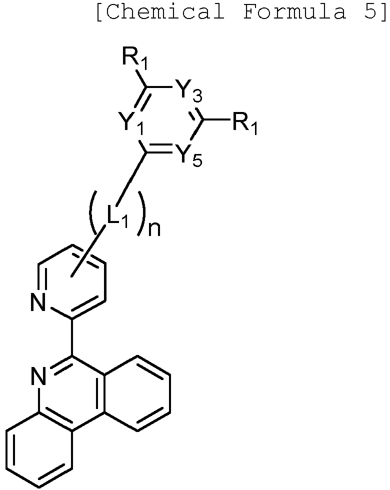 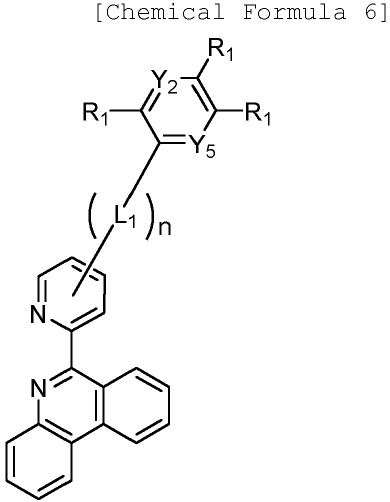 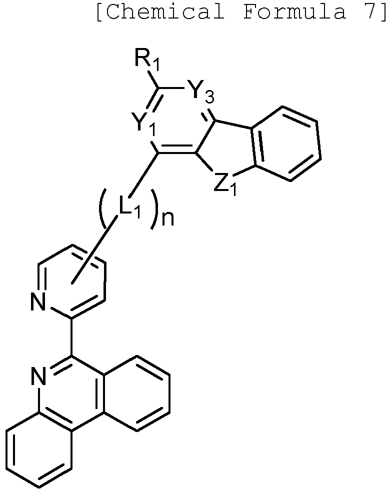 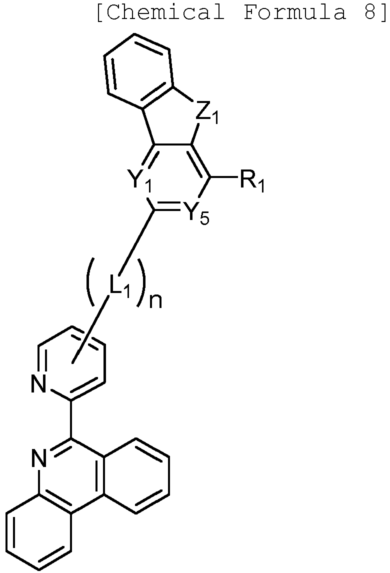 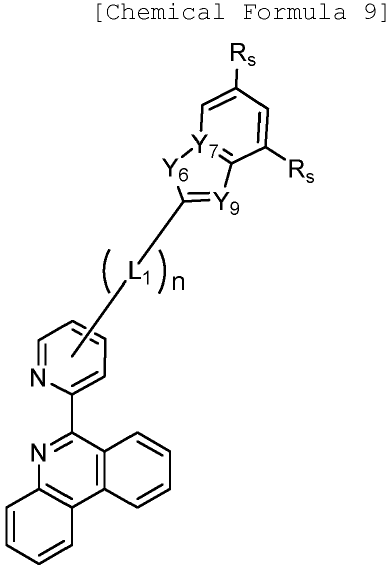 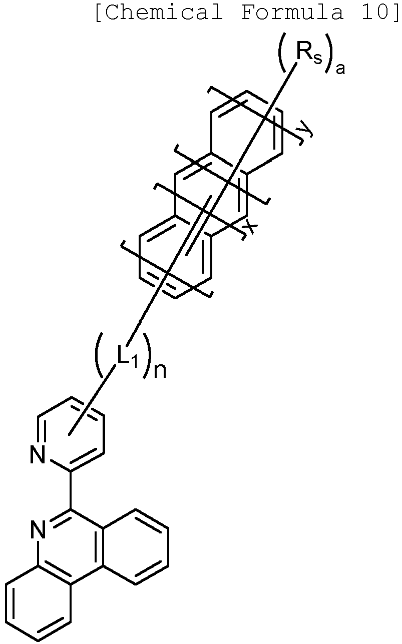 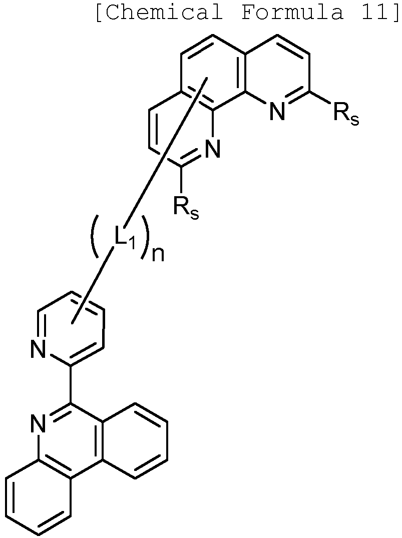 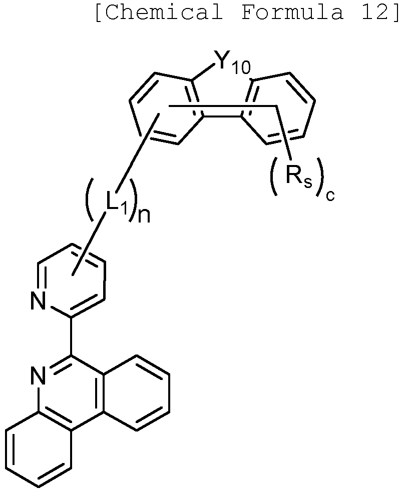 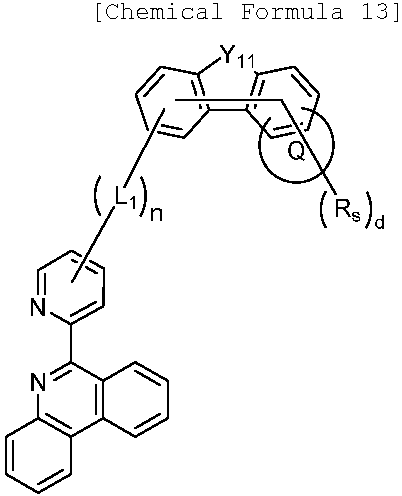 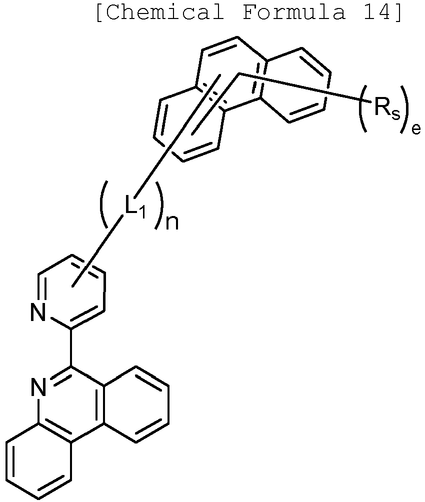 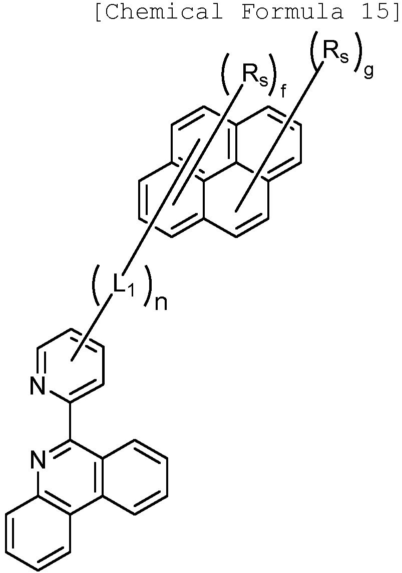 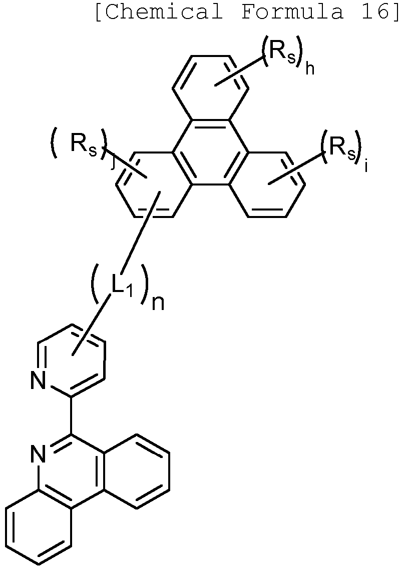 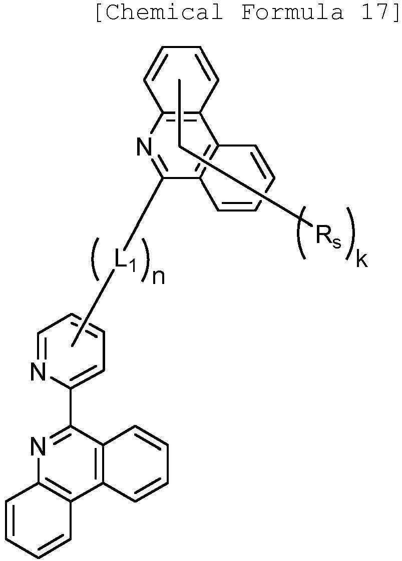 wherein,
n is an integer of 0 to 3,
L₁ is a direct bond or is selected from the group consisting of an arylene of C₆-C₆₀ and a heteroarylene of 5 to 60 nuclear atoms,
Y₁, Y₃, and Y₅, which are same or different, are each independently N or C(R₁), wherein at least one of Y₁, Y₃, and Y₅ is N and when two or more C(R₁) are present, the corresponding R₁'s are same or different,
Y₂ and Y₅, which are same or different, are each independently N or C(R₁) wherein at least one of Y₂ and Y₅ is N and when two or more C(R₁) are present, the corresponding R₁'s are same or different,
Y₁ and Y₃, which are same or different, are each independently N or C(R₁) wherein at least one of Y₁ and Y₃ is N and when two or more C(R₁) are present, the corresponding R₁'s are same or different,
Y₁ and Y₅, which are same or different, are each independently N or C(R₁) wherein at least one of Y₁ and Y₅ is N and when two or more C(R₁) are present, the corresponding R₁'s are same or different,
Y₁₀, Y₁₁, and Z₁ are each independently O or S,
Y₆, Y₇, and Y₉, which are same or different, are each independently N, N(R₂), C(R₃), or C(R₄)(R₅), wherein at least one of Y₆, Y₇, and Y₉ is N or N(R₂), and when two or more C(R₁) are present, the corresponding R₃'s are same or different, and when two or more C(R₄)(R₅)are present, the corresponding R₄'s are same or different and the corresponding R₅'s are same or different,
x and y are each independently 0 or 1,
cyclic Q is a benzene ring,
a, d, and e are each independently an integer of 0 to 9,
b and c are each independently an integer of 0 to 7,
f, h, and i are each independently an integer of 0 to 4,
g is an integer of 0 to 5,
j is an integer of 0 to 3,
k is an integer of 0 to 8,
R₁ to R₅, and Rₛ, which are same or different, are each independently selected from the group consisting of a hydrogen atom, a deuterium atom, a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀, or each independently bond to an adjacent group to form a fused ring,
the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, arylphosphineoxide, arylamine, and fused ring in R₁ to R₅ and Rₛ are each independently unsubstituted or substituted with at least one substituent selected from a deuterium atom, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₁-C₄₀, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀ wherein two or more substituents, if present, are same or different.

Concrete examples of the compound represented by Chemical Formula 1 according to the present disclosure include, but are not limited to, the following Compounds A-1 to C-126:

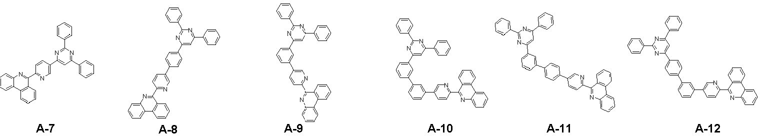

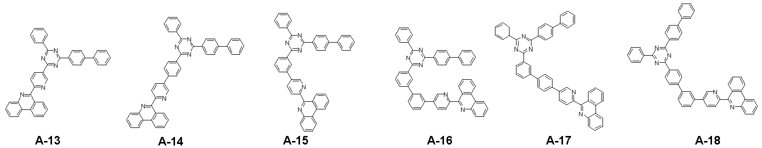

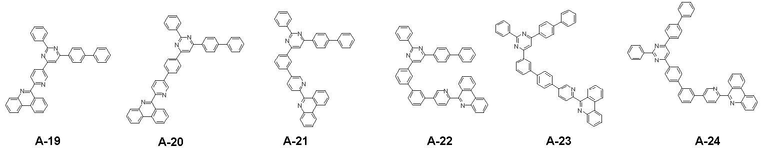

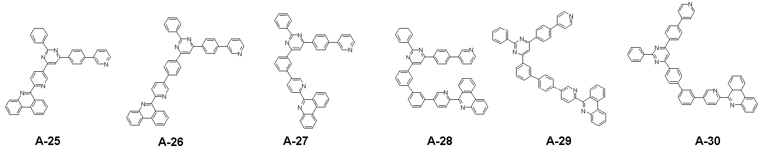

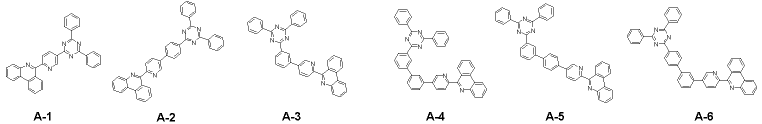

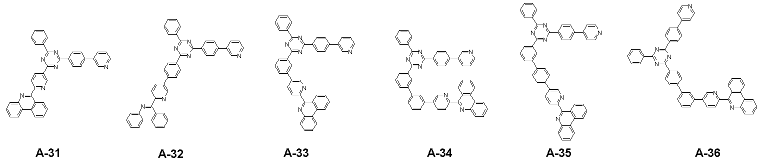

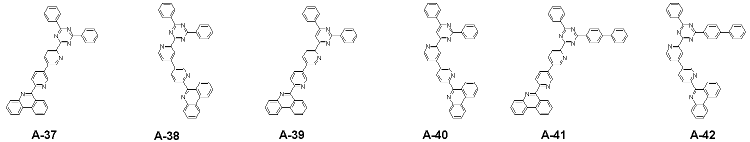

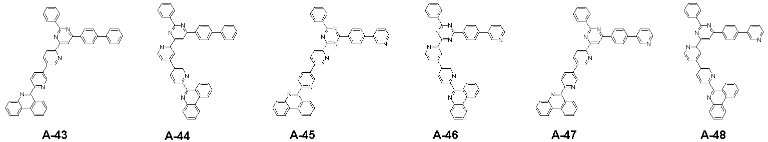

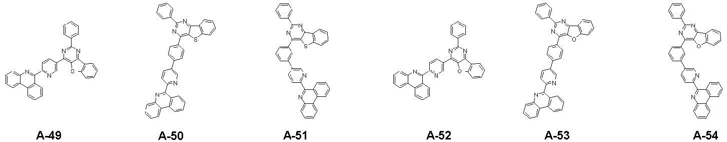

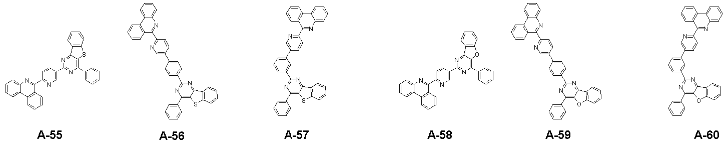

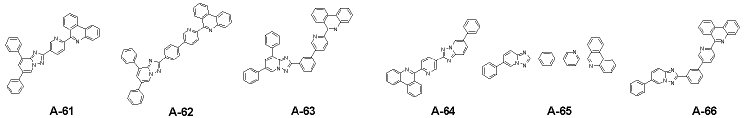

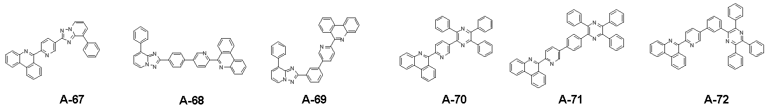

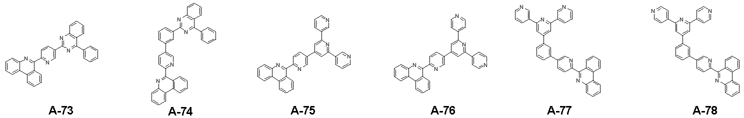

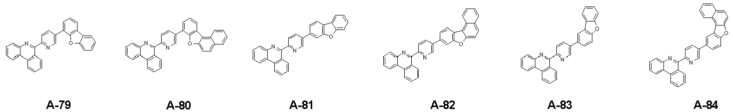

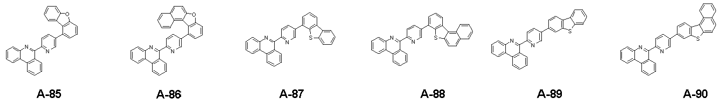

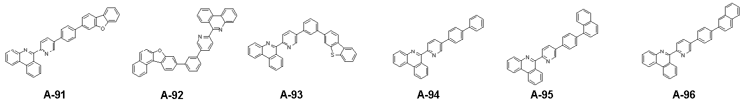

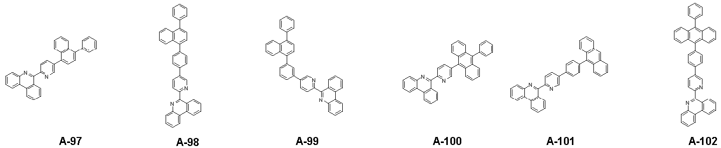

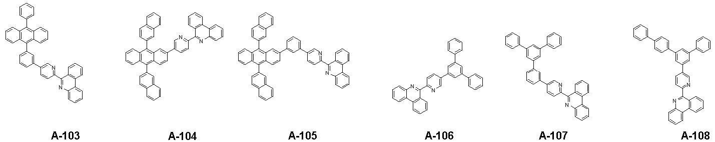

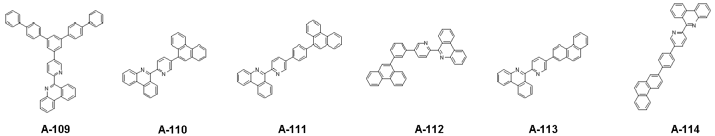

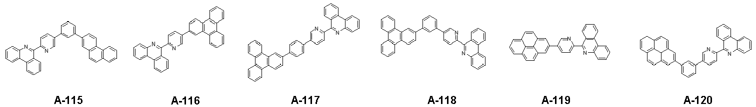

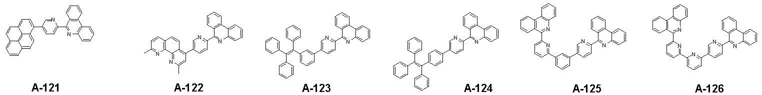

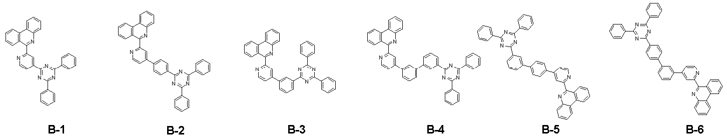

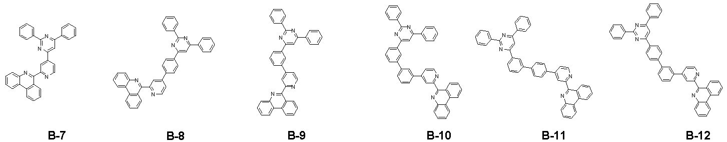

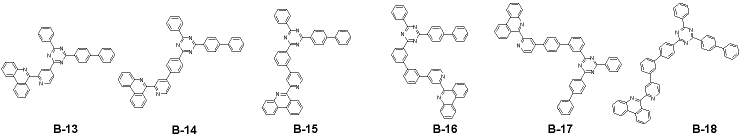

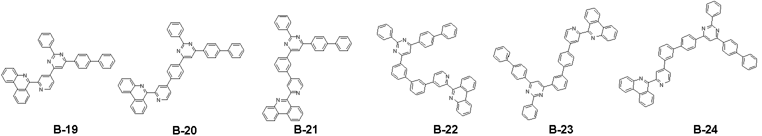

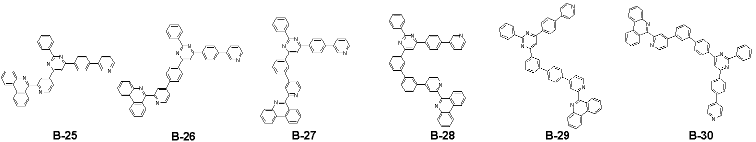

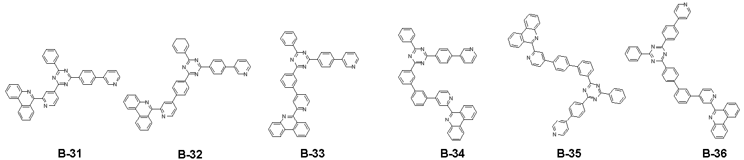

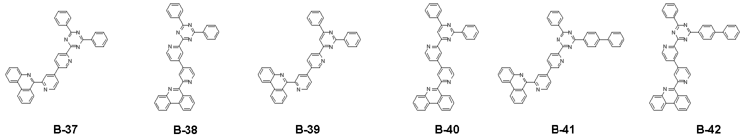

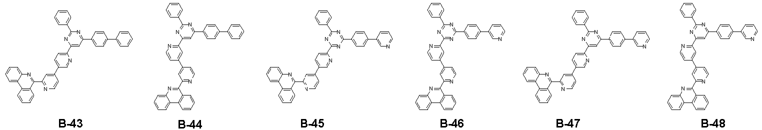

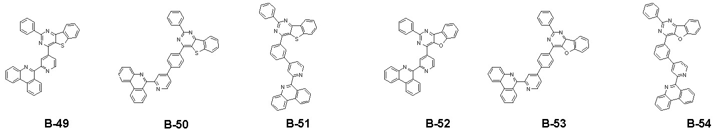

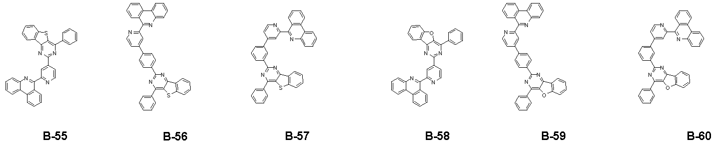

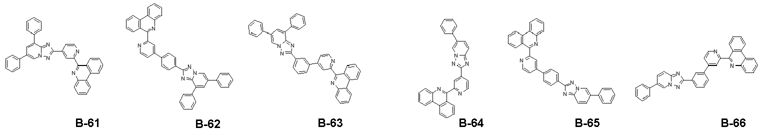

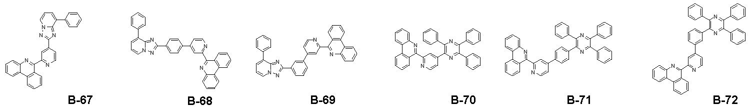

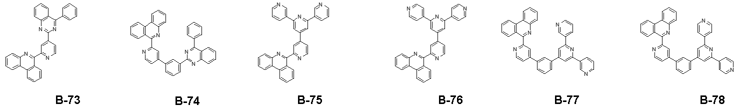

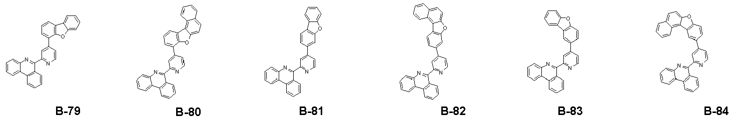

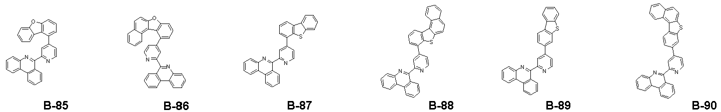

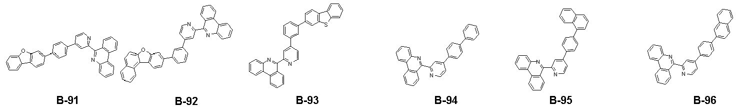

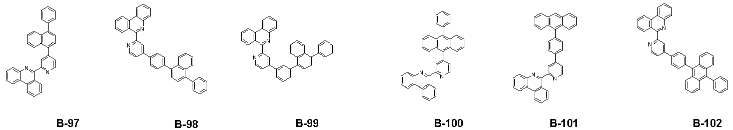

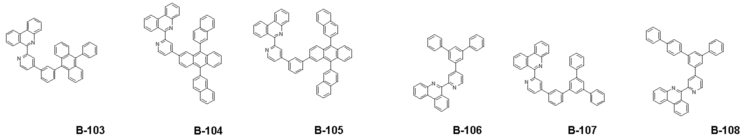

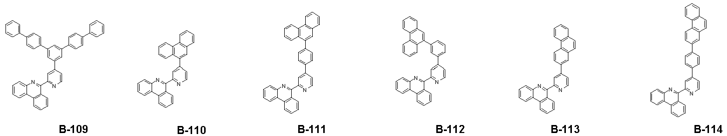

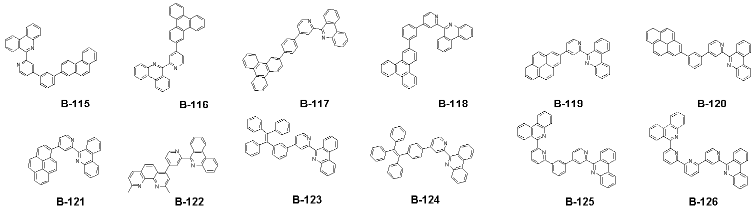

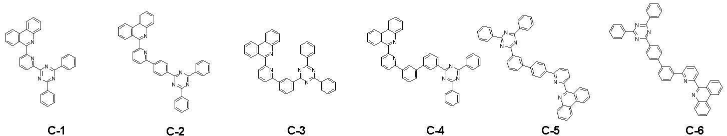

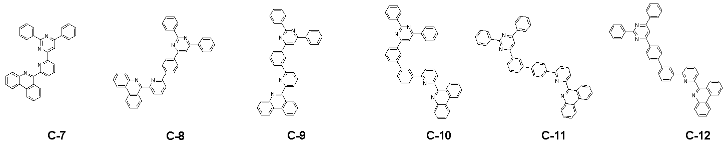

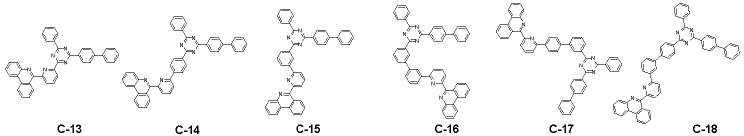

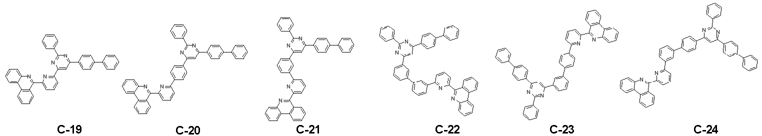

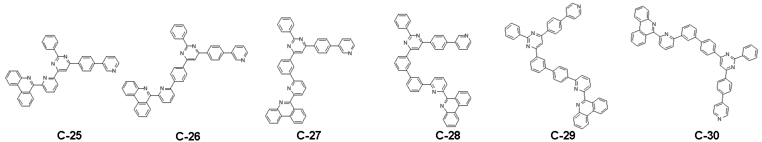

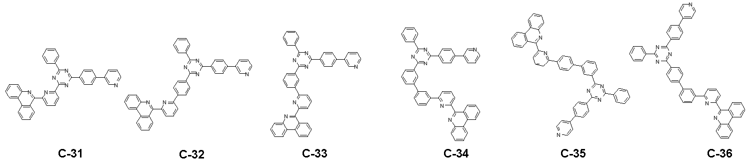

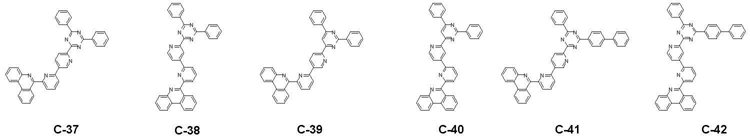

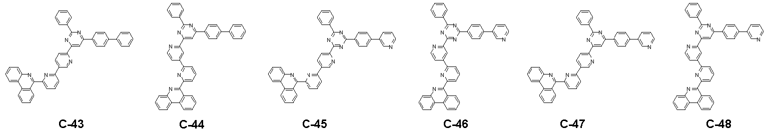

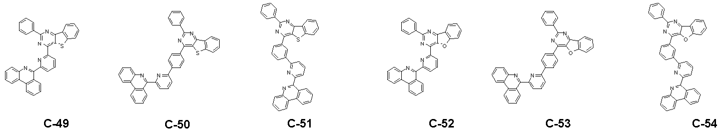

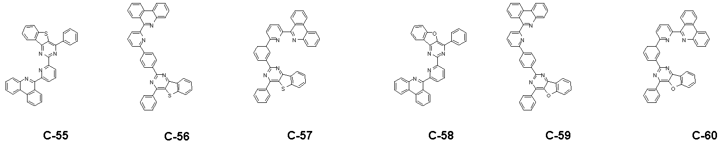

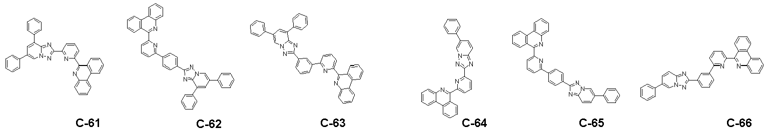

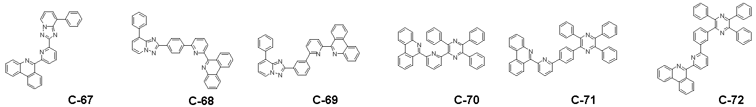

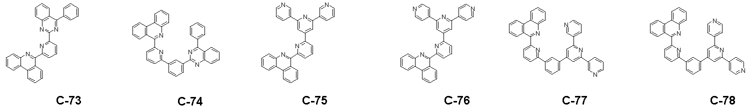

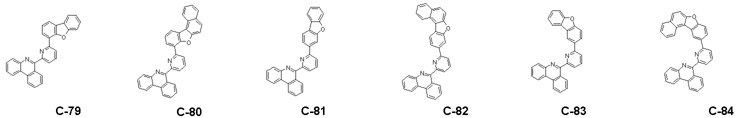

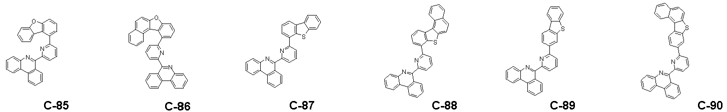

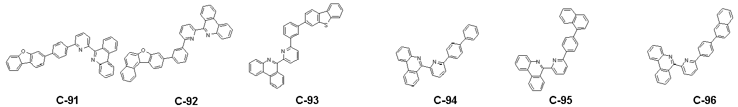

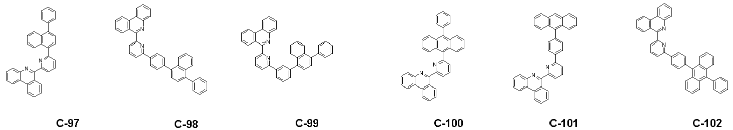

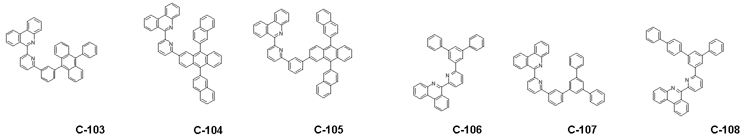

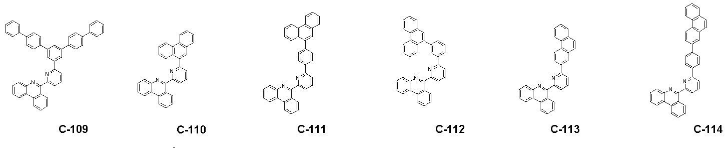

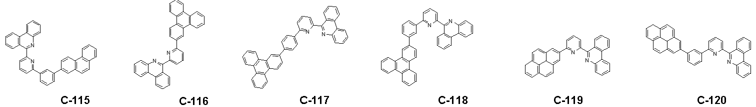

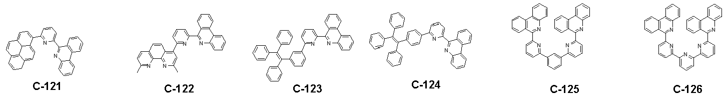

As used herein, the term "alkyl" refers to a monovalent substituent derived from a saturated, linear or branched hydrocarbon of 1 to 40 carbon atoms. Examples of such alkyl may include, but are not limited to, methyl, ethyl, propyl, isobutyl, sec-butyl, pentyl, iso-amyl, hexyl or the like.

As used herein, the term "alkenyl" refers to a monovalent substituent derived from an unsaturated, linear or branched hydrocarbon of 2 to 40 carbon atoms with at least one carbon-carbon double bond therein. Examples of such alkenyl may include, but are not limited to, vinyl, allyl, isopropenyl, 2-butenyl or the like.

As used herein, the term "alkynyl" refers to a monovalent substituent derived from an unsaturated, linear or branched hydrocarbon of 2 to 40 carbon atoms, with at least one carbon-carbon triple bond therein. Examples of such alkynyl may include, but are not limited to, ethynyl, 2-propynyl or the like.

As used herein, the term "cycloalkyl" refers to a monovalent substituent derived from a monocyclic or polycyclic non-aromatic hydrocarbon of 3 to 40 carbon atoms. Examples of such cycloalkyl may include, but are not limited to, cyclopropyl, cyclopentyl, cyclohexyl, norbornyl, adamantine or the like.

As used herein, "heterocycloalkyl" refers to a monovalent substituent derived from a non-aromatic hydrocarbon of 3 to 40 nuclear atoms, , of which one or more carbons on the ring, preferably 1 to 3 carbons, are substituted with a heteroatom, such as N, O, Se, or S. Examples of the heterocycloalkyl may include morpholine, piperazine, and the like, but are not limited thereto.

As used herein, "aryl" refers to a monovalent substituent derived from an aromatic hydrocarbon of 6 to 60 carbon atoms having a single ring or a combination of two or more rings. In addition, the two or more rings may be simply attached (pendant) or fused. Examples of such aryl may include, but are not limited to, phenyl, naphthyl, phenanthryl, anthryl or the like.

The term "heteroaryl", as used herein, refers to a monovalent substituent derived from a monoheterocyclic or polyheterocyclic aromatic hydrocarbon of 5 to 60 nuclear atoms. In this regard, the substituent bears one or more and preferably one to three heteroatoms such as N, O, S, or Se as ring members. In addition, two or more rings may be simply attached (pendant) or fused to each other and may be in a form fused to an aryl group. Examples of such heteroaryl may include, but are not limited to, a 6-membered monocyclic ring such as pyridyl, pyrazinyl, pyrimidinyl, pyridazinyl, and triazinyl; and a polycyclic ring such as phenoxathienyl, indolizinyl, indolyl, purinyl, quinolyl, benzothiazole and carbazolyl; 2-furanyl; N-imidazolyl; 2-isoxazolyl; 2-pyridinyl; 2-pyrimidinyl or the like.

As used herein, the term "alkyloxy" refers to a monovalent substituent represented by R'O-, where R' is an alkyl of 1 to 40 carbon atoms. Such alkyloxy may include a linear, branched or cyclic structure. Examples of such alkyloxy include, but are not limited to, methoxy, ethoxy, n-propoxy, 1-propoxy, t-butoxy, n-butoxy, pentoxy or the like.

The term "aryloxy", as used herein, refers to a monovalent substituent represented by RO-, where R is an aryl of 6 to 60 carbon atoms. Examples of such aryloxy include, but are not limited to, phenyloxy, naphthyloxy, diphenyloxy or the like.

The term "alkylsilyl", as used herein, refers to a silyl substituted with an alkyl of 1 to 40 carbon atoms and is intended to encompass di- and tri-alkylsilyl as well as mono-alkylsilyl. In addition, "arylsilyl" refers to a silyl substituted with an aryl of 5 to 60 carbon atoms and is intended to encompass a poly-arylsilyl such as di- and tri-arylsilyl as well as mono-arylsilyl.

As used herein, the term "alkylboron group" refers to a boron substituted with an alkyl of 1 to 40 carbon atoms, and the term "arylboron group" refers to a boron substituted with aryl of 6 to 60 carbon atoms.

As used herein, the term "alkylphosphinyl" refers to a phosphine substituted with an alkyl of 1 to 40 carbon atoms and is intended to encompass a di-alkylphosphinyl group as well as a mono-alkylphosphinyl group. As used herein, "arylphosphinyl group" refers to a phosphine substituted with a mono- or diaryl of 6 to 60 carbon atoms, and is intended to encompass a di-arylphosphinyl as well as a mono-arylphosphinyl.

As used herein, the term "arylamine" refers to an amine substituted with an aryl of 6 to 60 carbon atoms and is intended to encompass mono- and diarylamine.

The term "heteroarylamine", as used herein, refers to an amine substituted with a heteroaryl of 5 to 60 nuclear atoms and is indented to encompass mono- and diheteroarylamine.

The term "(aryl)(heteroaryl)amine", as used herein, refers to an amine substituted with an aryl of 6 to 60 carbon atom and a heteroaryl of 5 to 60 nuclear atoms.

As used herein, the term "fused ring" refers to a fused aliphatic ring of 3 to 40 carbon atoms, a fused aromatic ring of 6 to 60 carbon atoms, a fused heteroaliphatic ring of 3 to 60 nuclear atoms, a fused heteroaromatic ring of 5 to 60 nuclear atoms, or a combination thereof.

### <Organic EL device>

The present disclosure provides an organic electroluminescence device (hereinafter referred to as "organic EL device") comprising the compound represented by Chemical Formula 1.

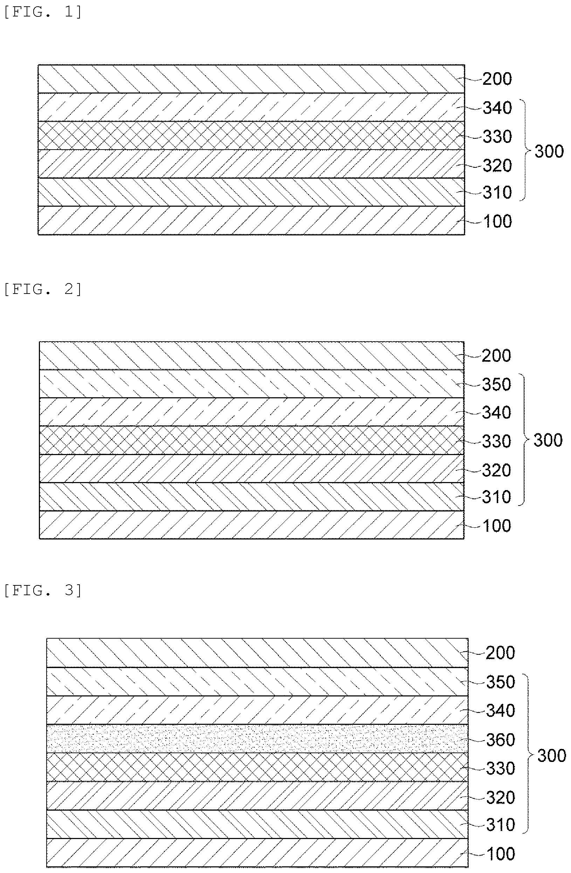

FIGS. 1 to 4 are schematic cross-sectional views of organic EL devices according to the first to fourth embodiments of the present disclosure.

Hereinafter, the organic EL devices according to the first to third embodiments of the present disclosure will be explained in detail with reference to FIGS. 1 to 3.

As shown in FIGS. 1 to 3, the organic EL device according to the present disclosure includes an anode (100), a cathode (200), and one or more organic layers (300) interposed between the anode and cathode, wherein at least one of the one or more organic layers includes the compound represented by Chemical Formula 1. In this regard, the compound can be used alone or in combination with another compound.

The one or more organic layers (300) may include any one or more of a hole injection layer (310), a hole transport layer (320), a light-emitting layer (330), an electron transport auxiliary layer (360), an electron transport layer (340), and an electron injection layer

(350), wherein at least one organic layer (300) contains the compound represented by Chemical Formula 1. Specifically, the organic layer containing the compound represented by Chemical Formula 1 may be an electron transport layer (340). That is, the compound represented by Chemical Formula 1 is included as an electron transport layer material in the organic EL device. In such an organic EL device, electrons are easily injected from the cathode or electron injection layer into the electron transport layer due to the compound of Chemical Formula 1 and can also quickly move from the electron transport layer to the light-emitting layer, resulting in a high binding force of holes and electrons in the light-emitting layer. Therefore, the organic EL device of the present disclosure exhibits excellent luminous efficiency, power efficiency, and brightness. Moreover, the compound of Chemical Formula 1 has excellent thermal and electrochemical stability, thereby improving the performance of the organic EL device.

The compound of Chemical Formula 1 can be used alone or in combination with an electron transport layer material known in the art.

The electron transport layer materials that can be mixed with the compound of Chemical Formula 1 include commonly known electron transport materials in the art. Non-limiting examples of usable electron transport materials include oxazole compounds, isoxazole compounds, triazole compounds, isothiazole compounds, oxadiazole compounds, thiadiazole compounds, perylene compounds, aluminum complexes (e.g., Alq₃, tris(8-quinolinolato)aluminum), gallium complexes (e.g., Gaq'2OPiv, Gaq'2OAc, 2(Gaq'2)), and others. These may be used alone or in combination.

In the present disclosure, when mixing the compound of Chemical Formula 1 with electron transport layer materials, the mixing ratio is not particularly limited and can be appropriately adjusted within the known range in the art.

The structure of the organic EL device of the present disclosure is not particularly limited, but for example, the anode (100), one or more organic layers (300), and the cathode (200) may be sequentially laminated on a substrate (see FIGS. 1 to 3). Furthermore, although not shown, an insulating layer or an adhesive layer may be inserted at the interface between the electrode and the organic layer.

In an embodiment, as shown in FIG. 1, the organic EL device may have a structure in which the anode (100), a hole injection layer (310), a hole transport layer (320), a light-emitting layer (330), an electron transport layer

(340), and the cathode (200) are sequentially laminated on a substrate. Optionally, as shown in FIG. 2, an electron injection layer (350) may be located between the electron transport layer (340) and the cathode (200). Additionally, an electron transport auxiliary layer (360) may be arranged between the light-emitting layer (330) and the electron transport layer (340) (see FIG. 3).

The organic EL device of the present disclosure may be manufactured by forming the organic layer and electrode using known materials and methods in the art, except that at least one of the organic layers (e.g., the electron transport layer (340)) includes the compound represented by Chemical Formula 1.

The organic layer may be formed by a vacuum deposition method or a solution coating method. Examples of the solution coating method include spin coating, dip coating, doctor blading, inkjet printing, or thermal transfer, but are not limited thereto.

The substrate usable in the present disclosure is not particularly limited and may include, for example, silicon wafers, quartz, glass plates, metal plates, plastic films, and sheets.

Examples of anode materials include metals such as vanadium, chromium, copper, zinc, and gold, or alloys thereof; metal oxides such as zinc oxide, indium oxide, indium tin oxide (ITO), and indium zinc oxide (IZO); combinations of metals and oxides such as ZnO:Al or SnO₂:Sb; conductive polymers such as polythiophene, poly(3-methylthiophene), poly[3,4-(ethylene-1,2-dioxy)thiophene] (PEDT), polypyrrole, or polyaniline; and carbon black, but are not limited thereto.

Examples of cathode materials include metals such as magnesium, calcium, sodium, potassium, titanium, indium, yttrium, lithium, gadolinium, aluminum, silver (Ag), tin, lead, and alloys thereof; and multilayer structures such as LiF/Al or LiO₂/Al, but are not limited thereto.

Furthermore, the hole injection layer, hole transport layer, light-emitting layer, and electron injection layer are not particularly limited and may employ conventional materials known in the art.

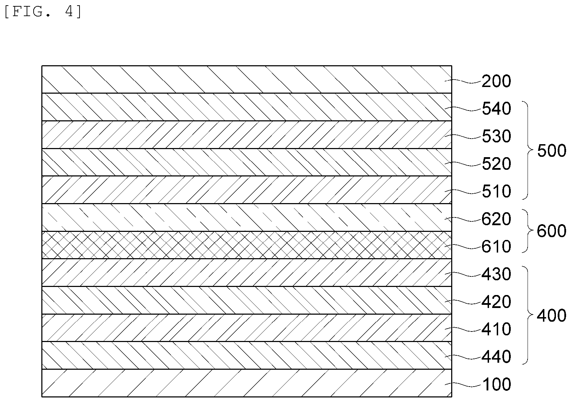

Hereinafter, the organic EL device according to the fourth embodiment of the present disclosure will be described with reference to FIG. 4.

As shown in FIG. 4, the organic EL device according to the fourth embodiment of the present disclosure is a tandem type device including: an anode (100) and a cathode (200) facing each other on a substrate; a plurality of lighting units (400, 500) interposed between the anode (100) and the cathode (200); and a charge generation layer (600) interposed between the adjacent lighting units (400, 500) and including an N-type charge generation layer (610) and a P-type charge generation layer (620). At this time, the N-type charge generation layer (610) includes the compound represented by Chemical Formula 1.

This tandem-type organic EL device has at least two lighting units, and the number of lighting units may be increased by interposing the charge generation layer between adjacent lighting units.

By way of example, a plurality of lighting units may include a first lighting unit (400), a second lighting unit (500), ..., and an m-th lighting unit (m = an integer of 3 or more, specifically 3-4). In this regard, a charge generation layer (600) including an N-type charge generation layer (610) and a P-type charge generation layer (620) is disposed between adjacent lighting units, and the N-type charge generation layer (610) includes the compound represented by Chemical Formula 1.

Specifically, the organic EL device according to the present disclosure includes: an anode (100) and a cathode (200) facing each other; a first lighting unit (400) disposed on the anode (100); a second lighting unit (500) disposed on the first lighting unit (400); and a charge generation layer (600) interposed between the first and second lighting units (400, 500) and including an N-type charge generation layer (610) and a P-type charge generation layer (620). In this regard, the N-type charge generation layer (610) contains the compound represented by Chemical Formula 1.

Each lighting unit (400, 500) includes a hole transport layer (410, 510), a light-emitting layer (420, 520), and an electron transport layer (430, 530). Specifically, the first lighting unit (400) includes a first hole transport layer (410), a first light-emitting layer (420), and a first electron transport layer (430), and the second lighting unit (500) includes a hole transport layer (510), a light-emitting layer (520), and an electron transport layer (530). Optionally, the first lighting unit (400) may additionally include a hole injection layer (440).

The hole transport layer (410, 510), light-emitting layer (420, 520), electron transport layer (430, 530), and hole injection layer (440) are not particularly limited and may employ conventional materials known in the art.

The charge generation layer (600) is disposed between the adjacent lighting units (400, 500), adjusting the charge between the lighting units (400, 500) to achieve charge balance.

The charge generation layer (600) includes: an N-type charge generation layer (610) located adjacent to the first lighting unit (400) and supplying electrons to the first lighting unit (400); and a P-type charge generation layer (620) located adjacent to the second lighting unit (500) and supplying holes to the second lighting unit (500).

The N-type charge generation layer (610) includes the compound represented by Chemical Formula 1. The compound of Chemical Formula 1 has excellent electron mobility and superior electron injection and transport capabilities. Therefore, when applied as an N-type charge generation layer material in an organic EL device, the compound of Chemical Formula 1 can prevent the increase in progressive driving voltage and the degradation of lifespan.

According to an embodiment, the N-type charge generation layer (610) includes a host with electron transport properties, wherein the host is the compound represented by Chemical Formula 1.

The N-type charge generation layer (610) may further include an N-type dopant.

So long as it is generally used in N-type charge generation layers, any N-type dopant is usable in the present disclosure without particular limitations thereto. For example, alkali metals such as Li, Na, K, Rb, Cs, and Fr; alkaline earth metals such as Be, Mg, Ca, Sr, Ba, and Ra; lanthanide metals such as La (lanthanum), Ce (cerium), Pr (preseodyminum), Nd (neodymium), Pm (promethium), Sm (samarium), europium (europium), Gd (gadolinium), Tb (terbium), Dy (dysprosium), Ho (holmium), Er (erbium), Tm (thulium), Yb (ytterbium), and Lu (lutetium); and compounds of one or more of these metals may be used. Additionally, the N-type dopant may be an organic N-type dopant with electron donor properties that can donate at least a part of its electron charge to the organic host (e.g., the compound of Chemical Formula 1) to form a charge-transfer complex with the organic host, such as bis(ethylenedithio)tetrathiafulvalene (BEDT-TTF) and tetrathiafulvalene (TTF).

The thickness of the N-type charge generation layer (610) is not particularly limited and may be in the range of about 5 to 30 nm, for example.

The p-type charge generation layer (620) may be made of a metal or a p-type doped organic material. Examples of the metal include Al, Cu, Fe, Pb, Zn, Au, Pt, W, In, Mo, Ni, and Ti, which may be used alone or as alloys of two or more. Additionally, the materials for the p-type dopant and host used in the p-type doped organic material are not particularly limited as long as they are commonly used materials. For example, the p-type dopant may be F4-TCNQ (2,3,5,6-tetrafluoro-7,7,8,8-tetracyanoquinodimethane), iodine, FeCl₃, FeF₃, and SbCl₅, which may be used alone or in combination of two or more. Non-limiting examples of the host include NPB (N,N'-bis(naphthalen-1-yl)-N,N'-bis(phenyl)-benzidine), TPD (N,N'-bis(3-methylphenyl)-N,N'-bis(phenyl)-benzidine), and TNB (N,N,N',N'-tetranaphthalenyl-benzidine), which may be used alone or in combination of two or more.

The description of the substrate (not shown), anode (100), cathode (200), hole injection layer, hole transport layer, light-emitting layer, and electron injection layer is the same as described in the sections of the first to third embodiments above and thus is omitted here.

A better understanding of the present disclosure may be obtained through the following examples which are set forth to illustrate, but are not to be construed to limit, the present disclosure.

### [SYNTHESIS EXAMPLE 1] Synthesis of Compound A-1

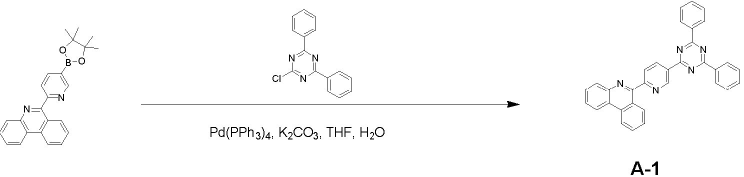

6-(5-(4,4,5,5-Tetramethyl-1,3,2-dioxaborolan-2-yl)pyridin-2-yl)phenanthridine (7 g, 18.3 mmol), 2-chloro-4,6-diphenyl-1,3,5-triazine (4.9 g, 18.3 mmol), Pd(PPh₃)₄ (1.1 g, 0.9 mmol), and K₂CO₃ (7.6 g, 54.9 mmol) in a mixture of toluene (50 ml), EtOH (10 ml) and H₂O (10 ml) were heated for 12 hour under reflux. After completion of the reaction, the reaction mixture was subjected to extraction with methylene chloride. The organic layer thus obtained was added with MgSO₄ and filtered. The solvent was removed from the filtered organic layer, followed by column chromatography to afford the title compound (7.8 g, yield 87 %).
[LCMS] : 488

### [SYNTHESIS EXAMPLE 2] Synthesis of Compound A-3

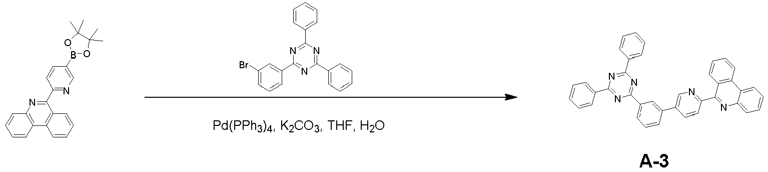

The same procedure as in Synthesis Example 1 with the exception of using 2-(3-bromophenyl)-4,6-diphenyl-1,3,5-triazine instead of 2-chloro-4,6-diphenyl-1,3,5-triazine was carried out to afford the title compound (8.6 g, yield 83 %).
[LCMS] : 564

### [SYNTHESIS EXAMPLE 3] Synthesis of Compound A-7

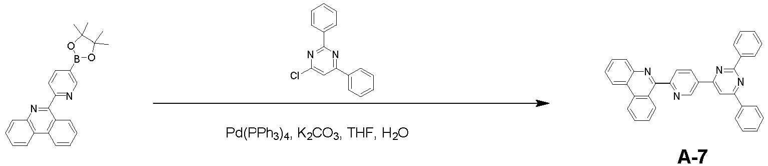

The same procedure as in Synthesis Example 1 with the exception of using 4-chloro-2,6-diphenylpyrimidine instead of 2-chloro-4,6-diphenyl-1,3,5-triazine was carried out to afford the title compound (7.7 g, yield 86 %).
[LCMS] : 487

### [SYNTHESIS EXAMPLE 41 Synthesis of Compound A-8

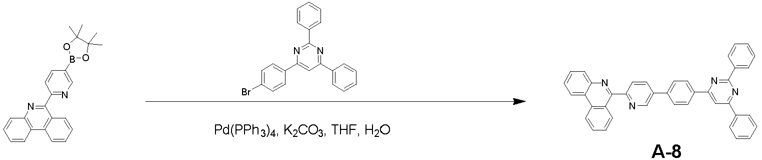

The same procedure as in Synthesis Example 1 with the exception of using 4-(4-bromophenyl)-2,6-diphenylpyrimidine instead of 2-chloro-4,6-diphenyl-1,3,5-triazine was carried out to afford the title compound (8.6 g, yield 83 %).
[LCMS] : 563

### [SYNTHESIS EXAMPLE 5] Synthesis of Compound A-13

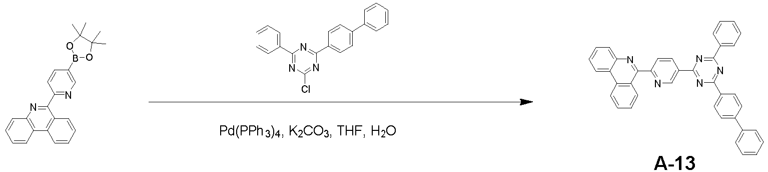

The same procedure as in Synthesis Example 1 with the exception of using 2-([1,1'-biphenyl]-4-yl)-4-chloro-6-phenyl-1,3,5-triazine instead of 2-chloro-4,6-diphenyl-1,3,5-triazine was carried out to afford the title compound (8.5 g, yield 82 %).
[LCMS] : 564

### [SYNTHESIS EXAMPLE 6] Synthesis of Compound A-19

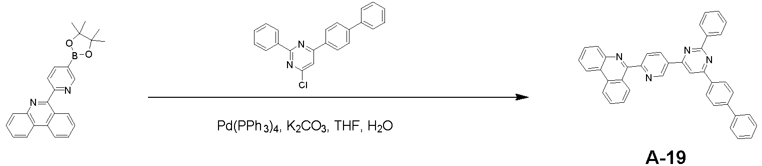

The same procedure as in Synthesis Example 1 with the exception of using 4-([1,1'-biphenyl]-4-yl)-6-chloro-2-phenylpyrimidine instead of 2-chloro-4,6-diphenyl-1,3,5-triazine was carried out to afford the title compound (8.6 g, yield 83 %).
[LCMS] : 563

### [SYNTHESIS EXAMPLE 7] Synthesis of Compound A-25

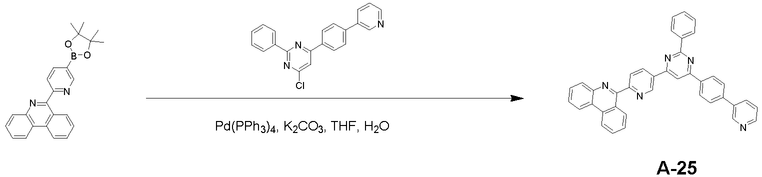

The same procedure as in Synthesis Example 1 with the exception of using 4-chloro-2-phenyl-6-(4-(pyridin-3-yl)phenyl)pyrimidine instead of 2-chloro-4,6-diphenyl-1,3,5-triazine was carried out to afford the title compound (8.5 g, yield 82 %).
[LCMS] : 564

### [SYNTHESIS EXAMPLE 8] Synthesis of Compound A-31

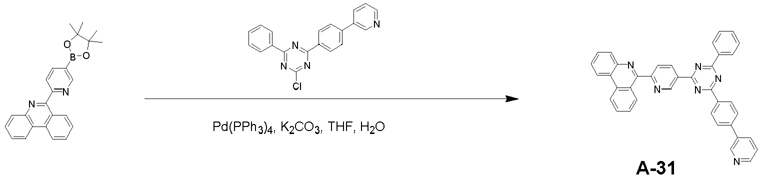

The same procedure as in Synthesis Example 1 with the exception of using 2-chloro-4-phenyl-6-(4-(pyridin-3-yl)phenyl)-1,3,5-triazine instead of 2-chloro-4,6-diphenyl-1,3,5-triazine was carried out to afford the title compound (8.3 g, yield 80 %).
[LCMS] : 565

### [SYNTHESIS EXAMPLE 9] Synthesis of Compound A-37

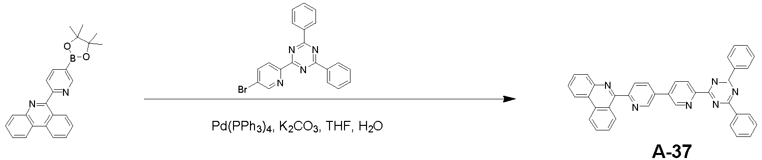

The same procedure as in Synthesis Example 1 with the exception of using 2-(5-bromopyridin-2-yl)-4,6-diphenyl-1,3,5-triazine instead of 2-chloro-4,6-diphenyl-1,3,5-triazine was carried out to afford the title compound (8.0 g, yield 77 %).
[LCMS] : 565

### [SYNTHESIS EXAMPLE 10] Synthesis of Compound A-38

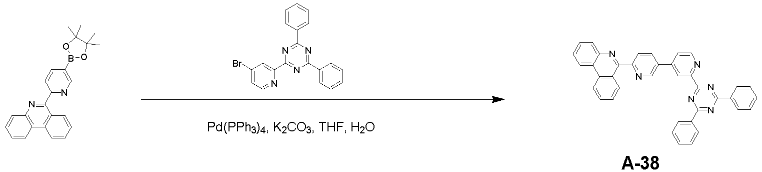

The same procedure as in Synthesis Example 1 with the exception of using 2-(4-bromopyridin-2-yl)-4,6-diphenyl-1,3,5-triazine instead of 2-chloro-4,6-diphenyl-1,3,5-triazine was carried out to afford the title compound (8.0 g, yield 77 %).
[LCMS] : 565

### [SYNTHESIS EXAMPLE 11] Synthesis of Compound A-40

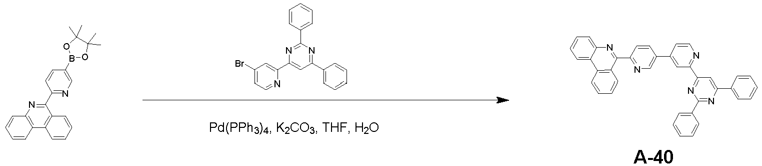

The same procedure as in Synthesis Example 1 with the exception of using 4-(4-bromopyridin-2-yl)-2,6-diphenylpyrimidine instead of 2-chloro-4,6-diphenyl-1,3,5-triazine was carried out to afford the title compound (7.9 g, yield 76 %).
[LCMS] : 564

### [SYNTHESIS EXAMPLE 12] Synthesis of Compound A-43

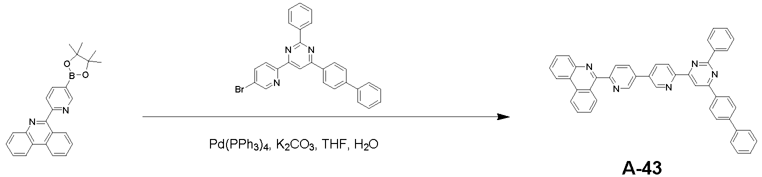

The same procedure as in Synthesis Example 1 with the exception of using 4-([1,1'-biphenyl]-4-yl)-6-(5-bromopyridin-2-yl)-2-phenylpyrimidine instead of 2-chloro-4,6-diphenyl-1,3,5-triazine was carried out to afford the title compound (9.0 g, yield 76 %).
[LCMS] : 640

### [SYNTHESIS EXAMPLE 13] Synthesis of Compound A-49

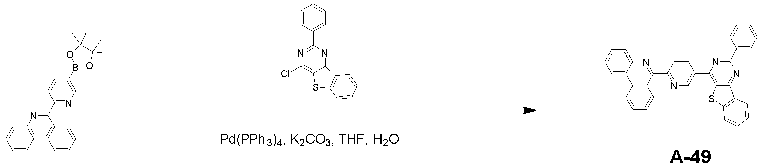

The same procedure as in Synthesis Example 1 with the exception of using 4-chloro-2-phenylbenzo[4,5]thieno[3,2-d]pyrimidine instead of 2-chloro-4,6-diphenyl-1,3,5-triazine was carried out to afford the title compound (7.6 g, yield 80 %).
[LCMS] : 517

### [SYNTHESIS EXAMPLE 14] Synthesis of Compound A-51

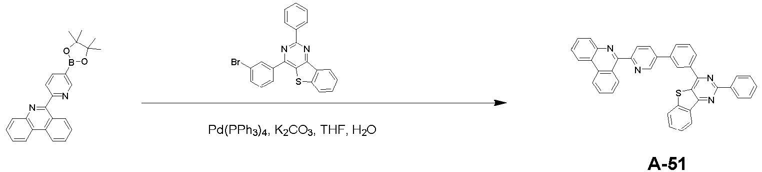

The same procedure as in Synthesis Example 1 with the exception of using 4-(3-bromophenyl)-2-phenylbenzo[4,5]thieno[3,2-d]pyrimidine instead of 2-chloro-4,6-diphenyl-1,3,5-triazine was carried out to afford the title compound (8.6 g, yield 79 %).
[LCMS] : 593

### [SYNTHESIS EXAMPLE 15] Synthesis of Compound A-52

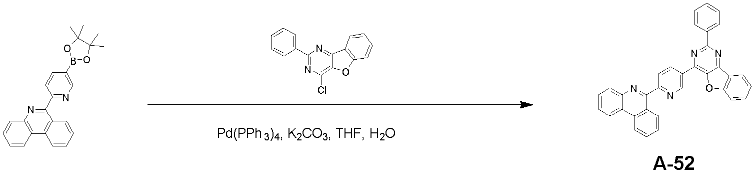

The same procedure as in Synthesis Example 1 with the exception of using 4-chloro-2-phenylbenzofuro[3,2-d]pyrimidine instead of 2-chloro-4,6-diphenyl-1,3,5-triazine was carried out to afford the title compound (7.5 g, yield 82 %).
[LCMS] : 501

### [SYNTHESIS EXAMPLE 16] Synthesis of Compound A-55

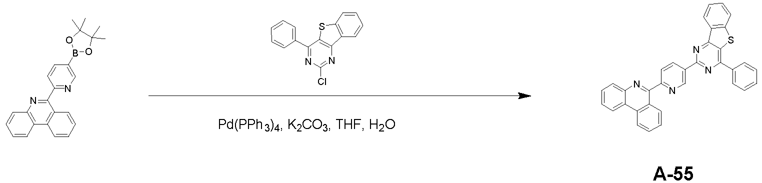

The same procedure as in Synthesis Example 1 with the exception of using 2-chloro-4-phenylbenzo[4,5]thieno[3,2-d]pyrimidine instead of 2-chloro-4,6-diphenyl-1,3,5-triazine was carried out to afford the title compound (7.8 g, yield 82 %).
[LCMS] : 517

### [SYNTHESIS EXAMPLE 17] Synthesis of Compound A-56

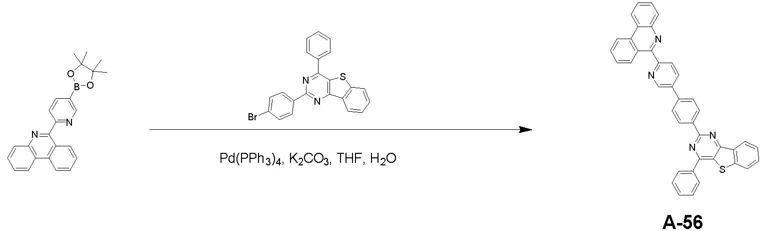

The same procedure as in Synthesis Example 1 with the exception of using 2-(4-bromophenyl)-4-phenylbenzo[4,5]thieno[3,2-d]pyrimidine instead of 2-chloro-4,6-diphenyl-1,3,5-triazine was carried out to afford the title compound (8.6 g, yield 79 %).
[LCMS] : 593

### [SYNTHESIS EXAMPLE 18] Synthesis of Compound A-61

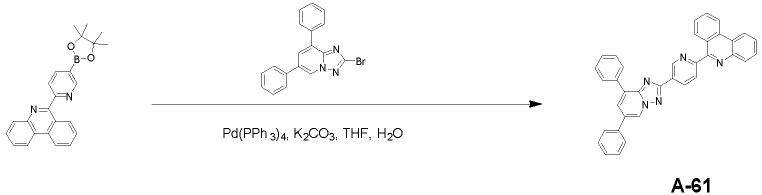

The same procedure as in Synthesis Example 1 with the exception of using 2-bromo-6,8-diphenyl-[1,2,4]triazolo[1,5-a]pyridine instead of 2-chloro-4,6-diphenyl-1,3,5-triazine was carried out to afford the title compound (7.6 g, yield 79 %).
[LCMS] : 526

### [SYNTHESIS EXAMPLE 19] Synthesis of Compound A-70

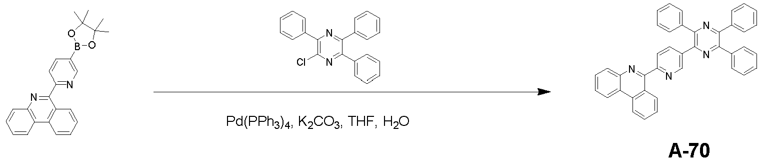

The same procedure as in Synthesis Example 1 with the exception of using 2-chloro-3,5,6-triphenylpyrazine instead of 2-chloro-4,6-diphenyl-1,3,5-triazine was carried out to afford the title compound (8.0 g, yield 77 %) .
[LCMS] : 563

### [SYNTHESIS EXAMPLE 20] Synthesis of Compound A-72

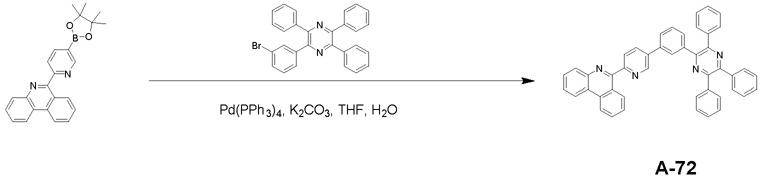

The same procedure as in Synthesis Example 1 with the exception of using 2-(3-bromophenyl)-3,5,6-triphenylpyrazine instead of 2-chloro-4,6-diphenyl-1,3,5-triazine was carried out to afford the title compound (9.2 g, yield 78 %).
[LCMS] : 639

### [SYNTHESIS EXAMPLE 21] Synthesis of Compound A-73

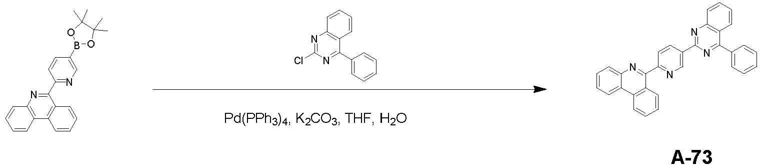

The same procedure as in Synthesis Example 1 with the exception of using 2-chloro-4-phenylquinazoline instead of 2-chloro-4,6-diphenyl-1,3,5-triazine was carried out to afford the title compound (6.7 g, yield 79 %).
[LCMS] : 461

### [SYNTHESIS EXAMPLE 22] Synthesis of Compound A-75

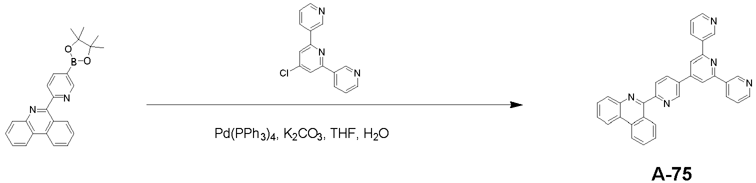

The same procedure as in Synthesis Example 1 with the exception of using 4'-chloro-3,2':6',3"-terpyridine instead of 2-chloro-4,6-diphenyl-1,3,5-triazine was carried out to afford the title compound (7.0 g, yield 78 %).
[LCMS] : 488

### [SYNTHESIS EXAMPLE 23] Synthesis of Compound A-79

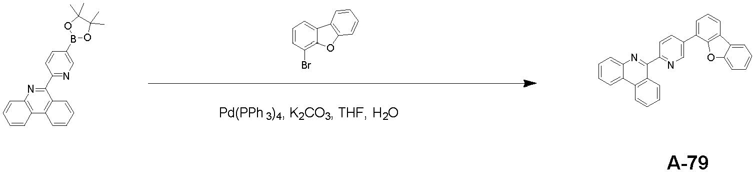

The same procedure as in Synthesis Example 1 with the exception of using 4-bromodibenzo[b,d]furan instead of 2-chloro-4,6-diphenyl-1,3,5-triazine was carried out to afford the title compound (6.3 g, yield 81 %).
[LCMS] : 423

### [SYNTHESIS EXAMPLE 24] Synthesis of Compound A-81

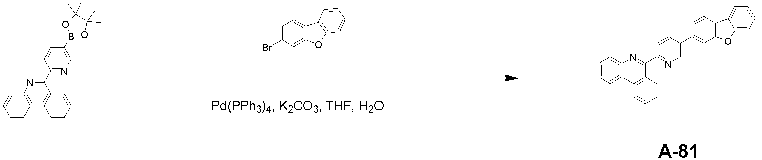

The same procedure as in Synthesis Example 1 with the exception of using 3-bromodibenzo[b,d]furan instead of 2-chloro-4,6-diphenyl-1,3,5-triazine was carried out to afford the title compound (6.3 g, yield 81 %).
[LCMS] : 423

### [SYNTHESIS EXAMPLE 25] Synthesis of Compound A-82

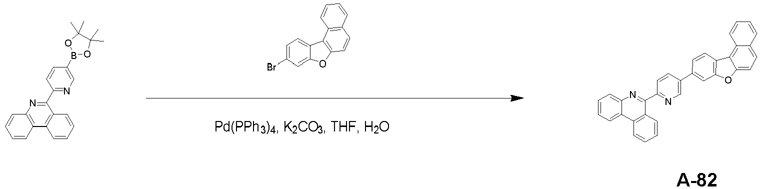

The same procedure as in Synthesis Example 1 with the exception of using 9-bromonaphtho[2,1-b]benzofuran instead of 2-chloro-4,6-diphenyl-1,3,5-triazine was carried out to afford the title compound (6.7 g, yield 77 %).
[LCMS] : 473

### [SYNTHESIS EXAMPLE 26] Synthesis of Compound A-88

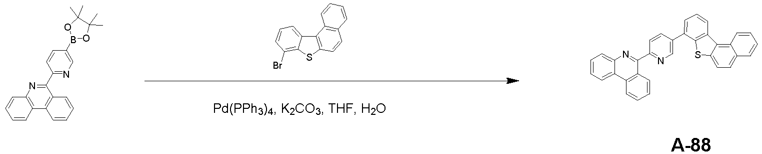

The same procedure as in Synthesis Example 1 with the exception of using 8-bromobenzo[b]naphtho[1,2-d]thiophene instead of 2-chloro-4,6-diphenyl-1,3,5-triazine was carried out to afford the title compound (6.9 g, yield 77 %) .
[LCMS] : 489

### [SYNTHESIS EXAMPLE 27] Synthesis of Compound A-89

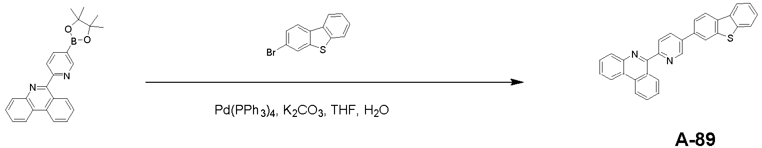

The same procedure as in Synthesis Example 1 with the exception of using 3-bromodibenzo[b,d]thiophene instead of 2-chloro-4,6-diphenyl-1,3,5-triazine was carried out to afford the title compound (6.4 g, yield 80 %).
[LCMS] : 439

### [SYNTHESIS EXAMPLE 28] Synthesis of Compound A-96

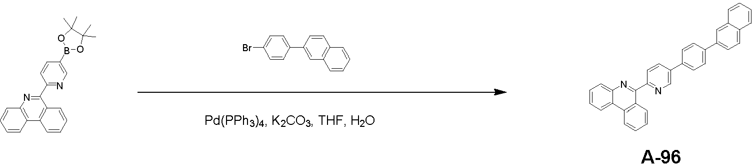

The same procedure as in Synthesis Example 1 with the exception of using 2-(4-bromophenyl)naphthalene instead of 2-chloro-4,6-diphenyl-1,3,5-triazine was carried out to afford the title compound (6.7 g, yield 80 %).
[LCMS] : 459

### [SYNTHESIS EXAMPLE 29] Synthesis of Compound A-97

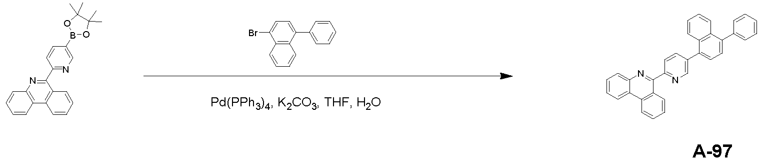

The same procedure as in Synthesis Example 1 with the exception of using 1-bromo-4-phenylnaphthalene instead of 2-chloro-4,6-diphenyl-1,3,5-triazine was carried out to afford the title compound (6.6 g, yield 78 %).
[LCMS] : 459

### [SYNTHESIS EXAMPLE 30] Synthesis of Compound A-100

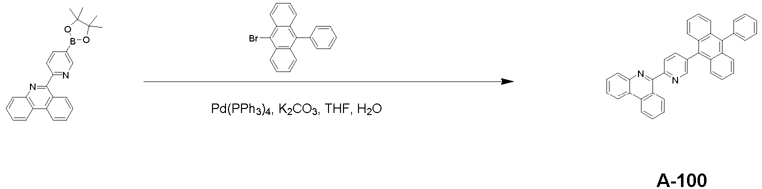

The same procedure as in Synthesis Example 1 with the exception of using 9-bromo-10-phenylanthracene instead of 2-chloro-4,6-diphenyl-1,3,5-triazine was carried out to afford the title compound (6.9 g, yield 74 %).
[LCMS] : 509

### [SYNTHESIS EXAMPLE 31] Synthesis of Compound A-104

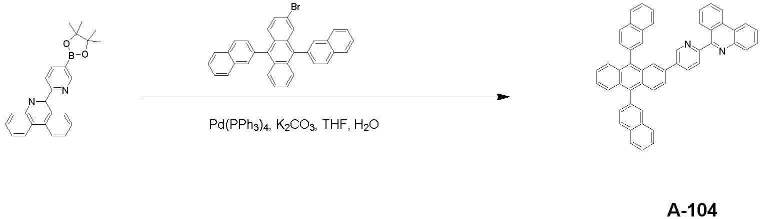

The same procedure as in Synthesis Example 1 with the exception of using 2-bromo-9,10-di(naphthalen-2-yl)anthracene instead of 2-chloro-4,6-diphenyl-1,3,5-triazine was carried out to afford the title compound (9.3 g, yield 74 %).
[LCMS] : 685

### [SYNTHESIS EXAMPLE 32] Synthesis of Compound A-106

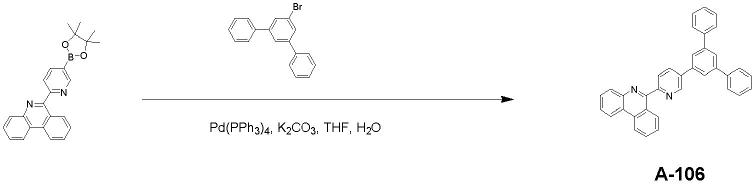

The same procedure as in Synthesis Example 1 with the exception of using 5 '-bromo-1,1':3',1"-terphenyl instead of 2-chloro-4,6-diphenyl-1,3,5-triazine was carried out to afford the title compound (6.8 g, yield 76 %).
[LCMS] : 485

### [SYNTHESIS EXAMPLE 33] Synthesis of Compound A-110

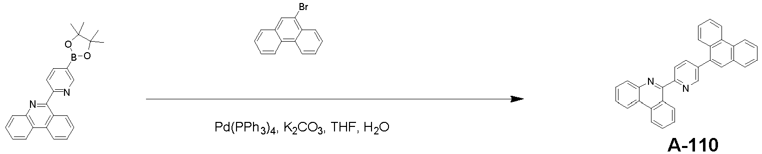

The same procedure as in Synthesis Example 1 with the exception of using 9-bromophenanthrene instead of 2-chloro-4,6-diphenyl-1,3,5-triazine was carried out to afford the title compound (6.0 g, yield 76 %).
[LCMS] : 433

### [SYNTHESIS EXAMPLE 34] Synthesis of Compound A-112

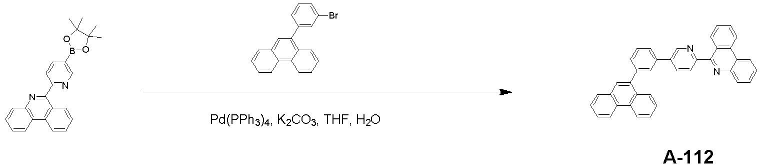

The same procedure as in Synthesis Example 1 with the exception of using 9-(3-bromophenyl)phenanthrene instead of 2-chloro-4,6-diphenyl-1,3,5-triazine was carried out to afford the title compound (7.0 g, yield 75 %).
[LCMS] : 509

### [SYNTHESIS EXAMPLE 35] Synthesis of Compound A-113

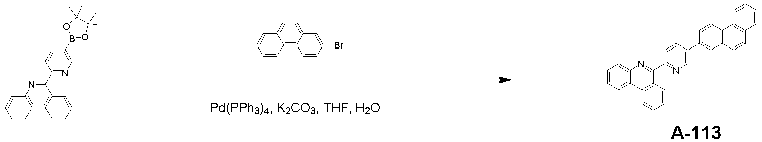

The same procedure as in Synthesis Example 1 with the exception of using 2-bromophenanthrene instead of 2-chloro-4,6-diphenyl-1,3,5-triazine was carried out to afford the title compound (6.0 g, yield 76 %).
[LCMS] : 433

### [SYNTHESIS EXAMPLE 361 Synthesis of Compound A-116

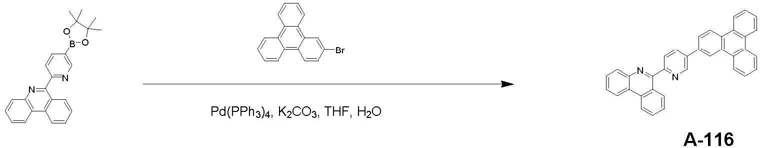

The same procedure as in Synthesis Example 1 with the exception of using 2-bromotriphenylene instead of 2-chloro-4,6-diphenyl-1,3,5-triazine was carried out to afford the title compound (6.3 g, yield 71 %).
[LCMS] : 483

### [SYNTHESIS EXAMPLE 37] Synthesis of Compound A-119

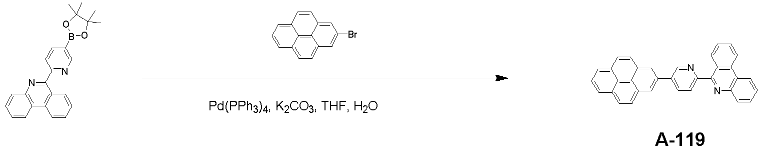

The same procedure as in Synthesis Example 1 with the exception of using 2-bromopyrene instead of 2-chloro-4,6-diphenyl-1,3,5-triazine was carried out to afford the title compound (6.1 g, yield 73 %).
[LCMS] : 457

### [SYNTHESIS EXAMPLE 38] Synthesis of Compound A-121

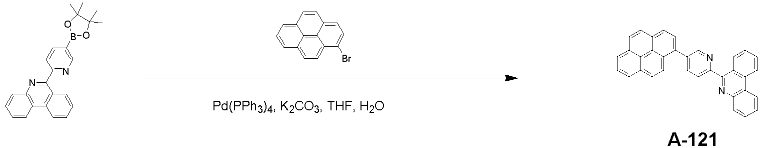

The same procedure as in Synthesis Example 1 with the exception of using 1-bromopyrene instead of 2-chloro-4,6-diphenyl-1,3,5-triazine was carried out to afford the title compound (6.1 g, yield 73 %).
[LCMS] : 457

### [SYNTHESIS EXAMPLE 39] Synthesis of Compound A-122

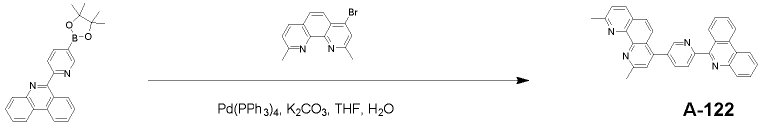

The same procedure as in Synthesis Example 1 with the exception of using 4-bromo-2,9-dimethyl-1,10-phenanthroline instead of 2-chloro-4,6-diphenyl-1,3,5-triazine was carried out to afford the title compound (6.1 g, yield 72 %).
[LCMS] : 463

### [SYNTHESIS EXAMPLE 40] Synthesis of Compound B-1

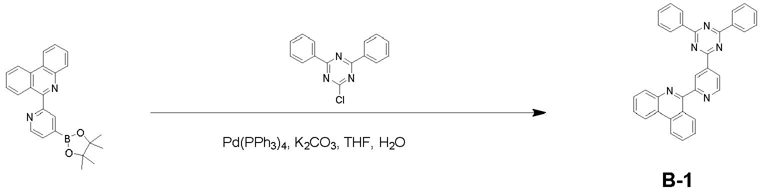

6-(4-(4,4,5,5-Tetramethyl-1,3,2-dioxaborolan-2-yl)pyridin-2-yl)phenanthridine (7 g, 18.3 mmol), 2-chloro-4,6-diphenyl-1,3,5-triazine (4.9 g, 18.3 mmol), Pd(PPh₃)₄ (1.1 g, 0.9 mmol), and K₂CO₃ (7.6 g, 54.9 mmol) in a mixture of toluene (50 ml), EtOH (10 ml), and H₂O (10 ml) were heated for 12 hour under reflux. After completion of the reaction, the reaction mixture was subjected to extraction with methylene chloride. The organic layer thus obtained was added with MgSO₄ and filtered. The solvent was removed from the filtered organic layer, followed by column chromatography to afford the title compound (7.7 g, yield 86 %).
[LCMS] : 488

### [SYNTHESIS EXAMPLE 41] Synthesis of Compound B-3

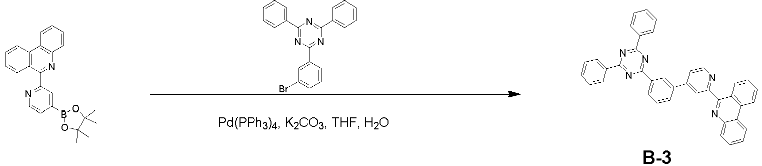

The same procedure as in Synthesis Example 40 with the exception of using 2-(3-bromophenyl)-4,6-diphenyl-1,3,5-triazine instead of 2-chloro-4,6-diphenyl-1,3,5-triazine was carried out to afford the title compound (8.5 g, yield 82 %).
[LCMS] : 564

### [SYNTHESIS EXAMPLE 42] Synthesis of Compound B-7

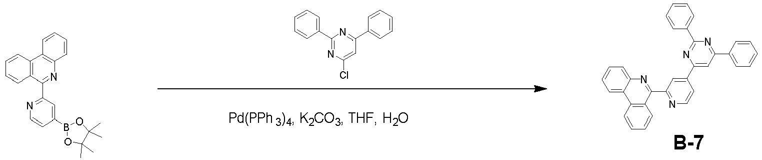

The same procedure as in Synthesis Example 40 with the exception of using 4-chloro-2,6-diphenylpyrimidine instead of 2-chloro-4,6-diphenyl-1,3,5-triazine was carried out to afford the title compound (7.6 g, yield 85 %).
[LCMS] : 487

### [SYNTHESIS EXAMPLE 43] Synthesis of Compound B-8

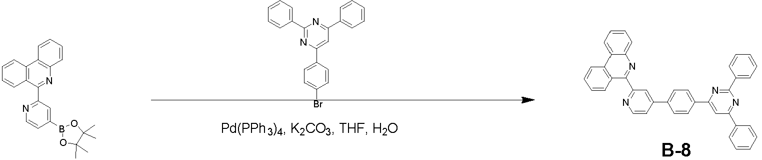

The same procedure as in Synthesis Example 40 with the exception of using 4-(4-bromophenyl)-2,6-diphenylpyrimidine instead of 2-chloro-4,6-diphenyl-1,3,5-triazine was carried out to afford the title compound (8.5 g, yield 82 %).
[LCMS] : 563

### [SYNTHESIS EXAMPLE 44] Synthesis of Compound B-13

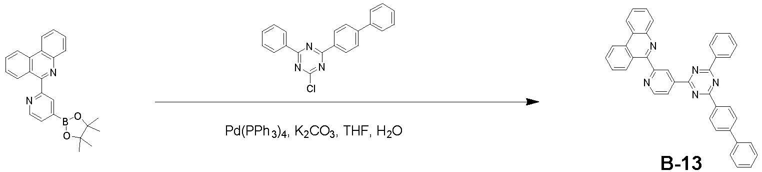

The same procedure as in Synthesis Example 40 with the exception of using 2-([1,1'-biphenyl]-4-yl)-4-chloro-6-phenyl-1,3,5-triazine instead of 2-chloro-4,6-diphenyl-1,3,5-triazine was carried out to afford the title compound (8.4 g, yield 81 %).
[LCMS] : 564

### [SYNTHESIS EXAMPLE 45] Synthesis of Compound B-19

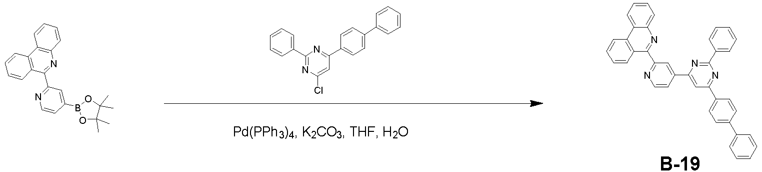

The same procedure as in Synthesis Example 40 with the exception of using 4-([1,1'-biphenyl]-4-yl)-6-chloro-2-phenylpyrimidine instead of 2-chloro-4,6-diphenyl-1,3,5-triazine was carried out to afford the title compound (8.5 g, yield 82 %).
[LCMS] : 563

### [SYNTHESIS EXAMPLE 46] Synthesis of Compound B-25

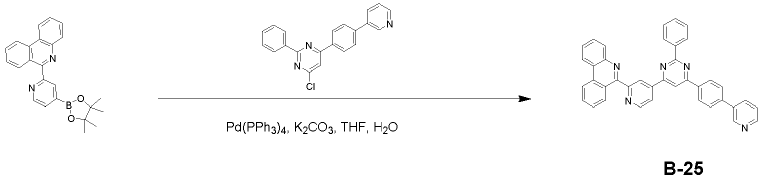

The same procedure as in Synthesis Example 40 with the exception of using 4-chloro-2-phenyl-6-(4-(pyridin-3-yl)phenyl)pyrimidine instead of 2-chloro-4,6-diphenyl-1,3,5-triazine was carried out to afford the title compound (8.4 g, yield 81 %).
[LCMS] : 564

### [SYNTHESIS EXAMPLE 47] Synthesis of Compound B-31

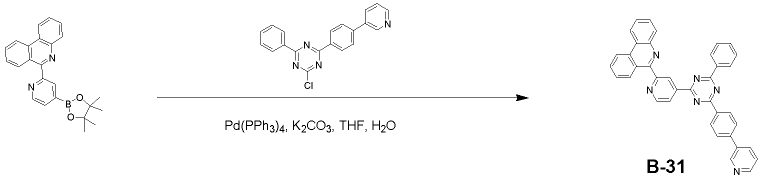

The same procedure as in Synthesis Example 40 with the exception of using 2-chloro-4-phenyl-6-(4-(pyridin-3-yl)phenyl)-1,3,5-triazine instead of 2-chloro-4,6-diphenyl-1,3,5-triazine was carried out to afford the title compound (8.2 g, yield 79 %).
[LCMS] : 565

### [SYNTHESIS EXAMPLE 48] Synthesis of Compound B-37

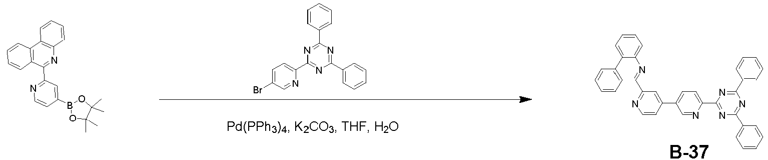

The same procedure as in Synthesis Example 40 with the exception of using 2-(5-bromopyridin-2-yl)-4,6-diphenyl-1,3,5-triazine instead of 2-chloro-4,6-diphenyl-1,3,5-triazine was carried out to afford the title compound (7.9 g, yield 76 %).
[LCMS] : 565

### [SYNTHESIS EXAMPLE 49] Synthesis of Compound B-38

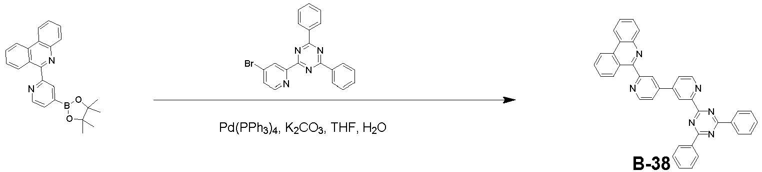

The same procedure as in Synthesis Example 40 with the exception of using 2-(4-bromopyridin-2-yl)-4,6-diphenyl-1,3,5-triazine instead of 2-chloro-4,6-diphenyl-1,3,5-triazine was carried out to afford the title compound (7.9 g, yield 76 %) .
[LCMS] : 565

### [SYNTHESIS EXAMPLE 50] Synthesis of Compound B-40

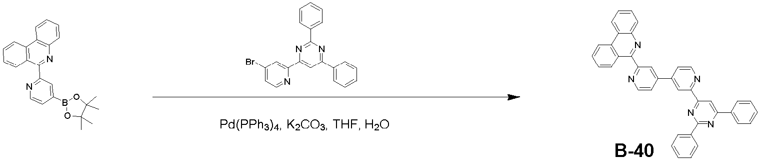

The same procedure as in Synthesis Example 40 with the exception of using 4-(4-bromopyridin-2-yl)-2,6-diphenylpyrimidine instead of 2-chloro-4,6-diphenyl-1,3,5-triazine was carried out to afford the title compound (7.8 g, yield 75 %).
[LCMS] : 564

### [SYNTHESIS EXAMPLE 51] Synthesis of Compound B-43

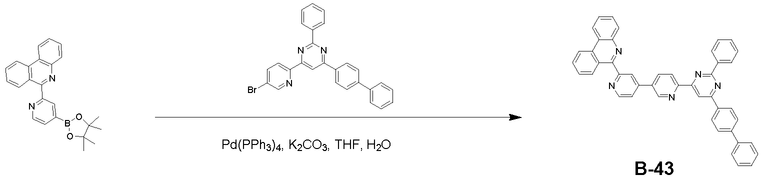

The same procedure as in Synthesis Example 40 with the exception of using 4-([1,1'-biphenyl]-4-yl)-6-(5-bromopyridin-2-yl)-2-phenylpyrimidine instead of 2-chloro-4,6-diphenyl-1,3,5-triazine was carried out to afford the title compound (8.9 g, yield 75 %).
[LCMS] : 640

### [SYNTHESIS EXAMPLE 52] Synthesis of Compound B-49

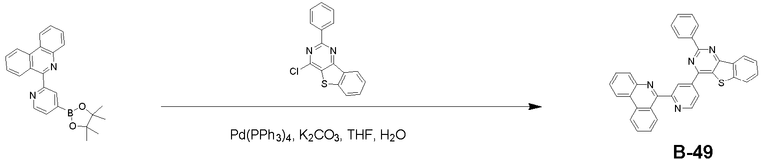

The same procedure as in Synthesis Example 40 with the exception of using 4-chloro-2-phenylbenzo[4,5]thieno[3,2-d]pyrimidine instead of 2-chloro-4,6-diphenyl-1,3,5-triazine was carried out to afford the title compound (7.5 g, yield 79 %).
[LCMS] : 517

### [SYNTHESIS EXAMPLE 53] Synthesis of Compound B-51

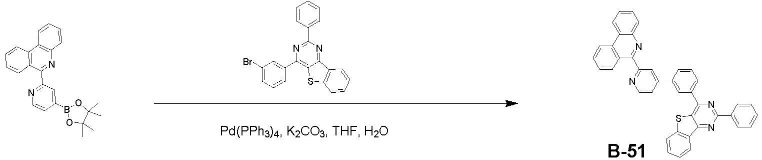

The same procedure as in Synthesis Example 40 with the exception of using 4-(3-bromophenyl)-2-phenylbenzo[4,5]thieno[3,2-d]pyrimidine instead of 2-chloro-4,6-diphenyl-1,3,5-triazine was carried out to afford the title compound (8.5 g, yield 78 %).
[LCMS] : 593

### [SYNTHESIS EXAMPLE 54] Synthesis of Compound B-52

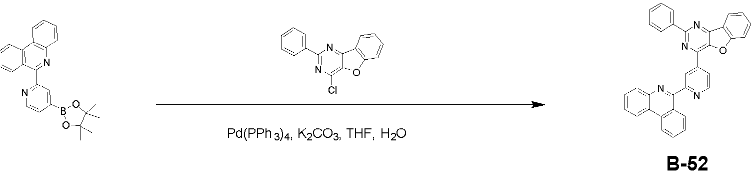

The same procedure as in Synthesis Example 40 with the exception of using 4-chloro-2-phenylbenzofuro[3,2-d]pyrimidine instead of 2-chloro-4,6-diphenyl-1,3,5-triazine was carried out to afford the title compound (7.4 g, yield 81 %).
[LCMS] : 501

### [SYNTHESIS EXAMPLE 55] Synthesis of Compound B-55

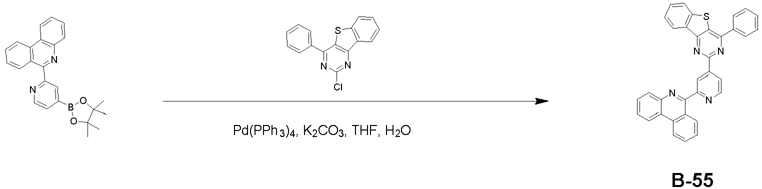

The same procedure as in Synthesis Example 40 with the exception of using 2-chloro-4-phenylbenzo[4,5]thieno[3,2-d]pyrimidine instead of 2-chloro-4,6-diphenyl-1,3,5-triazine was carried out to afford the title compound (7.7 g, yield 81 %).
[LCMS] : 517

### [SYNTHESIS EXAMPLE 56] Synthesis of Compound B-56

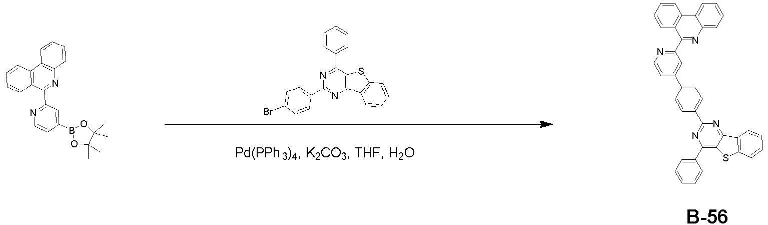

The same procedure as in Synthesis Example 40 with the exception of using 2-(4-bromophenyl)-4-phenylbenzo[4,5]thieno[3,2-d]pyrimidine instead of 2-chloro-4,6-diphenyl-1,3,5-triazine was carried out to afford the title compound (8.5 g, yield 78 %).
[LCMS] : 593

### [SYNTHESIS EXAMPLE 57] Synthesis of Compound B-61

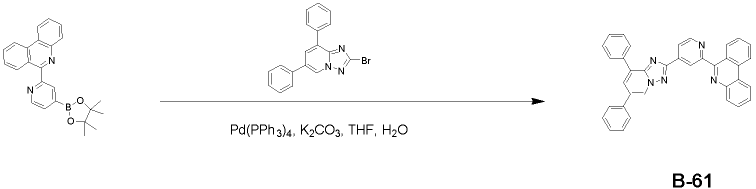

The same procedure as in Synthesis Example 40 with the exception of using 2-bromo-6,8-diphenyl-[1,2,4]triazolo[1,5-a]pyridine instead of 2-chloro-4,6-diphenyl-1,3,5-triazine was carried out to afford the title compound (7.5 g, yield 78 %).
[LCMS] : 526

### [SYNTHESIS EXAMPLE 58] Synthesis of Compound B-70

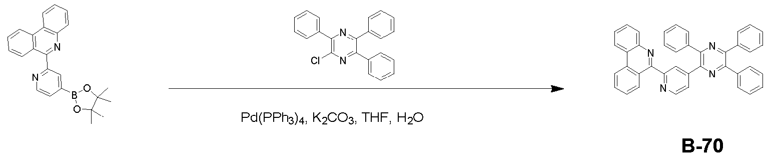

The same procedure as in Synthesis Example 40 with the exception of using 2-chloro-3,5,6-triphenylpyrazine instead of 2-chloro-4,6-diphenyl-1,3,5-triazine was carried out to afford the title compound (7.9 g, yield 76 %) .
[LCMS] : 563

### [SYNTHESIS EXAMPLE 59] Synthesis of Compound B-72

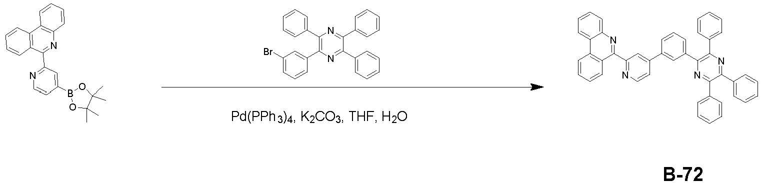

The same procedure as in Synthesis Example 40 with the exception of using 2-(3-bromophenyl)-3,5,6-triphenylpyrazine instead of 2-chloro-4,6-diphenyl-1,3,5-triazine was carried out to afford the title compound (9.1 g, yield 77 %).
[LCMS] : 639

### [SYNTHESIS EXAMPLE 60] Synthesis of Compound B-73

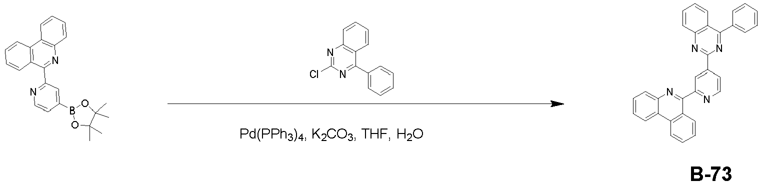

The same procedure as in Synthesis Example 40 with the exception of using 2-chloro-4-phenylquinazoline instead of 2-chloro-4,6-diphenyl-1,3,5-triazine was carried out to afford the title compound (6.6 g, yield 78 %).
[LCMS] : 461

### [SYNTHESIS EXAMPLE 61] Synthesis of Compound B-75

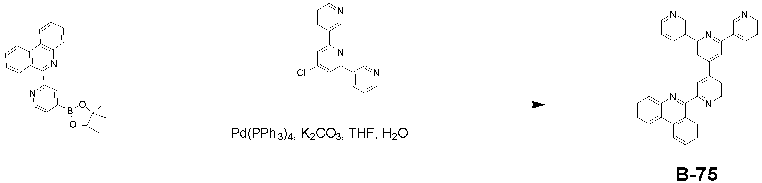

The same procedure as in Synthesis Example 40 with the exception of using 4'-chloro-3,2':6',3"-terpyridine instead of 2-chloro-4,6-diphenyl-1,3,5-triazine was carried out to afford the title compound (6.9 g, yield 77 %) .
[LCMS] : 488

### [SYNTHESIS EXAMPLE 62] Synthesis of Compound B-79

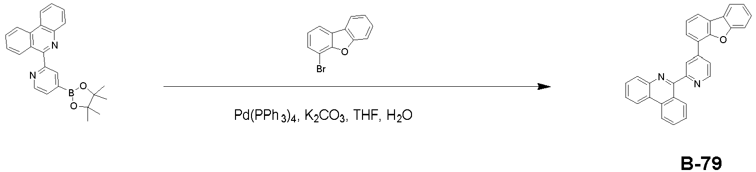

The same procedure as in Synthesis Example 40 with the exception of using 4-bromodibenzo[b,d]furan instead of 2-chloro-4,6-diphenyl-1,3,5-triazine was carried out to afford the title compound (6.2 g, yield 80 %).
[LCMS] : 423

### [SYNTHESIS EXAMPLE 63] Synthesis of Compound B-81

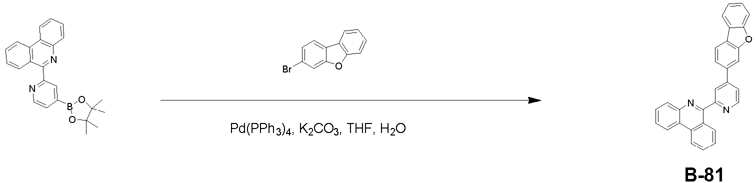

The same procedure as in Synthesis Example 40 with the exception of using 3-bromodibenzo[b,d]furan instead of 2-chloro-4,6-diphenyl-1,3,5-triazine was carried out to afford the title compound (6.2 g, yield 80 %).
[LCMS] : 423

### [SYNTHESIS EXAMPLE 64] Synthesis of Compound B-82

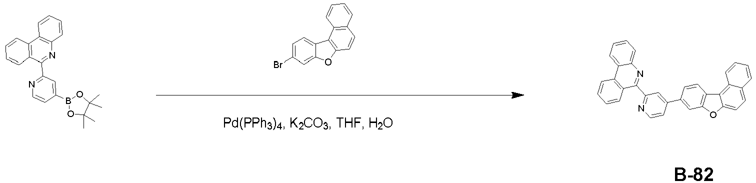

The same procedure as in Synthesis Example 40 with the exception of using 9-bromonaphtho[2,1-b]benzofuran instead of 2-chloro-4,6-diphenyl-1,3,5-triazine was carried out to afford the title compound (6.6 g, yield 76 %) .
[LCMS] : 473

### [SYNTHESIS EXAMPLE 65] Synthesis of Compound B-88

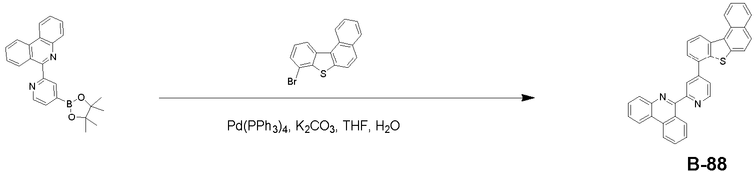

The same procedure as in Synthesis Example 40 with the exception of using 8-bromobenzo[b]naphtho[1,2-d]thiophene instead of 2-chloro-4,6-diphenyl-1,3,5-triazine was carried out to afford the title compound (6.8 g, yield 76 %).
[LCMS] : 489

### [SYNTHESIS EXAMPLE 66] Synthesis of Compound B-89

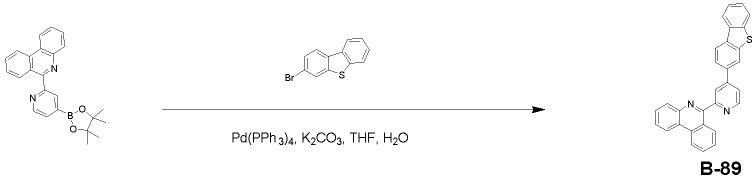

The same procedure as in Synthesis Example 40 with the exception of using 3-bromodibenzo[b,d]thiophene instead of 2-chloro-4,6-diphenyl-1,3,5-triazine was carried out to afford the title compound (6.3 g, yield 79 %) .
[LCMS] : 439

### [SYNTHESIS EXAMPLE 67] Synthesis of Compound B-96

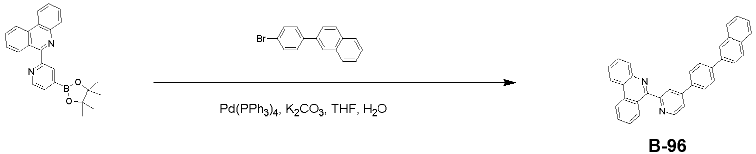

The same procedure as in Synthesis Example 40 with the exception of using 2-(4-bromophenyl)naphthalene instead of 2-chloro-4,6-diphenyl-1,3,5-triazine was carried out to afford the title compound (6.6 g, yield 79 %) .
[LCMS] : 459

### [SYNTHESIS EXAMPLE 68] Synthesis of Compound B-97

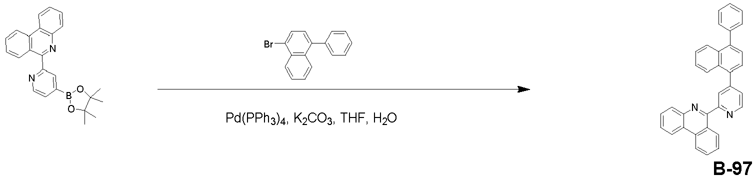

The same procedure as in Synthesis Example 40 with the exception of using 1-bromo-4-phenylnaphthalene instead of 2-chloro-4,6-diphenyl-1,3,5-triazine was carried out to afford the title compound (6.5 g, yield 77 %).
[LCMS] : 459

### [SYNTHESIS EXAMPLE 69] Synthesis of Compound B-100

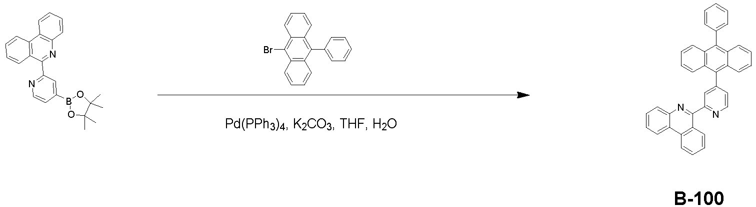

The same procedure as in Synthesis Example 40 with the exception of using 9-bromo-10-phenylanthracene instead of 2-chloro-4,6-diphenyl-1,3,5-triazine was carried out to afford the title compound (6.8 g, yield 73 %).
[LCMS] : 509

### [SYNTHESIS EXAMPLE 70] Synthesis of Compound B-104

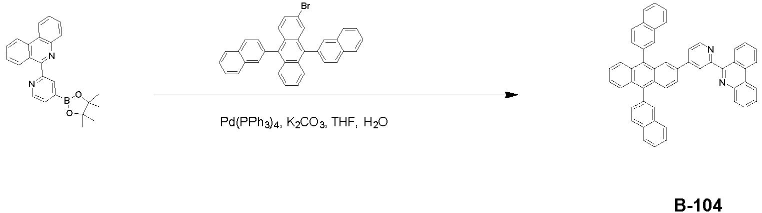

The same procedure as in Synthesis Example 40 with the exception of using 2-bromo-9,10-di(naphthalen-2-yl)anthracene instead of 2-chloro-4,6-diphenyl-1,3,5-triazine was carried out to afford the title compound (9.2 g, yield 73 %).
[LCMS] : 685

### [SYNTHESIS EXAMPLE 71] Synthesis of Compound B-106

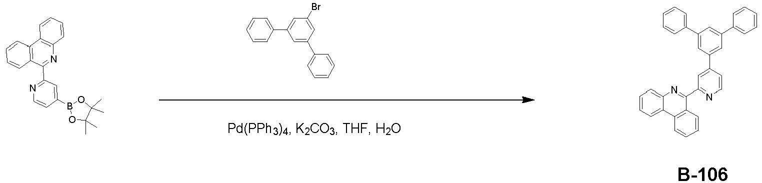

The same procedure as in Synthesis Example 40 with the exception of using 5'-bromo-1,1':3',1"-terphenyl instead of 2-chloro-4,6-diphenyl-1,3,5-triazine was carried out to afford the title compound (6.7 g, yield 75 %).
[LCMS] : 485

### [SYNTHESIS EXAMPLE 72] Synthesis of Compound B-110

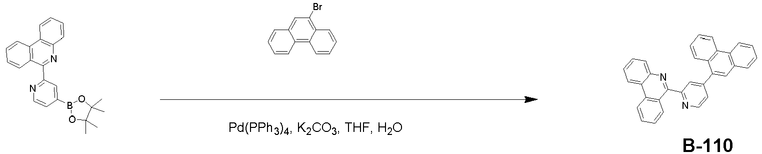

The same procedure as in Synthesis Example 40 with the exception of using 9-bromophenanthrene instead of 2-chloro-4,6-diphenyl-1,3,5-triazine was carried out to afford the title compound (5.9 g, yield 75 %).
[LCMS] : 433

### [SYNTHESIS EXAMPLE 73] Synthesis of Compound B-112

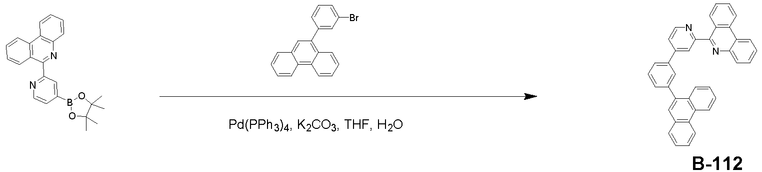

The same procedure as in Synthesis Example 40 with the exception of using 9-(3-bromophenyl)phenanthrene instead of 2-chloro-4,6-diphenyl-1,3,5-triazine was carried out to afford the title compound (6.9 g, yield 74 %) .
[LCMS] : 509

### [SYNTHESIS EXAMPLE 74] Synthesis of Compound B-113

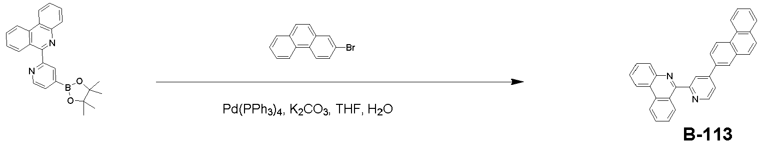

The same procedure as in Synthesis Example 40 with the exception of using 2-bromophenanthrene instead of 2-chloro-4,6-diphenyl-1,3,5-triazine was carried out to afford the title compound (5.9 g, yield 75 %).
[LCMS] : 433

### [SYNTHESIS EXAMPLE 75] Synthesis of Compound B-116

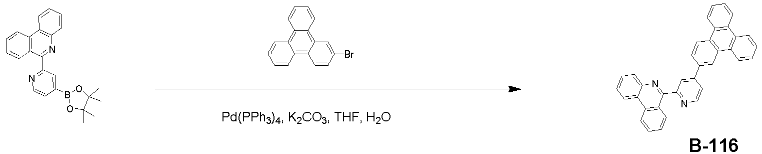

The same procedure as in Synthesis Example 40 with the exception of using 2-bromotriphenylene instead of 2-chloro-4,6-diphenyl-1,3,5-triazine was carried out to afford the title compound (6.2 g, yield 70 %).
[LCMS] : 483

### [SYNTHESIS EXAMPLE 76] Synthesis of Compound B-119

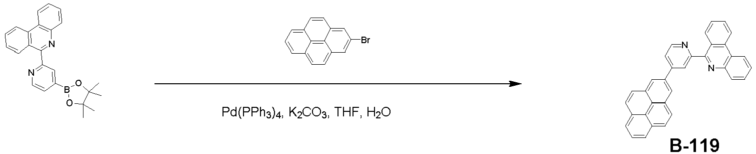

The same procedure as in Synthesis Example 40 with the exception of using 2-bromopyrene instead of 2-chloro-4,6-diphenyl-1,3,5-triazine was carried out to afford the title compound (6.0 g, yield 72 %).
[LCMS] : 457

### [SYNTHESIS EXAMPLE 77] Synthesis of Compound B-121

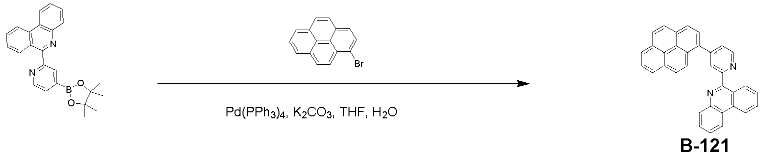

The same procedure as in Synthesis Example 40 with the exception of using 1-bromopyrene instead of 2-chloro-4,6-diphenyl-1,3,5-triazine was carried out to afford the title compound (6.0 g, yield 72 %).
[LCMS] : 457

### [SYNTHESIS EXAMPLE 78] Synthesis of Compound B-122

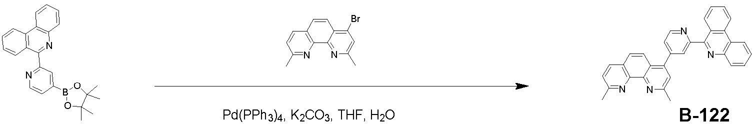

The same procedure as in Synthesis Example 40 with the exception of using 4-bromo-2,9-dimethyl-1,10-phenanthroline instead of 2-chloro-4,6-diphenyl-1,3,5-triazine was carried out to afford the title compound (6.0 g, yield 71 %).
[LCMS] : 463

### [SYNTHESIS EXAMPLE 79] Synthesis of Compound C-1

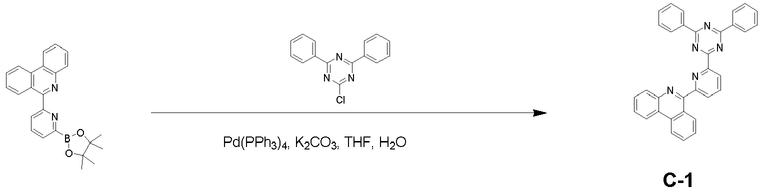

6-(6-(4,4,5,5-tetramethyl-1,3,2-dioxaborolan-2-yl)pyridin-2-yl)phenanthridine (7 g, 18.3 mmol), 2-chloro-4,6-diphenyl-1,3,5-triazine (4.9 g, 18.3 mmol), Pd(PPh₃)₄ (1.1 g, 0.9 mmol), and K₂CO₃ (7.6 g, 54.9 mmol) in a mixture of toluene (50 ml), EtOH (10 ml), and H₂O (10 ml) were heated for 12 hour under reflux. After completion of the reaction, the reaction mixture was subjected to extraction with methylene chloride. The organic layer thus obtained was added with MgSO₄ and filtered. The solvent was removed from the filtered organic layer, followed by column chromatography to afford the title compound (7.5 g, yield 84 %).
[LCMS] : 488

### [SYNTHESIS EXAMPLE 80] Synthesis of Compound C-3

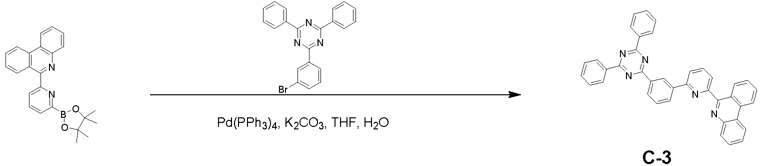

The same procedure as in Synthesis Example 79 with the exception of using 2-(3-bromophenyl)-4,6-diphenyl-1,3,5-triazine instead of 2-chloro-4,6-diphenyl-1,3,5-triazine was carried out to afford the title compound (8.7 g, yield 84 %).
[LCMS] : 564

### [SYNTHESIS EXAMPLE 81] Synthesis of Compound C-7

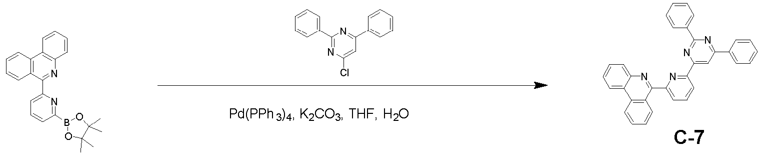

The same procedure as in Synthesis Example 79 with the exception of using 4-chloro-2,6-diphenylpyrimidine instead of 2-chloro-4,6-diphenyl-1,3,5-triazine was carried out to afford the title compound (7.5 g, yield 84 %).
[LCMS] : 487

### [SYNTHESIS EXAMPLE 82] Synthesis of Compound C-8

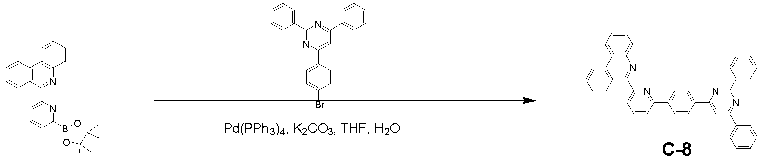

The same procedure as in Synthesis Example 79 with the exception of using 4-(4-bromophenyl)-2,6-diphenylpyrimidine instead of 2-chloro-4,6-diphenyl-1,3,5-triazine was carried out to afford the title compound (8.7 g, yield 84 %).
[LCMS] : 563

### [SYNTHESIS EXAMPLE 83] Synthesis of Compound C-13

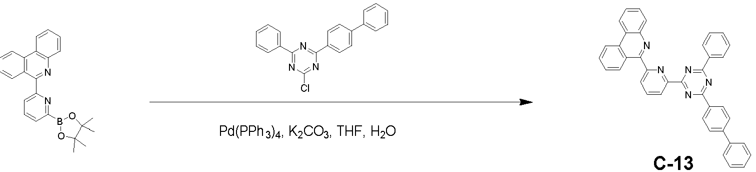

The same procedure as in Synthesis Example 79 with the exception of using 2-([1,1'-biphenyl]-4-yl)-4-chloro-6-phenyl-1,3,5-triazine instead of 2-chloro-4,6-diphenyl-1,3,5-triazine was carried out to afford the title compound (8.3 g, yield 80 %).
[LCMS] : 564

### [SYNTHESIS EXAMPLE 84] Synthesis of Compound C-19

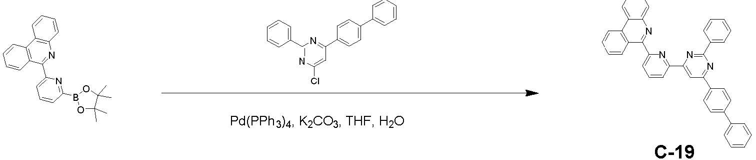

The same procedure as in Synthesis Example 79 with the exception of using 4-([1,1'-biphenyl]-4-yl)-6-chloro-2-phenylpyrimidine instead of 2-chloro-4,6-diphenyl-1,3,5-triazine was carried out to afford the title compound (8.7 g, yield 84 %).
[LCMS] : 563

### [SYNTHESIS EXAMPLE 85] Synthesis of Compound C-25

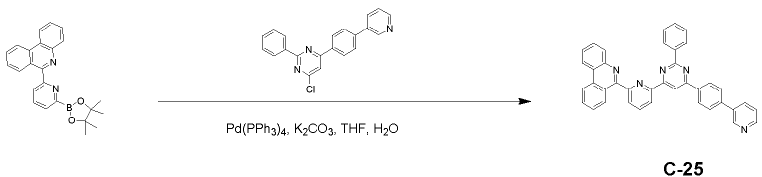

The same procedure as in Synthesis Example 79 with the exception of using 4-chloro-2-phenyl-6-(4-(pyridin-3-yl)phenyl)pyrimidine instead of 2-chloro-4,6-diphenyl-1,3,5-triazine was carried out to afford the title compound (8.3 g, yield 80 %).
[LCMS] : 564

### [SYNTHESIS EXAMPLE 86] Synthesis of Compound C-31

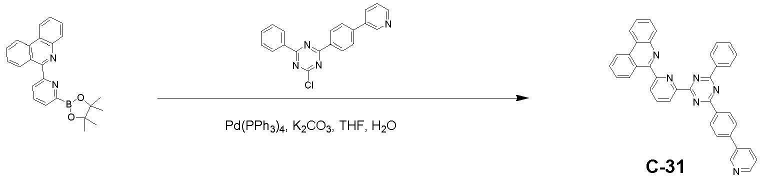

The same procedure as in Synthesis Example 79 with the exception of using 2-chloro-4-phenyl-6-(4-(pyridin-3-yl)phenyl)-1,3,5-triazine instead of 2-chloro-4,6-diphenyl-1,3,5-triazine was carried out to afford the title compound (8.3 g, yield 80 %).
[LCMS] : 565

### [SYNTHESIS EXAMPLE 87] Synthesis of Compound C-37

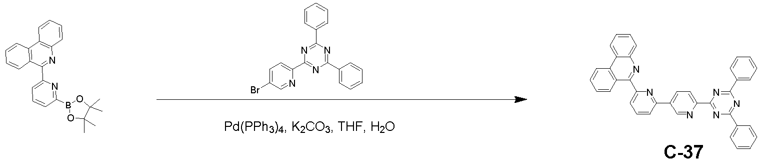

The same procedure as in Synthesis Example 79 with the exception of using 2-(5-bromopyridin-2-yl)-4,6-diphenyl-1,3,5-triazine instead of 2-chloro-4,6-diphenyl-1,3,5-triazine was carried out to afford the title compound (7.8 g, yield 71 %).
[LCMS] : 565

### [SYNTHESIS EXAMPLE 88] Synthesis of Compound C-38

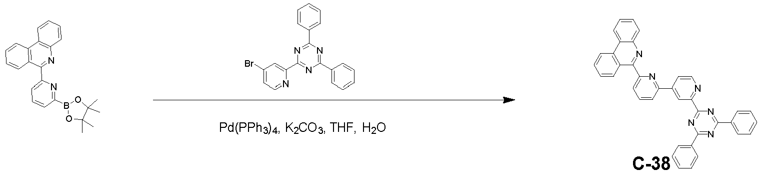

The same procedure as in Synthesis Example 79 with the exception of using 2-(4-bromopyridin-2-yl)-4,6-diphenyl-1,3,5-triazine instead of 2-chloro-4,6-diphenyl-1,3,5-triazine was carried out to afford the title compound (8.1 g, yield 78 %).
[LCMS] : 565

### [SYNTHESIS EXAMPLE 89] Synthesis of Compound C-40

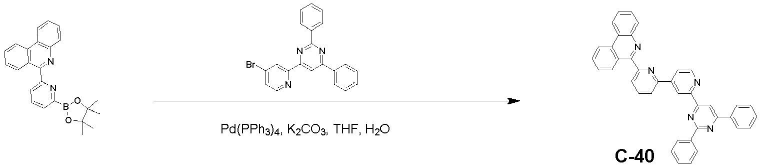

The same procedure as in Synthesis Example 79 with the exception of using 4-(4-bromopyridin-2-yl)-2,6-diphenylpyrimidine instead of 2-chloro-4,6-diphenyl-1,3,5-triazine was carried out to afford the title compound (8.0 g, yield 77 %).
[LCMS] : 564

### [SYNTHESIS EXAMPLE 90] Synthesis of Compound C-43

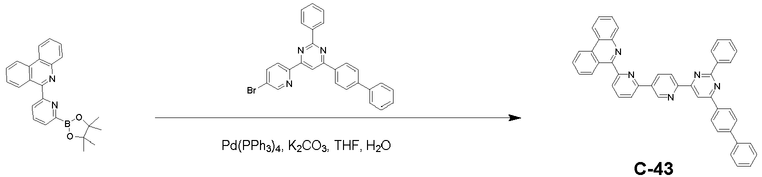

The same procedure as in Synthesis Example 79 with the exception of using 4-([1,1'-biphenyl]-4-yl)-6-(5-bromopyridin-2-yl)-2-phenylpyrimidine instead of 2-chloro-4,6-diphenyl-1,3,5-triazine was carried out to afford the title compound (8.8 g, yield 75 %).
[LCMS] : 640

### [SYNTHESIS EXAMPLE 91] Synthesis of Compound C-49

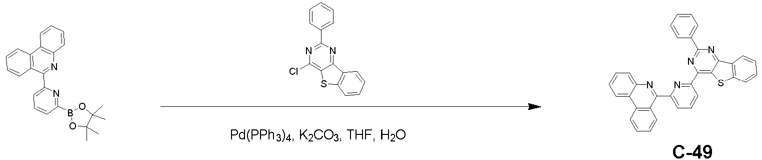

The same procedure as in Synthesis Example 79 with the exception of using 4-chloro-2-phenylbenzo[4,5]thieno[3,2-d]pyrimidine instead of 2-chloro-4,6-diphenyl-1,3,5-triazine was carried out to afford the title compound (7.4 g, yield 78 %).
[LCMS] : 517

### [SYNTHESIS EXAMPLE 92] Synthesis of Compound C-51

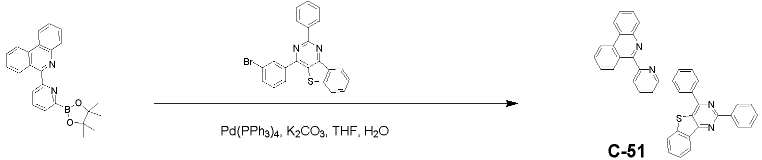

The same procedure as in Synthesis Example 79 with the exception of using 4-(3-bromophenyl)-2-phenylbenzo[4,5]thieno[3,2-d]pyrimidine instead of 2-chloro-4,6-diphenyl-1,3,5-triazine was carried out to afford the title compound (8.7 g, yield 80 %).
[LCMS] : 593

### [SYNTHESIS EXAMPLE 93] Synthesis of Compound C-52

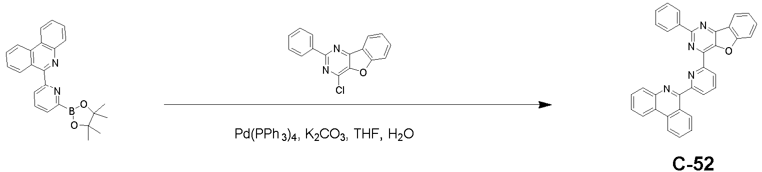

The same procedure as in Synthesis Example 79 with the exception of using 4-chloro-2-phenylbenzofuro[3,2-d]pyrimidine instead of 2-chloro-4,6-diphenyl-1,3,5-triazine was carried out to afford the title compound (7.3 g, yield 80 %).
[LCMS] : 501

### [SYNTHESIS EXAMPLE 94] Synthesis of Compound C-55

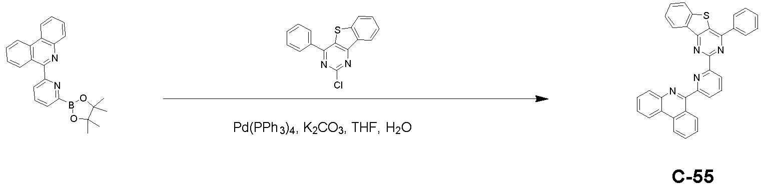

The same procedure as in Synthesis Example 79 with the exception of using 2-chloro-4-phenylbenzo[4,5]thieno[3,2-d]pyrimidine instead of 2-chloro-4,6-diphenyl-1,3,5-triazine was carried out to afford the title compound (7.8 g, yield 82 %).
[LCMS] : 517

### [SYNTHESIS EXAMPLE 95] Synthesis of Compound C-56

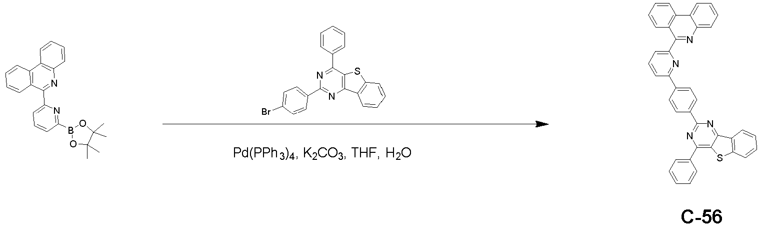

The same procedure as in Synthesis Example 79 with the exception of using 2-(4-bromophenyl)-4-phenylbenzo[4,5]thieno[3,2-d]pyrimidine instead of 2-chloro-4,6-diphenyl-1,3,5-triazine was carried out to afford the title compound (8.6 g, yield 79 %).
[LCMS] : 593

### [SYNTHESIS EXAMPLE 96] Synthesis of Compound C-61

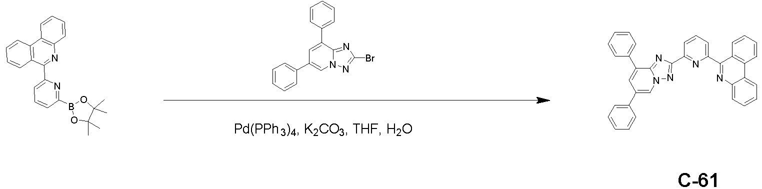

The same procedure as in Synthesis Example 79 with the exception of using 2-bromo-6,8-diphenyl-[1,2,4]triazolo[1,5-a]pyridine instead of 2-chloro-4,6-diphenyl-1,3,5-triazine was carried out to afford the title compound (7.7 g, yield 80 %).
[LCMS] : 526

### [SYNTHESIS EXAMPLE 97] Synthesis of Compound C-70

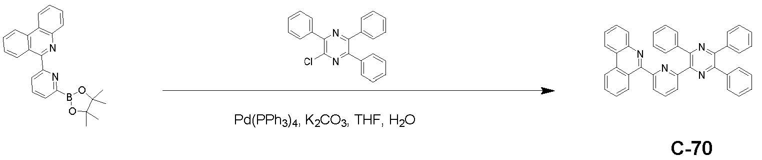

The same procedure as in Synthesis Example 79 with the exception of using 2-chloro-3,5,6-triphenylpyrazine instead of 2-chloro-4,6-diphenyl-1,3,5-triazine was carried out to afford the title compound (8.0 g, yield 77 %) .
[LCMS] : 563

### [SYNTHESIS EXAMPLE 98] Synthesis of Compound C-72

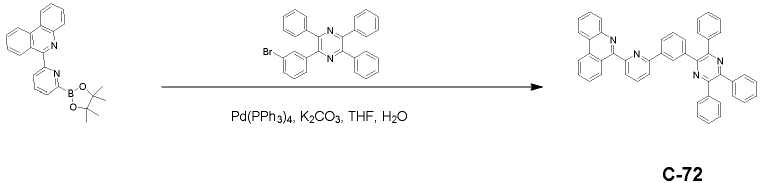

The same procedure as in Synthesis Example 79 with the exception of using 2-(3-bromophenyl)-3,5,6-triphenylpyrazine instead of 2-chloro-4,6-diphenyl-1,3,5-triazine was carried out to afford the title compound (9.0 g, yield 77 %).
[LCMS] : 639

### [SYNTHESIS EXAMPLE 99] Synthesis of Compound C-73

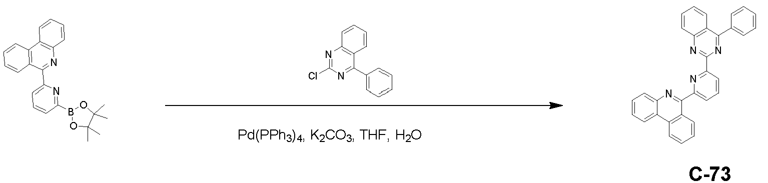

The same procedure as in Synthesis Example 79 with the exception of using 2-chloro-4-phenylquinazoline instead of 2-chloro-4,6-diphenyl-1,3,5-triazine was carried out to afford the title compound (6.5 g, yield 77 %) .
[LCMS] : 461

### [SYNTHESIS EXAMPLE 100] Synthesis of Compound C-75

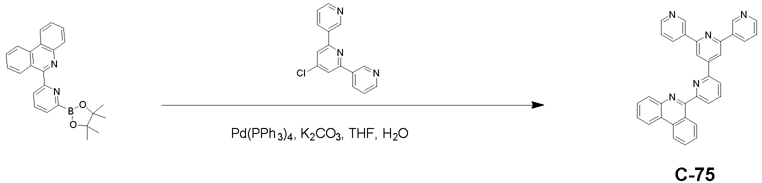

The same procedure as in Synthesis Example 79 with the exception of using 4'-chloro-3,2':6',3"-terpyridine instead of 2-chloro-4,6-diphenyl-1,3,5-triazine was carried out to afford the title compound (7.0 g, yield 78 %).
[LCMS] : 488

### [SYNTHESIS EXAMPLE 101] Synthesis of Compound C-79

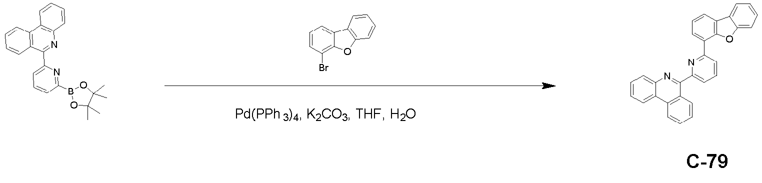

The same procedure as in Synthesis Example 79 with the exception of using 4-bromodibenzo[b,d]furan instead of 2-chloro-4,6-diphenyl-1,3,5-triazine was carried out to afford the title compound (6.4 g, yield 82 %).
[LCMS] : 423

### [SYNTHESIS EXAMPLE 102] Synthesis of Compound C-81

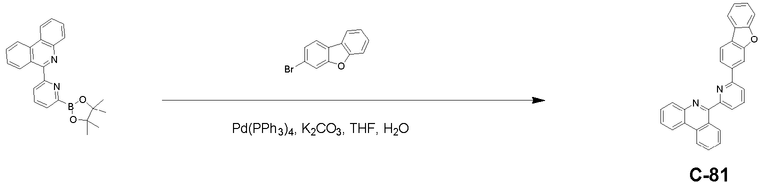

The same procedure as in Synthesis Example 79 with the exception of using 3-bromodibenzo[b,d]furan instead of 2-chloro-4,6-diphenyl-1,3,5-triazine was carried out to afford the title compound (6.3 g, yield 81 %).
[LCMS] : 423

### [SYNTHESIS EXAMPLE 103] Synthesis of Compound C-82

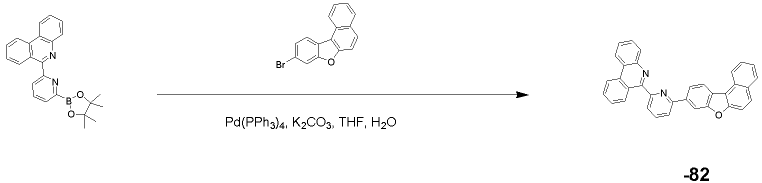

The same procedure as in Synthesis Example 79 with the exception of using 9-bromonaphtho[2,1-b]benzofuran instead of 2-chloro-4,6-diphenyl-1,3,5-triazine was carried out to afford the title compound (6.5 g, yield 75 %).
[LCMS] : 473

### [SYNTHESIS EXAMPLE 104] Synthesis of Compound C-88

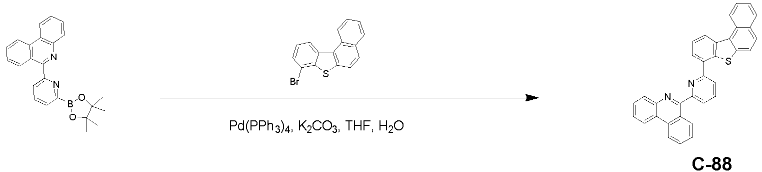

The same procedure as in Synthesis Example 79 with the exception of using 8-bromobenzo[b]naphtho[1,2-d]thiophene instead of 2-chloro-4,6-diphenyl-1,3,5-triazine was carried out to afford the title compound (6.7 g, yield 75 %).
[LCMS] : 489

### [SYNTHESIS EXAMPLE 105] Synthesis of Compound C-89

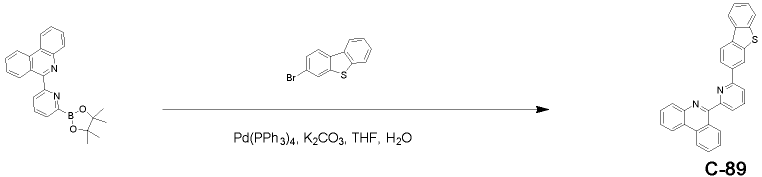

The same procedure as in Synthesis Example 79 with the exception of using 3-bromodibenzo[b,d]thiophene instead of 2-chloro-4,6-diphenyl-1,3,5-triazine was carried out to afford the title compound (6.4 g, yield 80 %).
[LCMS] : 439

### [SYNTHESIS EXAMPLE 106] Synthesis of Compound C-96

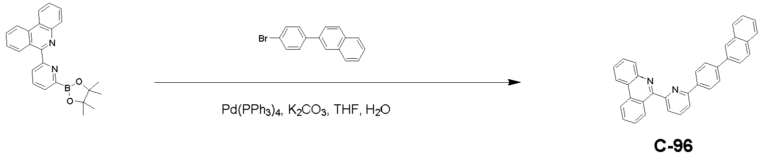

The same procedure as in Synthesis Example 79 with the exception of using 2-(4-bromophenyl)naphthalene instead of 2-chloro-4,6-diphenyl-1,3,5-triazine was carried out to afford the title compound (6.8 g, yield 81 %).
[LCMS] : 459

### [SYNTHESIS EXAMPLE 107] Synthesis of Compound C-97

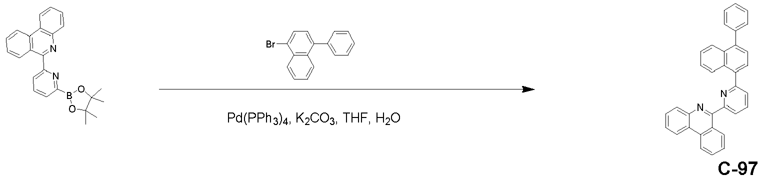

The same procedure as in Synthesis Example 79 with the exception of using 1-bromo-4-phenylnaphthalene instead of 2-chloro-4,6-diphenyl-1,3,5-triazine was carried out to afford the title compound (6.7 g, yield 79 %).
[LCMS] : 459

### [SYNTHESIS EXAMPLE 108] Synthesis of Compound C-100

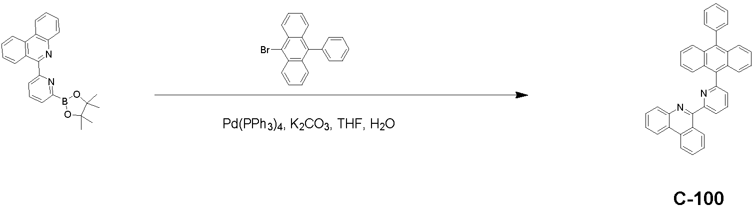

The same procedure as in Synthesis Example 79 with the exception of using 9-bromo-10-phenylanthracene instead of 2-chloro-4,6-diphenyl-1,3,5-triazine was carried out to afford the title compound (6.9 g, yield 74 %).
[LCMS] : 509

### [SYNTHESIS EXAMPLE 109] Synthesis of Compound C-104

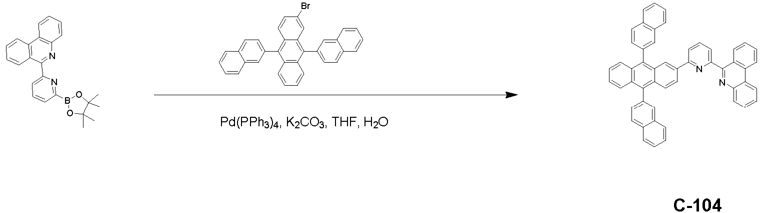

The same procedure as in Synthesis Example 79 with the exception of using 2-bromo-9,10-di(naphthalen-2-yl)anthracene instead of 2-chloro-4,6-diphenyl-1,3,5-triazine was carried out to afford the title compound (9.3 g, yield 74 %).
[LCMS] : 685

### [SYNTHESIS EXAMPLE 110] Synthesis of Compound C-106

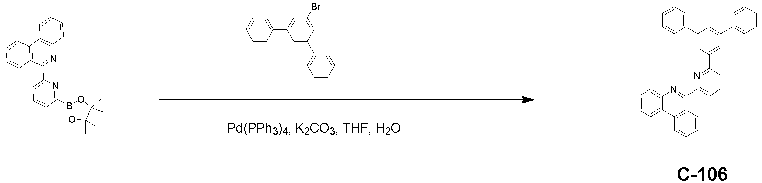

The same procedure as in Synthesis Example 79 with the exception of using 5'-bromo-1,1':3',1"-terphenyl instead of 2-chloro-4,6-diphenyl-1,3,5-triazine was carried out to afford the title compound (6.6 g, yield 74 %) .
[LCMS] : 485

### [SYNTHESIS EXAMPLE 111] Synthesis of Compound C-110

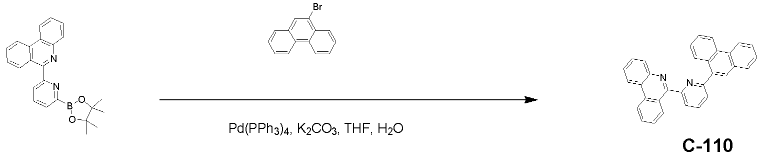

The same procedure as in Synthesis Example 79 with the exception of using 9-bromophenanthrene instead of 2-chloro-4,6-diphenyl-1,3,5-triazine was carried out to afford the title compound (6.1 g, yield 77 %).
[LCMS] : 433

### [SYNTHESIS EXAMPLE 112] Synthesis of Compound C-112

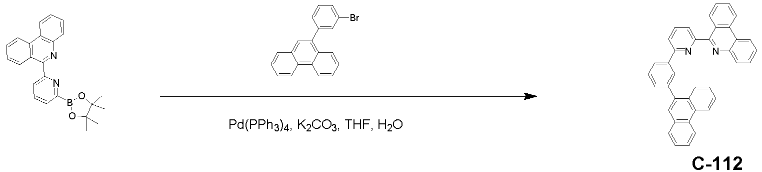

The same procedure as in Synthesis Example 79 with the exception of using 9-(3-bromophenyl)phenanthrene instead of 2-chloro-4,6-diphenyl-1,3,5-triazine was carried out to afford the title compound (7.1 g, yield 76 %) .
[LCMS] : 509

### [SYNTHESIS EXAMPLE 113] Synthesis of Compound C-113

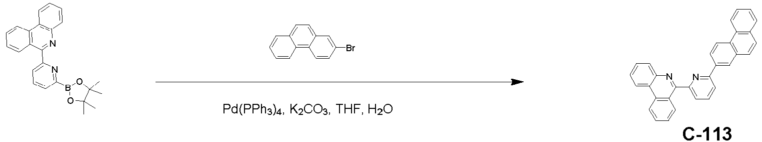

The same procedure as in Synthesis Example 79 with the exception of using 2-bromophenanthrene instead of 2-chloro-4,6-diphenyl-1,3,5-triazine was carried out to afford the title compound (6.0 g, yield 76 %).
[LCMS] : 433

### [SYNTHESIS EXAMPLE 114] Synthesis of Compound C-116

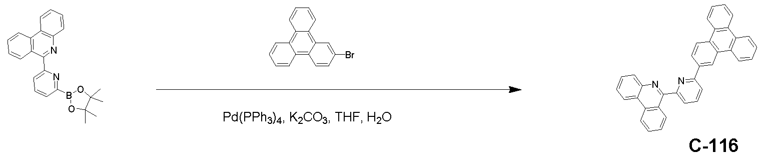

The same procedure as in Synthesis Example 79 with the exception of using 2-bromotriphenylene instead of 2-chloro-4,6-diphenyl-1,3,5-triazine was carried out to afford the title compound (6.4 g, yield 72 %).
[LCMS] : 483

### [SYNTHESIS EXAMPLE 115] Synthesis of Compound C-119

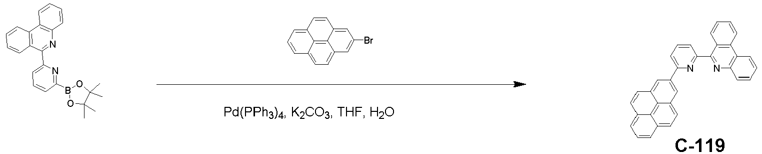

The same procedure as in Synthesis Example 79 with the exception of using 2-bromopyrene instead of 2-chloro-4,6-diphenyl-1,3,5-triazine was carried out to afford the title compound (6.1 g, yield 73 %).
[LCMS] : 457

### [SYNTHESIS EXAMPLE 116] Synthesis of Compound C-121

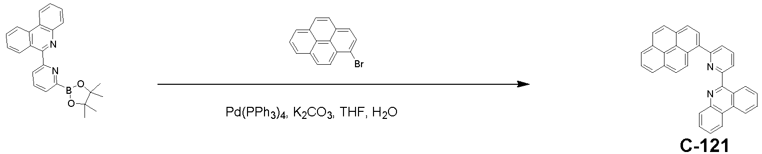

The same procedure as in Synthesis Example 79 with the exception of using 1-bromopyrene instead of 2-chloro-4,6-diphenyl-1,3,5-triazine was carried out to afford the title compound (6.2 g, yield 74 %).
[LCMS] : 457

### [SYNTHESIS EXAMPLE 117] Synthesis of Compound C-122

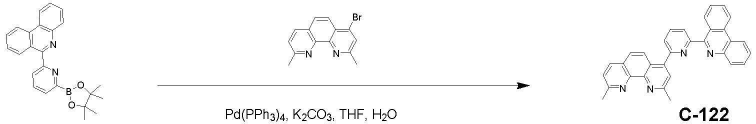

The same procedure as in Synthesis Example 79 with the exception of using 4-bromo-2,9-dimethyl-1,10-phenanthroline instead of 2-chloro-4,6-diphenyl-1,3,5-triazine was carried out to afford the title compound (6.1 g, yield 72 %).
[LCMS] : 463

### [EXAMPLE 1] Fabrication of Blue Organic EL Device

After Compound A-1 synthesized in the Synthesis Example was isolated to a high purity through sublimation, a blue organic EL device was fabricated using same as follows.

First, a glass substrate coated with 1,200 Å-thick indium tin oxide (ITO) thin film was cleansed by ultrasonication in distilled water. Following washing with distilled water, the substrate was washed with a solvent such as isopropyl alcohol, acetone, methanol, etc. under ultrasonication. Thereafter, the substrate was transferred to a UV OZONE cleaner (Power sonic 405, Hwashin Tech), cleaned for 5 minutes using UV, and then moved to a vacuum evaporator.

On the transparent ITO electrode prepared above, Compounds 1 and 2 were co-deposited at a weight ratio of 98:2 to form a 100 Å-thick hole injection on which Compound 1 was then deposited at a thickness of 1,400Å to form a hole transport layer. A hole transport auxiliary layer 50 Å thick was formed by deposition of Compound 3 on the hole transport layer, followed by co-depositing Compounds 4 and 5 at a weight ratio of 98:2 to form a light-emitting layer 200Å thick. An electron transport auxiliary layer was formed by depositing Compound 6 at a thickness of 50Å on the light-emitting layer, followed by co-depositing Compounds A-1 and 7 at a weight ratio of 1:1 to form an electron transport layer 300Å thick. Deposition of LiF on the electron transport layer formed a 10 Å-thick electron injection layer which was then coated with Al to form a 1,000 Å-thick cathode, leading to the fabrication of an organic EL device.

Structures of Compounds 1 to 7 used for the fabrication are as follows.

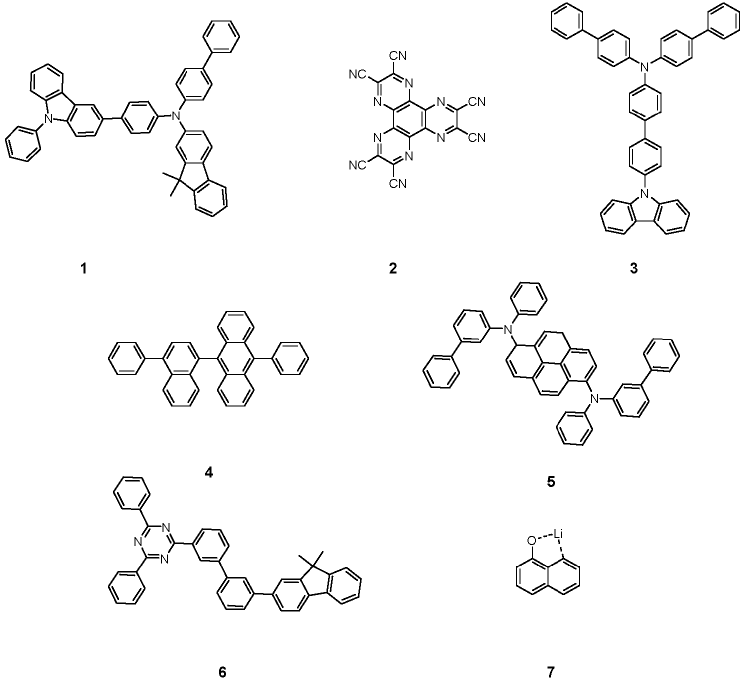

### [EXAMPLES 2 TO 117] Fabrication of Blue Organic EL Devices

Blue organic EL devices were fabricated in the same manner as in Example 1 with the exception of using the compounds listed in the following Table 1, instead of Compound A-1, as electron transport layer materials, respectively.

### [COMPARATIVE EXAMPLES 1 TO 12] Fabrication of Blue Organic EL Devices

Blue organic EL devices were fabricated in the same manner as in Example 1 with the exception of using Compounds A to L, instead of Compound A-1, as electron transport layer materials, respectively.

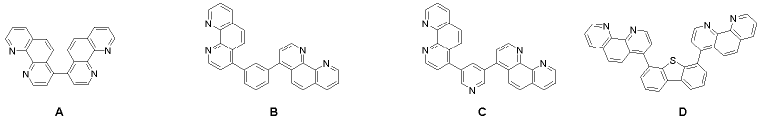

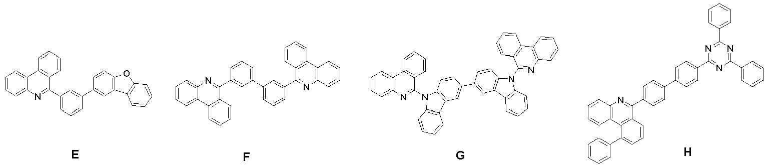

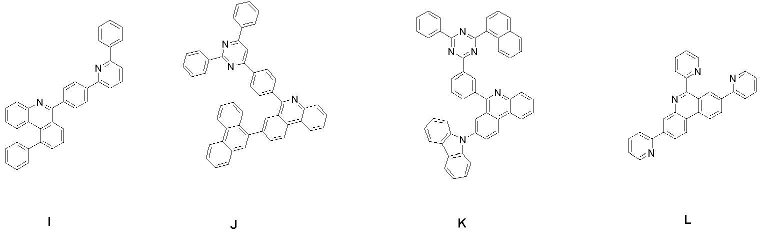

### [EVALUATION EXAMPLE 1]

The blue organic EL devices fabricated in Examples 1 to 117 and Comparative Examples 1 to 12 were measured for current density, driving voltage at 10 mA/cm², current efficiency, and emission wavelength, and the results are summarized in Table 1, below.

**TABLE 1**

| Sample | Electron transport layer | Driving volt. (V) | Emission peak (nm) | Current efficiency (cd/A) | Lifespan (h) |
|---|---|---|---|---|---|
| Ex. 1 | A-1 | 3.9 | 457 | 7.8 | 328 |
| Ex. 2 | A-3 | 4.1 | 456 | 8.1 | 320 |
| Ex. 3 | A-7 | 4.2 | 456 | 8.3 | 317 |
| Ex. 4 | A-8 | 4.1 | 457 | 7.8 | 298 |
| Ex. 5 | A-13 | 4.2 | 456 | 7.9 | 285 |
| Ex. 6 | A-19 | 3.9 | 458 | 7.9 | 312 |
| Ex. 7 | A-25 | 4.0 | 458 | 8.0 | 326 |
| Ex. 8 | A-31 | 4.1 | 454 | 7.8 | 250 |
| Ex. 9 | A-37 | 4.0 | 457 | 7.9 | 380 |
| Ex. 10 | A-38 | 4.2 | 458 | 7.7 | 376 |
| Ex. 11 | A-40 | 4.0 | 454 | 7.7 | 298 |
| Ex. 12 | A-43 | 4.0 | 454 | 7.8 | 295 |
| Ex. 13 | A-49 | 4.3 | 455 | 7.9 | 316 |
| Ex. 14 | A-51 | 4.0 | 455 | 7.8 | 324 |
| Ex. 15 | A-52 | 4.2 | 457 | 7.5 | 336 |
| Ex. 16 | A-55 | 4.2 | 457 | 7.5 | 348 |
| Ex. 17 | A-56 | 4.1 | 457 | 7.8 | 350 |
| Ex. 18 | A-61 | 4.3 | 455 | 7.7 | 343 |
| Ex. 19 | A-70 | 4.0 | 454 | 7.7 | 289 |
| Ex. 20 | A-72 | 4.2 | 455 | 7.6 | 297 |
| Ex. 21 | A-73 | 4.1 | 455 | 7.6 | 326 |
| Ex. 22 | A-75 | 4.3 | 457 | 8.0 | 328 |
| Ex. 23 | A-79 | 4.0 | 456 | 8.1 | 335 |
| Ex. 24 | A-81 | 4.3 | 457 | 7.9 | 332 |
| Ex. 25 | A-82 | 4.2 | 456 | 8.0 | 324 |
| Ex. 26 | A-88 | 4.1 | 456 | 7.7 | 365 |
| Ex. 27 | A-89 | 4.2 | 456 | 7.6 | 366 |
| Ex. 28 | A-96 | 3.9 | 458 | 7.9 | 385 |
| Ex. 29 | A-97 | 3.9 | 458 | 7.8 | 298 |
| Ex. 30 | A-100 | 4.1 | 454 | 7.6 | 321 |
| Ex. 31 | A-104 | 4.1 | 457 | 7.8 | 320 |
| Ex. 32 | A-106 | 4.2 | 458 | 7.8 | 354 |
| Ex. 33 | A-110 | 4.0 | 454 | 7.9 | 321 |
| Ex. 34 | A-112 | 4.1 | 454 | 7.8 | 326 |
| Ex. 35 | A-113 | 4.3 | 455 | 7.7 | 315 |
| Ex. 36 | A-116 | 4.2 | 455 | 7.9 | 316 |
| Ex. 37 | A-119 | 4.3 | 457 | 7.6 | 350 |
| Ex. 38 | A-121 | 4.3 | 457 | 7.8 | 342 |
| Ex. 39 | A-122 | 4.0 | 457 | 7.7 | 350 |
| Ex. 40 | B-1 | 4.1 | 455 | 7.6 | 340 |
| Ex. 41 | B-3 | 4.0 | 454 | 7.6 | 296 |
| Ex. 42 | B-7 | 4.3 | 455 | 7.8 | 295 |
| Ex. 43 | B-8 | 4.1 | 455 | 7.9 | 346 |
| Ex. 44 | B-13 | 4.3 | 457 | 7.4 | 332 |
| Ex. 45 | B-19 | 3.9 | 456 | 8.1 | 312 |
| Ex. 46 | B-25 | 4.1 | 457 | 7.8 | 326 |
| Ex. 47 | B-31 | 4.1 | 456 | 7.7 | 358 |
| Ex. 48 | B-37 | 4.1 | 456 | 7.7 | 321 |
| Ex. 49 | B-38 | 4.2 | 456 | 7.3 | 316 |
| Ex. 50 | B-40 | 3.9 | 458 | 8.0 | 319 |
| Ex. 51 | B-43 | 4.0 | 458 | 7.7 | 318 |
| Ex. 52 | B-49 | 4.1 | 454 | 8.0 | 326 |
| Ex. 53 | B-51 | 4.1 | 457 | 8.0 | 325 |
| Ex. 54 | B-52 | 4.3 | 458 | 7.6 | 329 |
| Ex. 55 | B-55 | 4.1 | 454 | 7.9 | 333 |
| Ex. 56 | B-56 | 4.0 | 454 | 7.8 | 312 |
| Ex. 57 | B-61 | 4.3 | 455 | 7.9 | 316 |
| Ex. 58 | B-70 | 4.0 | 455 | 8.0 | 305 |
| Ex. 59 | B-72 | 4.1 | 457 | 7.9 | 307 |
| Ex. 60 | B-73 | 4.1 | 457 | 7.6 | 298 |
| Ex. 61 | B-75 | 4.2 | 457 | 7.8 | 299 |
| Ex. 62 | B-79 | 4.1 | 455 | 7.9 | 326 |
| Ex. 63 | B-81 | 4.1 | 454 | 7.9 | 333 |
| Ex. 64 | B-82 | 4.2 | 455 | 7.6 | 326 |
| Ex. 65 | B-88 | 3.9 | 455 | 7.9 | 358 |
| Ex. 66 | B-89 | 4.1 | 457 | 7.5 | 354 |
| Ex. 67 | B-96 | 4.0 | 456 | 8.1 | 331 |
| Ex. 68 | B-97 | 4.1 | 457 | 7.9 | 321 |
| Ex. 69 | B-100 | 4.1 | 456 | 7.8 | 360 |
| Ex. 70 | B-104 | 4.2 | 456 | 7.9 | 361 |
| Ex. 71 | B-106 | 4.2 | 456 | 7.7 | 350 |
| Ex. 72 | B-110 | 3.9 | 458 | 8.0 | 340 |
| Ex. 73 | B-112 | 4.0 | 458 | 8.0 | 341 |
| Ex. 74 | B-113 | 4.3 | 454 | 7.5 | 326 |
| Ex. 75 | B-116 | 4.0 | 457 | 7.9 | 328 |
| Ex. 76 | B-119 | 4.3 | 458 | 7.6 | 329 |
| Ex. 77 | B-121 | 3.9 | 454 | 7.9 | 319 |
| Ex. 78 | B-122 | 4.0 | 454 | 7.8 | 315 |
| Ex. 79 | C-1 | 4.1 | 455 | 7.8 | 316 |
| Ex. 80 | C-3 | 4.1 | 455 | 8.0 | 328 |
| Ex. 81 | C-7 | 4.0 | 456 | 8.0 | 306 |
| Ex. 82 | C-8 | 4.1 | 456 | 8.1 | 338 |
| Ex. 83 | C-13 | 4.1 | 458 | 7.9 | 339 |
| Ex. 84 | C-19 | 4.2 | 458 | 8.0 | 334 |
| Ex. 85 | C-25 | 4.1 | 454 | 7.7 | 336 |
| Ex. 86 | C-31 | 4.1 | 457 | 7.6 | 345 |
| Ex. 87 | C-37 | 4.2 | 458 | 7.9 | 316 |
| Ex. 88 | C-38 | 3.9 | 454 | 7.8 | 328 |
| Ex. 89 | C-40 | 4.1 | 454 | 7.6 | 268 |
| Ex. 90 | C-43 | 4.0 | 455 | 7.8 | 234 |
| Ex. 91 | C-49 | 4.1 | 455 | 7.8 | 321 |
| Ex. 92 | C-51 | 4.1 | 457 | 7.9 | 329 |
| Ex. 93 | C-52 | 3.9 | 457 | 7.8 | 315 |
| Ex. 94 | C-55 | 3.9 | 457 | 7.7 | 345 |
| Ex. 95 | C-56 | 4.1 | 455 | 7.9 | 365 |
| Ex. 96 | C-61 | 4.1 | 454 | 7.6 | 312 |
| Ex. 97 | C-70 | 4.2 | 455 | 7.8 | 298 |
| Ex. 98 | C-72 | 4.0 | 455 | 7.7 | 265 |
| Ex. 99 | C-73 | 4.1 | 457 | 7.6 | 324 |
| Ex. 100 | C-75 | 4.3 | 456 | 7.6 | 326 |
| Ex. 101 | C-79 | 4.2 | 457 | 7.8 | 319 |
| Ex. 102 | C-81 | 4.3 | 456 | 7.9 | 239 |
| Ex. 103 | C-82 | 4.3 | 456 | 7.4 | 336 |
| Ex. 104 | C-88 | 4.1 | 456 | 8.1 | 354 |
| Ex. 105 | C-89 | 4.0 | 458 | 7.8 | 346 |
| Ex. 106 | C-96 | 4.1 | 454 | 7.7 | 357 |
| Ex. 107 | C-97 | 4.1 | 457 | 7.7 | 315 |
| Ex. 108 | C-100 | 3.9 | 458 | 7.3 | 326 |
| Ex. 109 | C-104 | 3.9 | 454 | 8.0 | 326 |
| Ex. 110 | C-106 | 4.1 | 454 | 7.7 | 354 |
| Ex. 111 | C-110 | 4.1 | 455 | 8.0 | 326 |
| Ex. 112 | C-112 | 4.2 | 455 | 8.0 | 312 |
| Ex. 113 | C-113 | 4.0 | 457 | 7.6 | 318 |
| Ex. 114 | C-116 | 4.1 | 457 | 7.9 | 320 |
| Ex. 115 | C-119 | 3.9 | 457 | 7.8 | 321 |
| Ex. 116 | C-121 | 3.8 | 455 | 7.9 | 330 |
| Ex. 117 | C-122 | 4.0 | 455 | 8.0 | 333 |
| C. Ex. 1 | Compound A | 4.4 | 456 | 6.9 | 50 |
| C. Ex. 2 | Compound B | 4.5 | 458 | 6.8 | 68 |
| C. Ex. 3 | Compound C | 4.4 | 459 | 6.6 | 69 |
| C. Ex. 4 | Compound D | 4.5 | 459 | 6.9 | 80 |
| C. Ex. 5 | Compound E | 5.3 | 460 | 5.9 | 84 |
| C. Ex. 6 | Compound F | 5.2 | 458 | 6.0 | 105 |
| C. Ex. 7 | Compound G | 4.6 | 458 | 6.7 | 68 |
| C. Ex. 8 | Compound H | 5.6 | 457 | 5.4 | 90 |
| C. Ex. 9 | Compound I | 5.9 | 458 | 5.2 | 85 |
| C. Ex. 10 | Compound J | 5.8 | 458 | 5.1 | 86 |
| C. Ex. 11 | Compound K | 4.7 | 459 | 6.7 | 76 |
| C. Ex. 12 | Compound L | 4.3 | 456 | 7.0 | 65 |

As understood from the data of Table 1, the blue organic EL devices of Examples 1 to 117 in which the compounds according to the present disclosure were used in the electron transport layer was superior in terms of driving voltage, emission peak, current efficiency, and lifespan, compared to the organic EL devices of Comparative Examples 1 to 4 characterized in that the phenanthroline was connected directly or via a linker; of Comparative Examples 5 to 11 characterized in that the phenanthridine is not bonded with pyridine; of Comparative Example 12 characterized in that three pyridines were linked to the phenanthridine.

### [EXAMPLE 118] Fabrication of Organic EL Device

After Compound A-1 synthesized in the Synthesis Example was isolated to a high purity through sublimation, a blue organic EL device wase fabricated using same as follows.

First, a glass substrate coated with 1,500 Å-thick indium tin oxide (ITO) thin film was cleansed by ultrasonication in distilled water. Following washing with distilled water, the substrate was washed with a solvent such as isopropyl alcohol, acetone, methanol, etc. under ultrasonication. Thereafter, the substrate was transferred to a UV OZONE cleaner (Power sonic 405, Hwashin Tech), cleaned for 5 minutes using UV, and then moved to a vacuum evaporator.

On the transparent ITO electrode prepared above, Compounds 1 and 2 were co-deposited at a weight ratio of 98:2 to form a 100 Å-thick hole injection on which Compound 1 was then deposited at a thickness of 200Å to form a hole transport layer. A hole transport auxiliary layer 50 Å thick was formed by deposition of Compound 3 on the hole transport layer, followed by co-depositing Compounds 4 and 5 at a weight ratio of 98:2 to form a light-emitting layer 200Å thick. Compound 7 was deposited at a thickness of 150 Å on the light-emitting layer to form an electron transport zone on which a charge generation layer was formed by depositing Compound A-1 of the Example at a thickness of 80 Å. Co-deposition of Compounds 1 and 2 at a weight ratio of 98:2 on the charge generation layer formed a 100 Å-thick hole injection layer. A 350 Å-thick hole transport layer was formed by depositing Compound 1 on the hole injection layer. On the hole transport layer was deposited Compound 3 at a thickness of 50 Å to form a hole transport auxiliary layer, followed by co-depositing Compounds 4 and 5 at a weight ratio of 98:2 to form a 200Å-thick light-emitting layer. On the light-emitting layer, deposition was made of Compound 6 to form a 50Å-thick electron transport auxiliary layer and then Compounds 7 and 8 at a weight ratio of 1:1 to form 300 Å-thick electron transport zone. Deposition of LiF on the electron transport layer formed a 10 Å-thick electron injection layer which was then coated with Al to form a 1,000 Å-thick cathode, leading to the fabrication of an organic EL device.

Structures of Compounds 1 to 7 used for the fabrication are as given in Example 1 and the structure of Compound 8 is as follows.

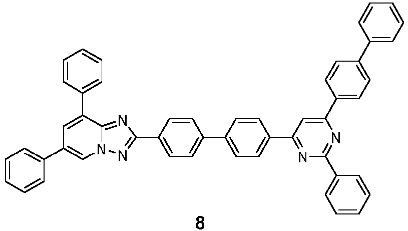

### [EXAMPLES 119 TO 234] Fabrication of Organic EL Devices

Organic EL devices were fabricated in the same manner as in Example 118 with the exception of using Compounds listed in Table 2, instead of Compound A-1, as charge generation layer materials, respectively.

### [COMPARATIVE EXAMPLES 13 TO 24] Fabrication of Organic EL Devices

Organic EL devices were fabricated in the same manner as in Example 118 with the exception of using Compounds A to L, instead of Compound A-1, as charge generation layer materials, respectively. The Compounds A to L are as described in Comparative Examples 1 to 12.

### [EVALUATION EXAMPLE 2]

The organic EL devices fabricated in Examples 118 to 234 and Comparative Examples 13 to 24 were measured for current density, driving voltage at 10 mA/cm², current efficiency, and emission wavelength, and the results are summarized in Table 2, below.

**TABLE 2**

| Sample | N-type charge generation layer | Driving volt. (V) | Current efficiency (cd/A) | Lifespan (h) |
|---|---|---|---|---|
| Ex. 118 | A-1 | 8.6 | 15.3 | 358 |
| Ex. 119 | A-3 | 8.3 | 15.9 | 321 |
| Ex. 120 | A-7 | 8.3 | 15.7 | 316 |
| Ex. 121 | A-8 | 8.2 | 15.5 | 319 |
| Ex. 122 | A-13 | 8.3 | 15.6 | 318 |
| Ex. 123 | A-19 | 8.5 | 15.3 | 326 |
| Ex. 124 | A-25 | 8.3 | 15.3 | 325 |
| Ex. 125 | A-31 | 8.2 | 15.7 | 329 |
| Ex. 126 | A-37 | 8.5 | 15.2 | 333 |
| Ex. 127 | A-38 | 8.2 | 15.5 | 312 |
| Ex. 128 | A-40 | 8.6 | 15.3 | 316 |
| Ex. 129 | A-43 | 8.4 | 15.0 | 305 |
| Ex. 130 | A-49 | 8.2 | 15.8 | 307 |
| Ex. 131 | A-51 | 8.4 | 15.4 | 298 |
| Ex. 132 | A-52 | 8.3 | 15.7 | 299 |
| Ex. 133 | A-55 | 8.2 | 15.7 | 326 |
| Ex. 134 | A-56 | 8.6 | 15.5 | 333 |
| Ex. 135 | A-61 | 8.3 | 15.9 | 326 |
| Ex. 136 | A-70 | 8.5 | 15.5 | 358 |
| Ex. 137 | A-72 | 8.4 | 15.8 | 354 |
| Ex. 138 | A-73 | 8.6 | 14.8 | 331 |
| Ex. 139 | A-75 | 8.3 | 15.6 | 321 |
| Ex. 140 | A-79 | 8.3 | 15.5 | 360 |
| Ex. 141 | A-81 | 8.2 | 15.7 | 361 |
| Ex. 142 | A-82 | 8.3 | 15.7 | 350 |
| Ex. 143 | A-88 | 8.3 | 15.8 | 340 |
| Ex. 144 | A-89 | 8.2 | 15.6 | 341 |
| Ex. 145 | A-96 | 8.4 | 15.2 | 326 |
| Ex. 146 | A-97 | 8.5 | 15.2 | 328 |
| Ex. 147 | A-100 | 8.3 | 15.7 | 329 |
| Ex. 148 | A-104 | 8.5 | 15.2 | 319 |
| Ex. 149 | A-106 | 8.2 | 15.6 | 315 |
| Ex. 150 | A-110 | 8.5 | 15.1 | 316 |
| Ex. 151 | A-112 | 8.4 | 15.0 | 328 |
| Ex. 152 | A-113 | 8.3 | 15.6 | 306 |
| Ex. 153 | A-116 | 8.5 | 15.1 | 338 |
| Ex. 154 | A-119 | 8.2 | 15.5 | 339 |
| Ex. 155 | A-121 | 8.3 | 15.7 | 342 |
| Ex. 156 | A-122 | 8.4 | 15.0 | 350 |
| Ex. 157 | B-1 | 8.2 | 15.7 | 340 |
| Ex. 158 | B-3 | 8.5 | 15.0 | 296 |
| Ex. 159 | B-7 | 8.3 | 15.6 | 295 |
| Ex. 160 | B-8 | 8.5 | 15.2 | 346 |
| Ex. 161 | B-13 | 8.6 | 15.4 | 332 |
| Ex. 162 | B-19 | 8.3 | 15.3 | 312 |
| Ex. 163 | B-25 | 8.3 | 15.9 | 326 |
| Ex. 164 | B-31 | 8.2 | 15.9 | 358 |
| Ex. 165 | B-37 | 8.3 | 15.7 | 321 |
| Ex. 166 | B-38 | 8.5 | 15.7 | 316 |
| Ex. 167 | B-40 | 8.3 | 15.4 | 319 |
| Ex. 168 | B-43 | 8.2 | 15.3 | 318 |
| Ex. 169 | B-49 | 8.5 | 15.7 | 331 |
| Ex. 170 | B-51 | 8.2 | 15.1 | 321 |
| Ex. 171 | B-52 | 8.6 | 15.4 | 360 |
| Ex. 172 | B-55 | 8.3 | 15.3 | 361 |
| Ex. 173 | B-56 | 8.3 | 15.9 | 350 |
| Ex. 174 | B-61 | 8.2 | 15.9 | 340 |
| Ex. 175 | B-70 | 8.3 | 15.7 | 341 |
| Ex. 176 | B-72 | 8.5 | 15.7 | 326 |
| Ex. 177 | B-73 | 8.3 | 15.4 | 328 |
| Ex. 178 | B-75 | 8.2 | 15.8 | 329 |
| Ex. 179 | B-79 | 8.5 | 15.3 | 319 |
| Ex. 180 | B-81 | 8.2 | 15.8 | 315 |
| Ex. 181 | B-82 | 8.6 | 15.4 | 316 |
| Ex. 182 | B-88 | 8.4 | 15.7 | 358 |
| Ex. 183 | B-89 | 8.2 | 15.4 | 331 |
| Ex. 184 | B-96 | 8.4 | 15.9 | 321 |
| Ex. 185 | B-97 | 8.3 | 15.8 | 360 |
| Ex. 186 | B-100 | 8.2 | 15.9 | 361 |
| Ex. 187 | B-104 | 8.6 | 15.8 | 350 |
| Ex. 188 | B-106 | 8.3 | 15.2 | 340 |
| Ex. 189 | B-110 | 8.5 | 15.4 | 341 |
| Ex. 190 | B-112 | 8.4 | 15.7 | 326 |
| Ex. 191 | B-113 | 8.6 | 15.4 | 328 |
| Ex. 192 | B-116 | 8.3 | 15.9 | 329 |
| Ex. 193 | B-119 | 8.3 | 15.8 | 319 |
| Ex. 194 | B-121 | 8.2 | 15.4 | 315 |
| Ex. 195 | B-122 | 8.3 | 15.4 | 316 |
| Ex. 196 | C-1 | 8.3 | 15, 5 | 316 |
| Ex. 197 | C-3 | 8.2 | 15.3 | 328 |
| Ex. 198 | C-7 | 8.4 | 15.4 | 306 |
| Ex. 199 | C-8 | 8.5 | 15.7 | 338 |
| Ex. 200 | C-13 | 8.3 | 15.4 | 339 |
| Ex. 201 | C-19 | 8.5 | 15.9 | 334 |
| Ex. 202 | C-25 | 8.2 | 15.8 | 336 |
| Ex. 203 | C-31 | 8.5 | 15.9 | 345 |
| Ex. 204 | C-37 | 8.4 | 15.8 | 316 |
| Ex. 205 | C-38 | 8.3 | 15.2 | 328 |
| Ex. 206 | C-40 | 8.5 | 15.4 | 268 |
| Ex. 207 | C-43 | 8.2 | 15.7 | 234 |
| Ex. 208 | C-49 | 8.3 | 15.4 | 321 |
| Ex. 209 | C-51 | 8.4 | 15.9 | 329 |
| Ex. 210 | C-52 | 8.2 | 15.8 | 315 |
| Ex. 211 | C-55 | 8.5 | 15.4 | 345 |
| Ex. 212 | C-56 | 8.3 | 15.4 | 365 |
| Ex. 213 | C-61 | 8.5 | 15, 5 | 316 |
| Ex. 214 | C-70 | 8.6 | 15.3 | 316 |
| Ex. 215 | C-72 | 8.3 | 15.1 | 328 |
| Ex. 216 | C-73 | 8.3 | 15.5 | 306 |
| Ex. 217 | C-75 | 8.2 | 15.7 | 338 |
| Ex. 218 | C-79 | 8.3 | 15.0 | 339 |
| Ex. 219 | C-81 | 8.5 | 15.7 | 334 |
| Ex. 220 | C-82 | 8.3 | 15.0 | 336 |
| Ex. 221 | C-88 | 8.2 | 15.6 | 316 |
| Ex. 222 | C-89 | 8.5 | 15.2 | 316 |
| Ex. 223 | C-96 | 8.2 | 15.4 | 328 |
| Ex. 224 | C-97 | 8.3 | 15.3 | 328 |
| Ex. 225 | C-100 | 8.2 | 15.9 | 306 |
| Ex. 226 | C-104 | 8.3 | 15.9 | 338 |
| Ex. 227 | C-106 | 8.5 | 15.7 | 339 |
| Ex. 228 | C-110 | 8.3 | 15.7 | 334 |
| Ex. 229 | C-112 | 8.2 | 15.4 | 336 |
| Ex. 230 | C-113 | 8.5 | 15.3 | 316 |
| Ex. 231 | C-116 | 8.2 | 15.7 | 316 |
| Ex. 232 | C-119 | 8.3 | 15.4 | 328 |
| Ex. 233 | C-121 | 8.3 | 15.8 | 334 |
| Ex. 234 | C-122 | 8.5 | 15.7 | 336 |
| C. Ex. 13 | Compound A | 8.9 | 14.5 | 98 |
| C. Ex. 14 | Compound B | 8.7 | 14.8 | 97 |
| C. Ex. 15 | Compound C | 8.6 | 14.6 | 89 |
| C. Ex. 16 | Compound D | 8.8 | 14.8 | 87 |
| C. Ex. 17 | Compound E | 9.5 | 12.2 | 84 |
| C. Ex. 18 | Compound F | 9.8 | 13.6 | 105 |
| C. Ex. 19 | Compound G | 9.7 | 13.5 | 68 |
| C. Ex. 20 | Compound H | 9.9 | 14.0 | 90 |
| C. Ex. 21 | Compound I | 9.8 | 12.0 | 85 |
| C. Ex. 22 | Compound J | 9.5 | 12.1 | 86 |
| C. Ex. 23 | Compound K | 9.3 | 13.5 | 76 |
| C. Ex. 24 | Compound L | 8.3 | 15.1 | 95 |

As understood from the data of Table 2, the organic EL devices of Examples 118 to 234 in which the compounds according to the present disclosure were used in the electron transport layer were superior in terms of driving voltage, current efficiency, and lifespan, compared to the organic EL devices of Comparative Examples 13 to 16 characterized in that the phenanthroline was connected directly or via a linker; of Comparative Examples 17 to 23 characterized in that the phenanthridine is not bonded with pyridine; of Comparative Example 24 characterized in that three pyridines were linked to the phenanthridine.

## Claims

1. A compound represented by the following Chemical Formula 1: 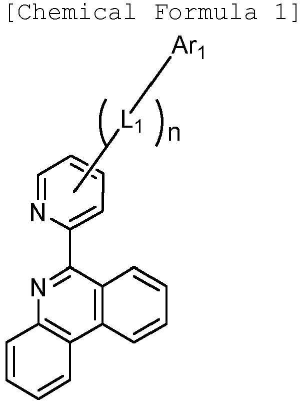 (wherein,
n is an integer of 0 to 3,
L₁ is a direct bond or is selected from the group consisting of an arylene of C₆-C₆₀ and a heteroarylene of 5 to 60 nuclear atoms,
Ar₁ is selected from the group consisting of a hydrogen atom, a deuterium atom (D), a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, an arylamine of C₆-C₆₀, and an aryl of C₆-C₆₀ substituted with an alkenyl of C₂-C₄₀, or is bonded to an adjacent group to form a fused ring,
the arylene and heteroarylene groups of L₁, and the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, arylphosphine oxide, arylamine, alkenyl-substituted aryl groups, and fused ring of Ar₁ each being unsubstituted or substituted with at least one substituent selected from the group consisting of a deuterium atom, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₂-C₄₀, an aryl of C₆-C₆₀, a heteroaryl of 5 to 60 nuclear atoms, an alkyloxy of C₂-C₄₀, an aryloxy of C₆-C₆₀ an alkylsilyl of C₂-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀, wherein two or more substituents, if present, are same or different).

2. The compound of claim 1, wherein the compound represented by Chemical Formula 1 is a compound represented by any one of the following Chemical Formulas 2 to 4: 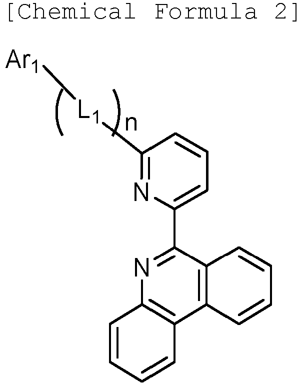 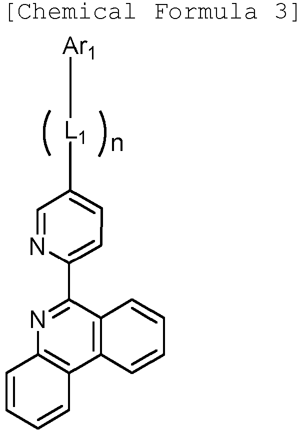 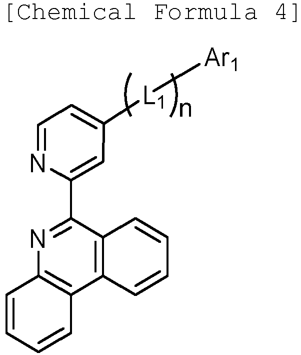 (wherein,
n, L₁, Ar₁ are as defined in claim 1) .

3. The compound of claim 1, wherein 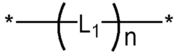 is a linker represented by any one of the following Chemical Formulas L1 to L5: 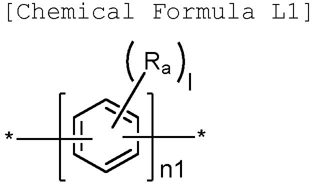 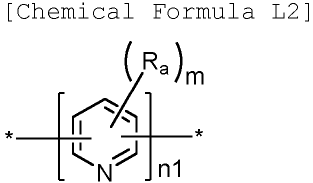 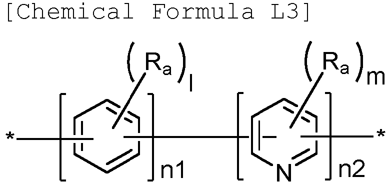 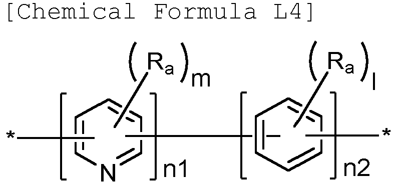 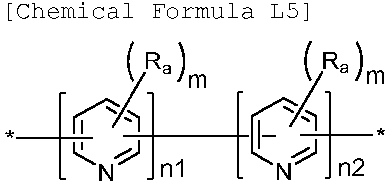 (wherein,
n1 and n2 are each an integer of 0 to 3, with a proviso of 0≤n1+n2≤3,
l is an integer of 0 to 4,
m is an integer of 0 to 3, and
two or more Rₐ's, if present, are same or different,
Rₐ being selected from the group consisting of a hydrogen atom, a deuterium atom, a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀ an arylphosphine of C₆-C₆₀, an arylphosphine oxide of C₆-C₆₀, and an arylamine of C₆-C₆₀.)

4. The compound of claim 1, wherein the Ar₁ is a substituent represented by any one of the following Chemical Formulas S1 to S12: 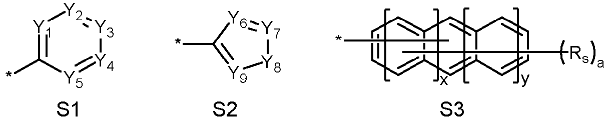 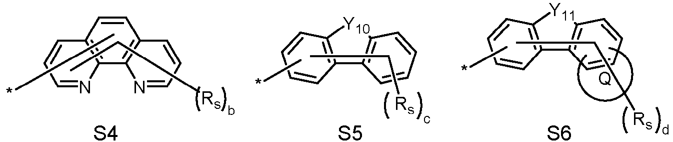 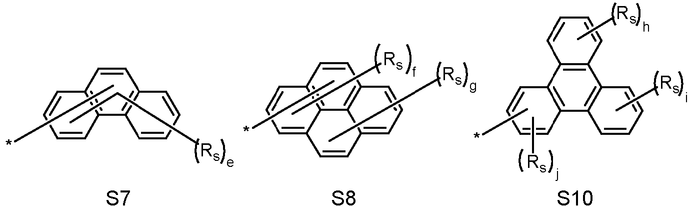 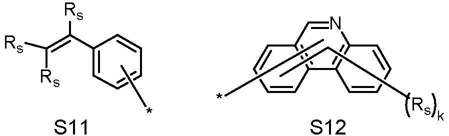 (wherein,
Y₁ to Y₅, which are same or different, are each independently N or C(R₁) wherein at least one of Y₁ to Y₅ is N and when two or more C(R₁) are present, the corresponding R₁'s are same or different,
Y₆ to Y₉, which are same or different, are each independently N, N(R₂), C(R₃), or C(R₄)(R₅), wherein at least one of Y₆ to Y₉ is N or N(R₂), and when two or more C(R₂) are present, the corresponding R₃'s are same or different, and when two or more C(R₄)(R₅) are present, the corresponding R₄'s are same or different and the corresponding R₅'s are same or different, with sameness or difference between R₄ and R₅,
Y₁₀ and Y₁₁ are each independently O or S,
cyclic Q is a benzene ring,
x and y are each independently 0 or 1, a, d, and e are each independently an integer of 0 to 9,
b and c are each independently an integer of 0 to 7,
f, h, and i are each independently an integer of 0 to 4,
g is an integer of 0 to 5,
j is an integer of 0 to 3,
k is an integer of 0 to 8,
R₁ to R₅ and Rₛ, which are same or different, are each independently selected from the group consisting of a hydrogen atom, a deuterium atom, a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀ an arylphosphine of C₆-C₆₀, an arylphosphine oxide of C₆-C₆₀, and an arylamine of C₆-C₆₀ or each independently bond to an adjacent group to form a fused ring,
the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, arylphosphineoxide, arylamine, and fused ring in R₁ to R₅ and Rₛ each being independently unsubstituted or substituted with at least one substituent selected from the group consisting of a deuterium hydrogen, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₁-C₄₀, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀ an arylphosphine of C₆-C₆₀, an arylphosphine oxide of C₆-C₆₀ and an arylamine of C₆-C₆₀ wherein two or more substituents, if present, are same or different.)

5. The compound of claim 4, wherein the Ar₁ is a substituent selected from the group consisting of the following substituents S2-1 to S2-68: 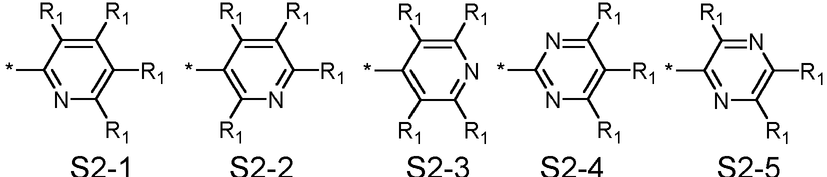 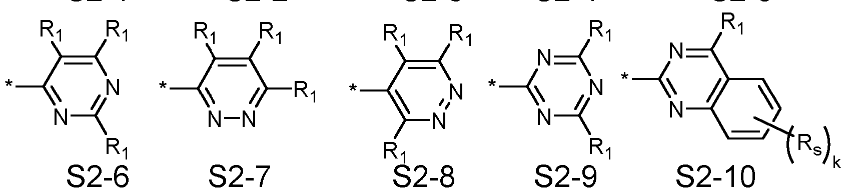 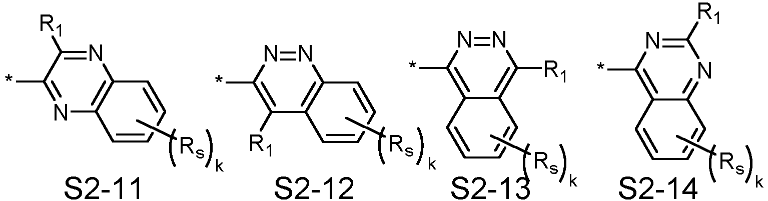 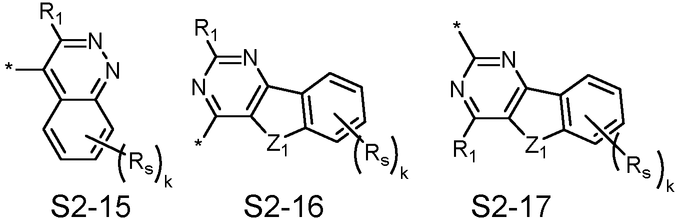 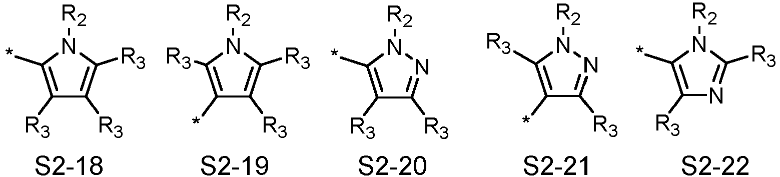 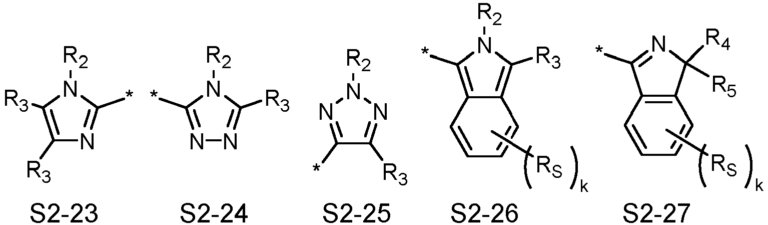 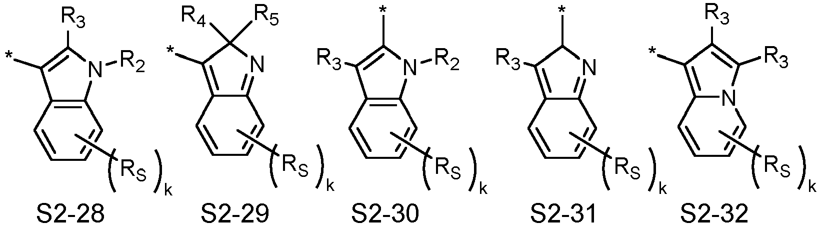 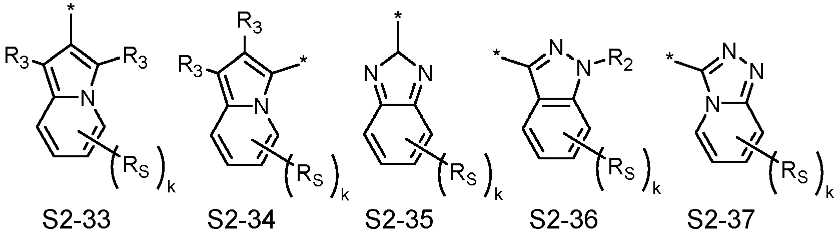 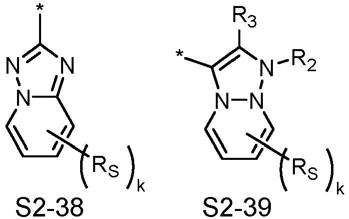 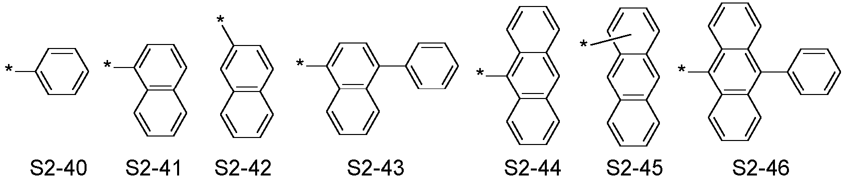 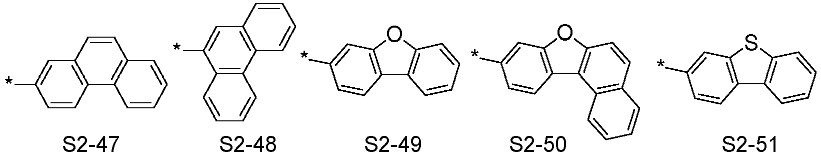 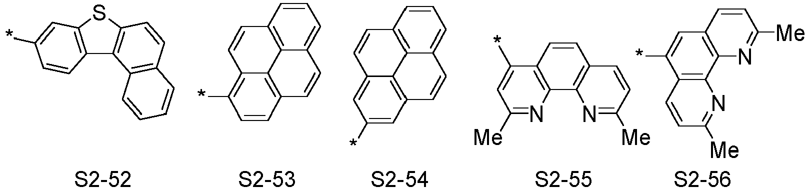 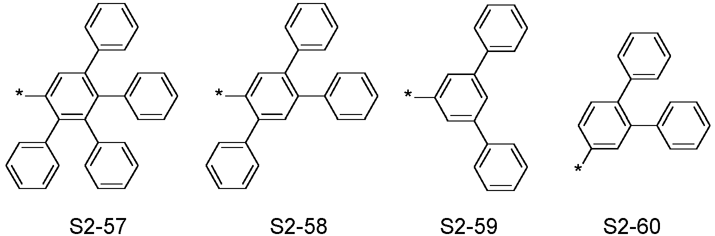 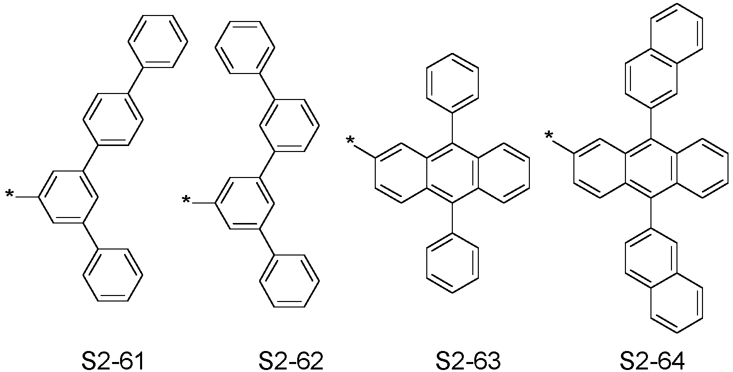 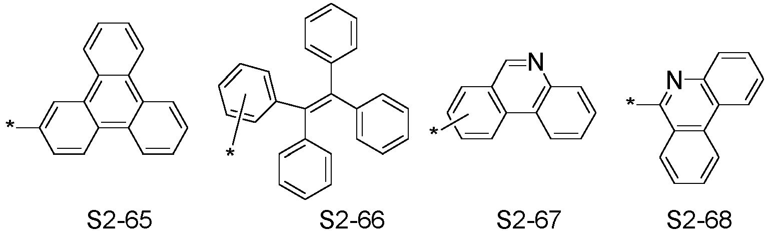 (wherein,
R₁ to R₅ and Rₛ are each as defined in claim 4,
k is an integer of 0 to 4).

6. The compound of claim 1, wherein the compound represented by Chemical Formula 1 is a compound represented by any one of the following Chemical Formulas 5 to 17: 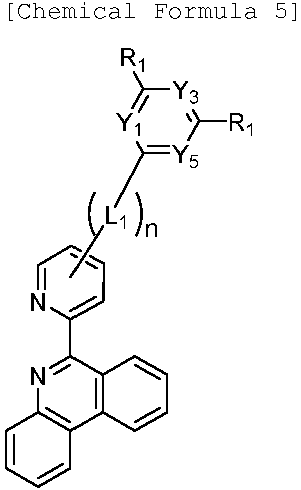 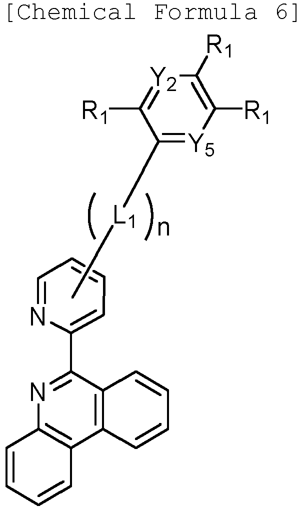 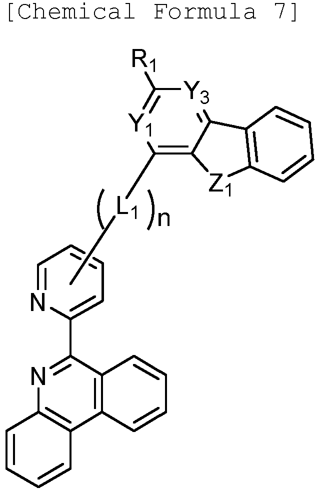 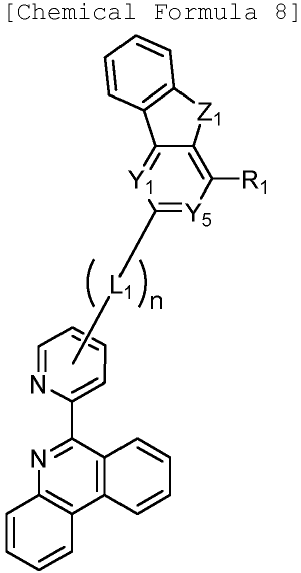 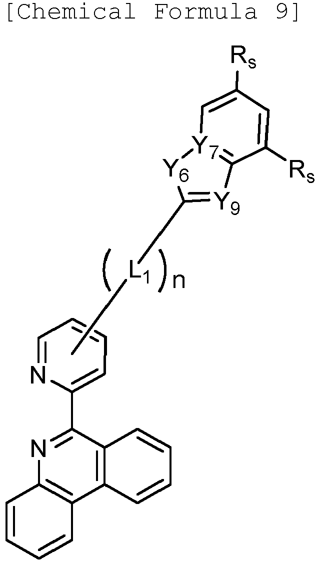 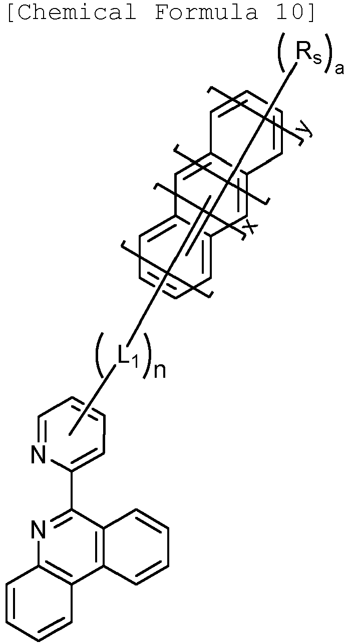 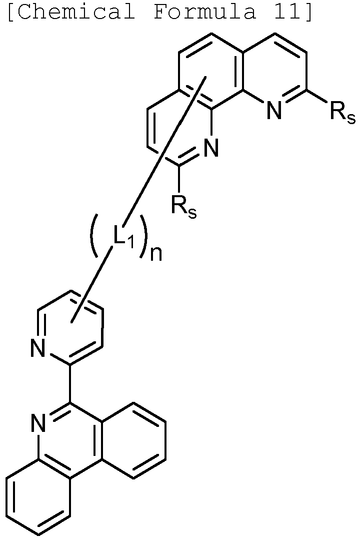 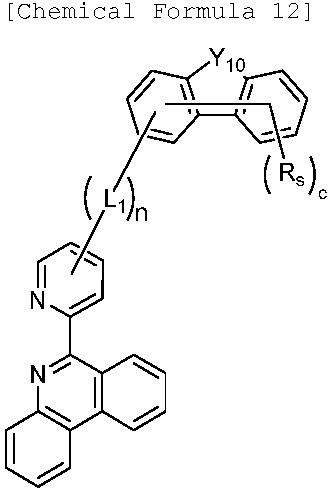 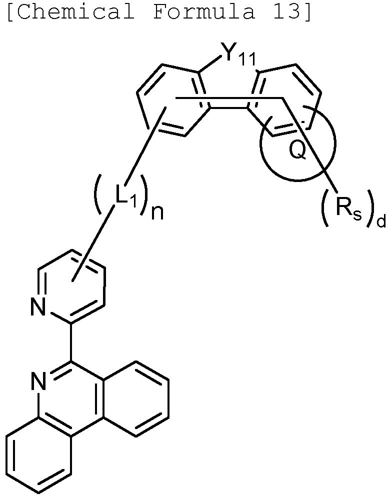 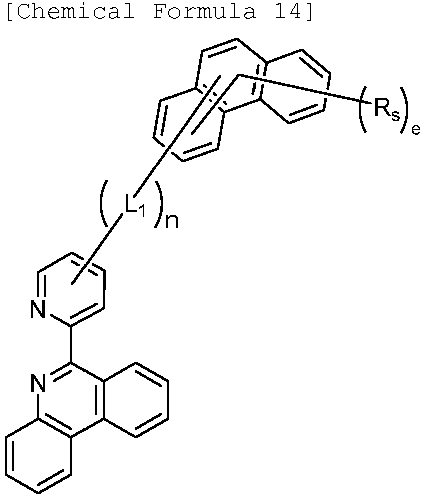 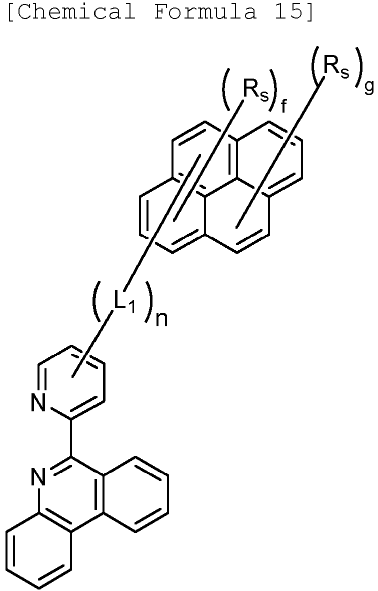 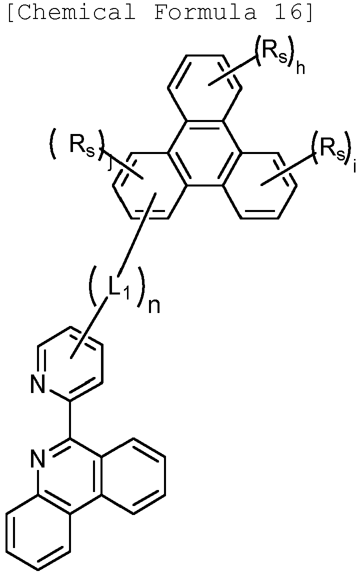 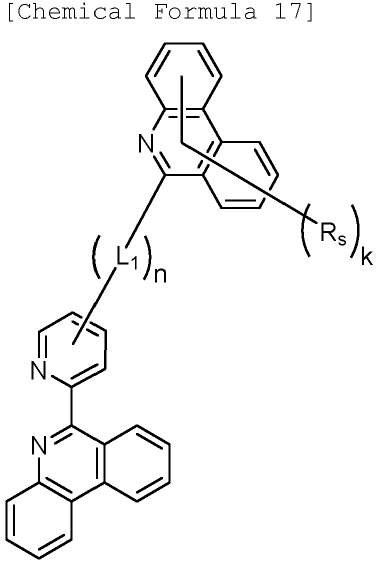 (wherein,
n is an integer of 0 to 3,
L₁ is a direct bond or is selected from the group consisting of an arylene of C₆-C₆₀ and a heteroarylene of 5 to 60 nuclear atoms,
Y₁, Y₃, and Y₅, which are same or different, are each independently N or C(R₁), wherein at least one of Y₁, Y₃, and Y₅ is N and when two or more C(R₁) are present, the corresponding R₁'s are same or different,
Y₂ and Y₅, which are same or different, are each independently N or C(R₁) wherein at least one of Y₂ and Y₅ is N and when two or more C(R₁) are present, the corresponding R₁'s are same or different,
Y₁ and Y₃, which are same or different, are each independently N or C(R₁) wherein at least one of Y₁ and Y₃ is N and when two or more C(R₁) are present, the corresponding R₁'s are same or different,
Y₁ and Y₅, which are same or different, are each independently N or C(R₁) wherein at least one of Y₁ and Y₅ is N and when two or more C(R₁) are present, the corresponding R₁'s are same or different,
Y₁₀, Y₁₁, and Z₁ are each independently O or S,
Y₆, Y₇, and Y₉, which are same or different, are each independently N, N(R₂), C(R₃), or C(R₄)(R₅), wherein at least one of Y₆, Y₇, and Y₉ is N or N(R₂), and when two or more C(R₁) are present, the corresponding R₃'s are same or different, and when two or more C(R₄)(R₅) are present, the corresponding R₄'s are same or different and the corresponding R₅'s are same or different,
x and y are each independently 0 or 1,
cyclic Q is a benzene ring,
a, d, and e are each independently an integer of 0 to 9,
b and c are each independently an integer of 0 to 7,
f, h, and i are each independently an integer of 0 to 4,
g is an integer of 0 to 5,
j is an integer of 0 to 3,
k is an integer of 0 to 8,
R₁ to R₅, and Rₛ, which are same or different, are each independently selected from the group consisting of a hydrogen atom, a deuterium atom, a halogen, a cyano, a nitro, an amino, an alkyl of C₁-C₄₀, an alkenyl of C₂-C₄₀, an alkynyl of C₂-C₄₀, a cycloalkyl of C₃-C₄₀, a heterocycloalkyl of 3 to 40 nuclear atoms, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀, or each independently bond to an adjacent group to form a fused ring,
the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, arylphosphineoxide, arylamine, and fused ring in R₁ to R₅ and Rₛ are each independently unsubstituted or substituted with at least one substituent selected from a deuterium atom, a halogen, a cyano, a nitro, an alkenyl of C₂-C₄₀, an alkynyl of C₂-C₄₀, a cycloalkyl of C₃-C₄₀, a heterocycloalkyl of 3 to 40 nuclear atoms, an alkyl of C₁-C₄₀, an aryl of C₆-C₆₀, a heteroaryl of 5 to 60 nuclear atoms, an alkyloxy of C₁-C₄₀, an aryloxy of C₆-C₆₀, an alkylsilyl of C₁-C₄₀, an arylsilyl of C₆-C₆₀, an alkylboron of C₁-C₄₀, an arylboron of C₆-C₆₀, an arylphosphine of C₆-C₆₀, an arylphosphine oxide of C₆-C₆₀, and an arylamine of C₆-C₆₀ wherein two or more substituents, if present, are same or different).

7. The compound of claim 1, wherein the compound represented by Chemical Formula 1 is selected from the group consisting of the following Compounds A-1 to C-126:

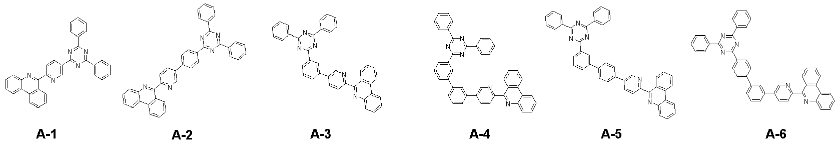

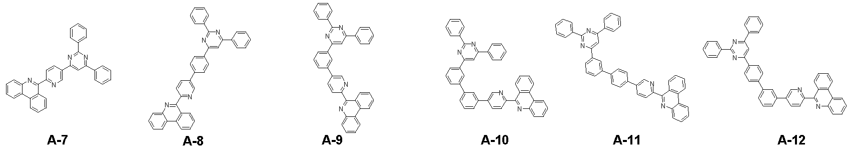

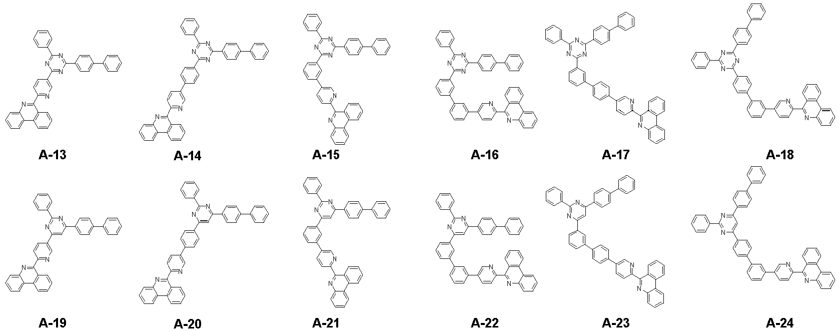

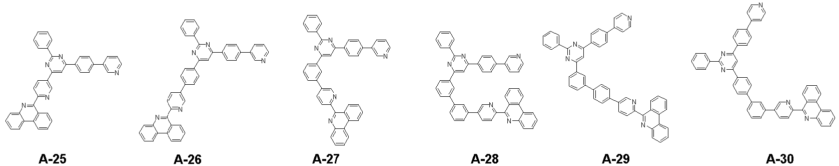

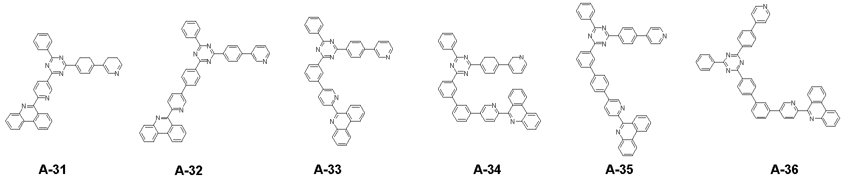

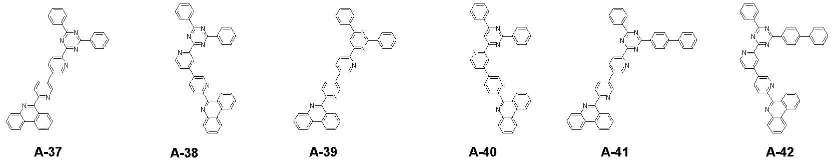

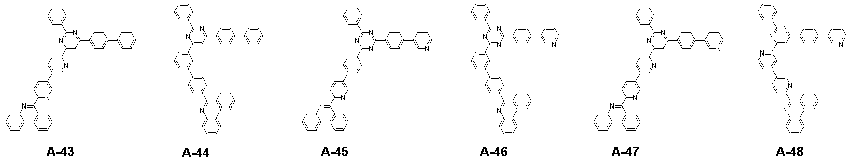

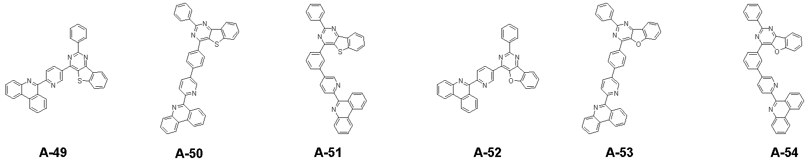

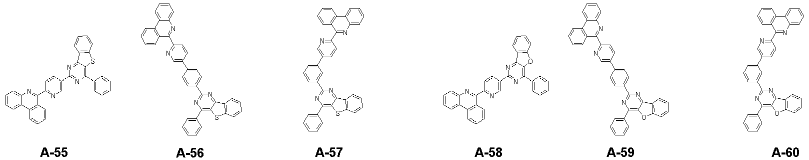

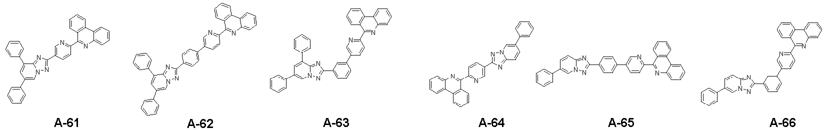

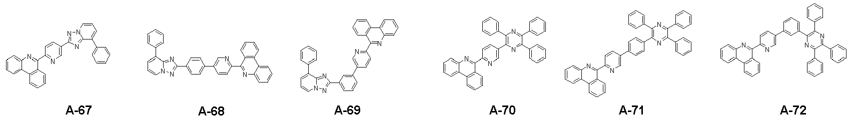

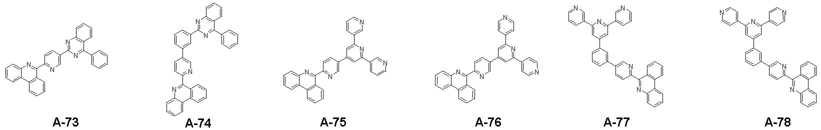

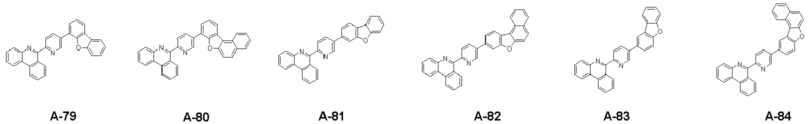

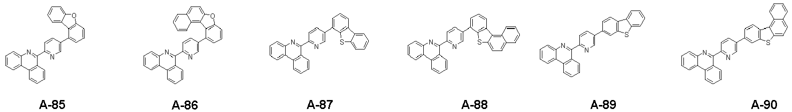

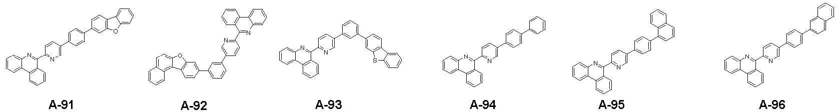

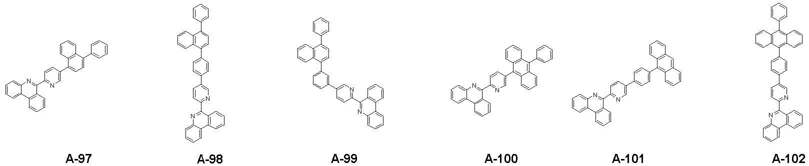

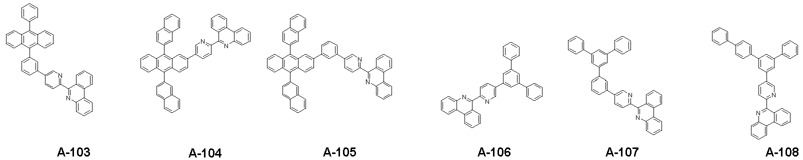

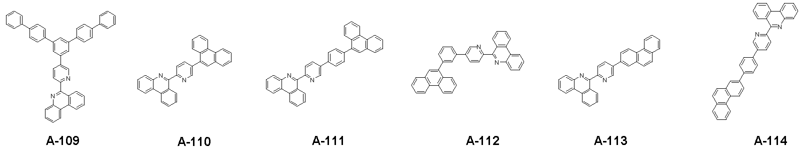

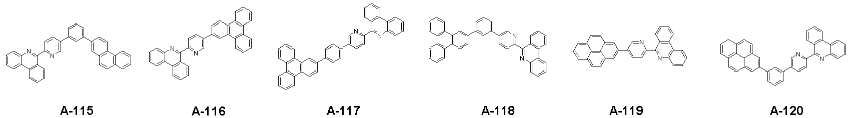

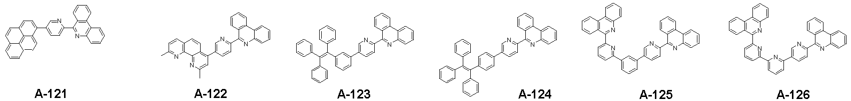

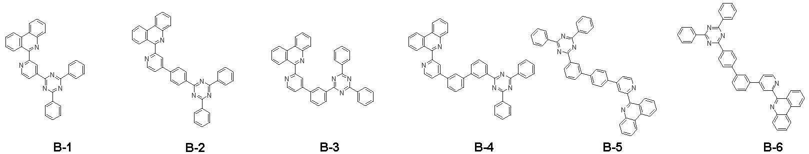

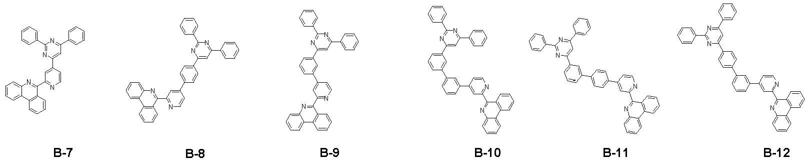

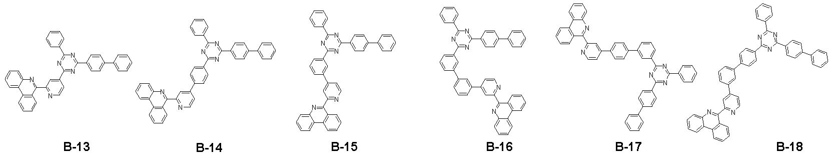

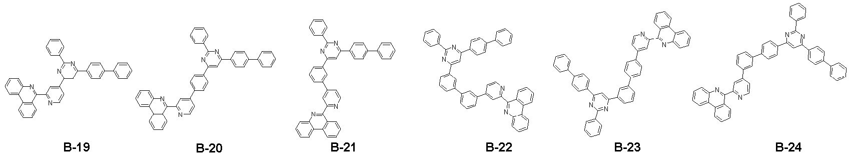

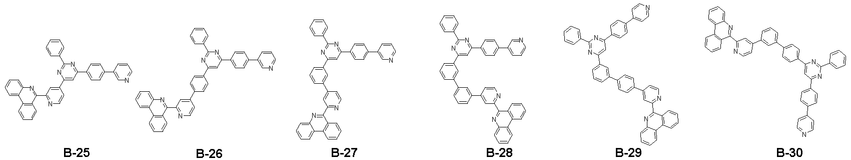

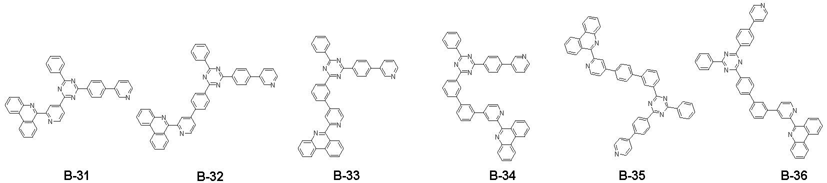

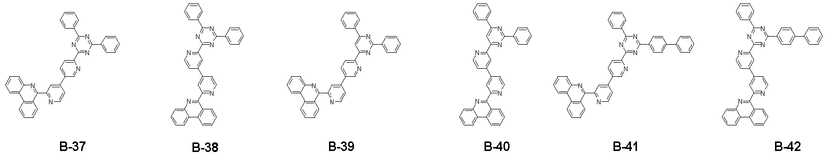

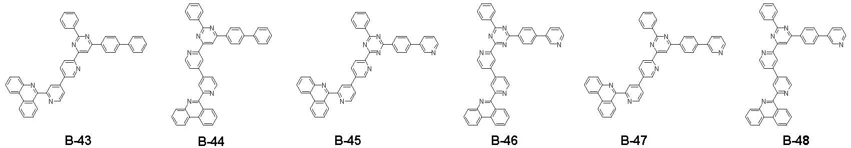

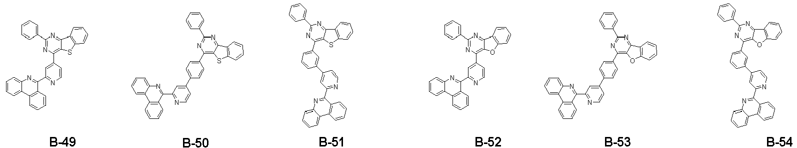

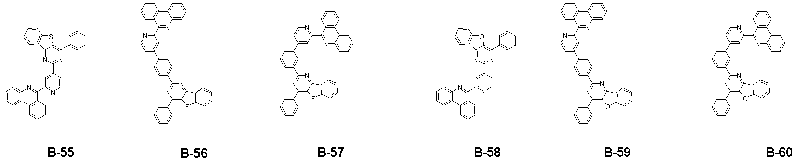

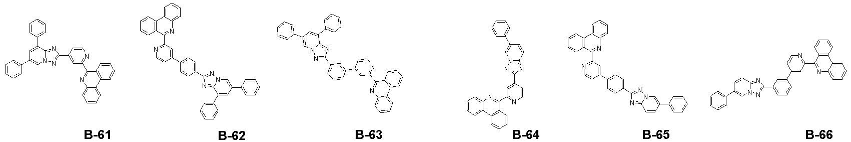

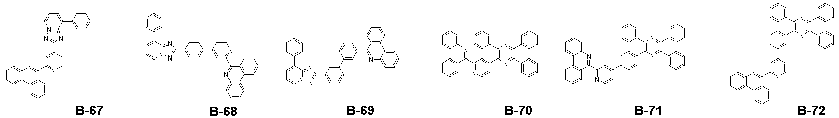

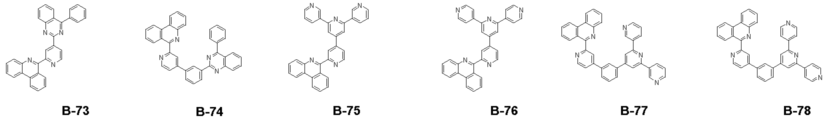

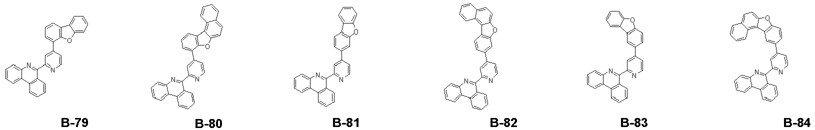

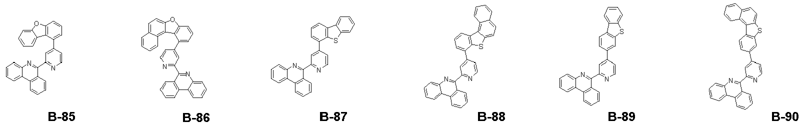

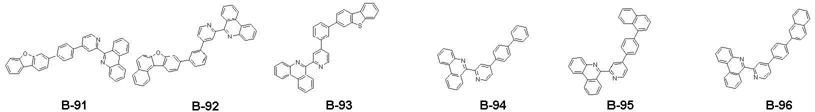

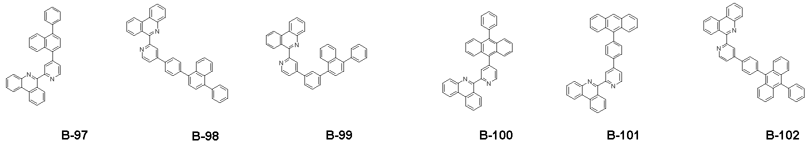

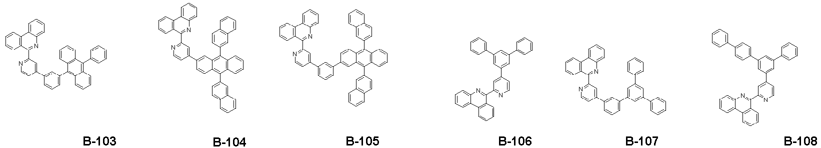

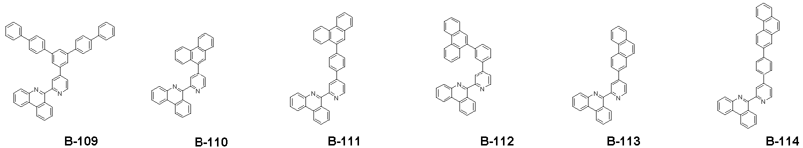

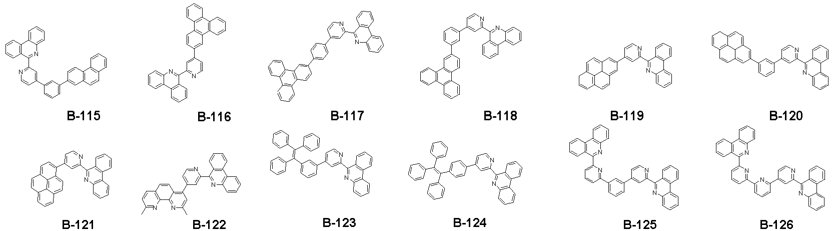

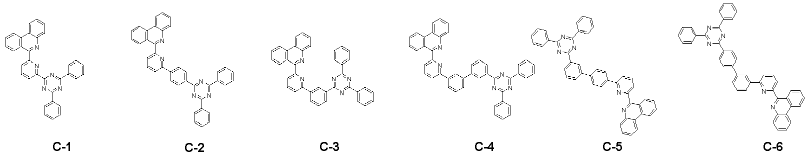

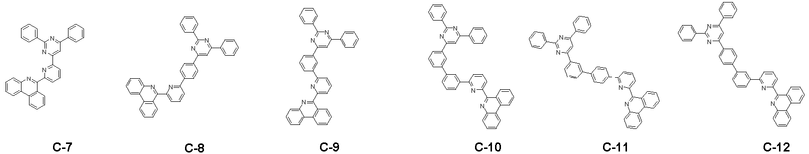

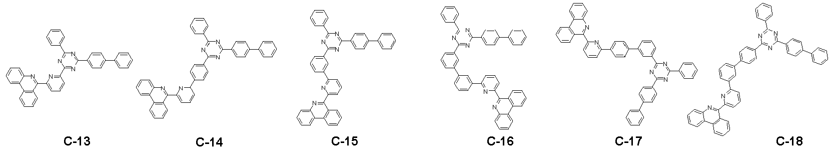

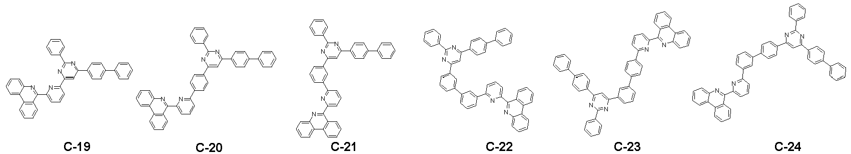

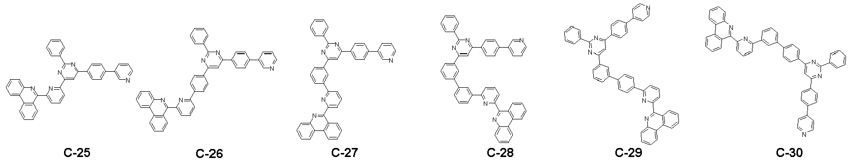

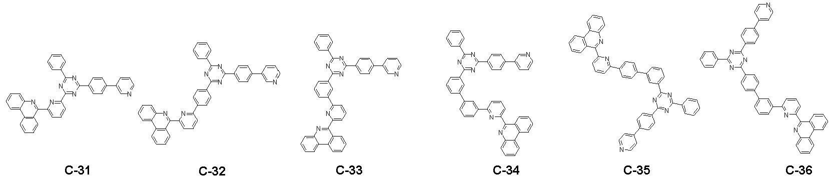

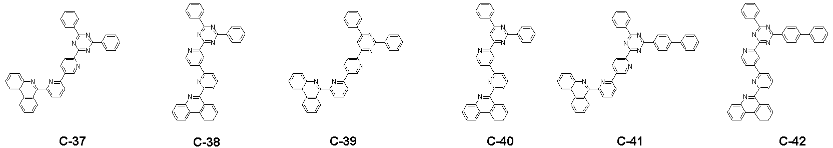

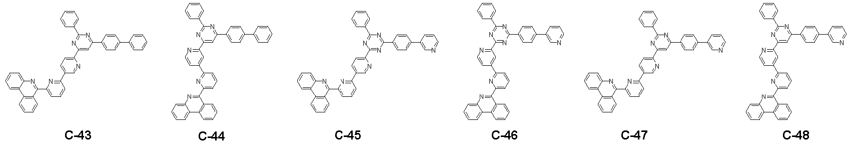

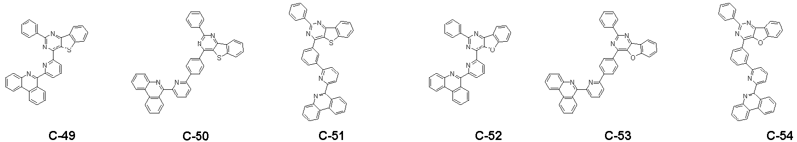

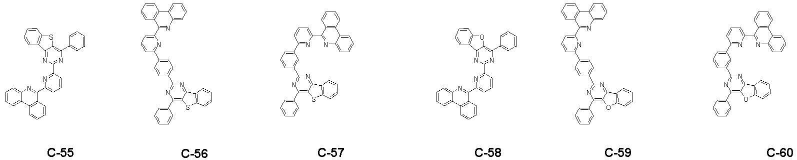

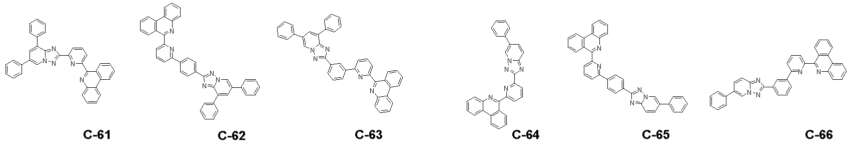

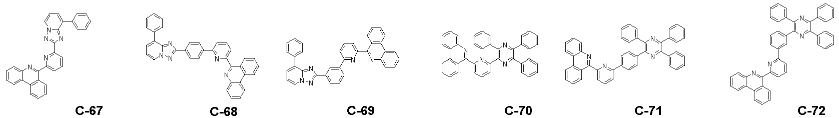

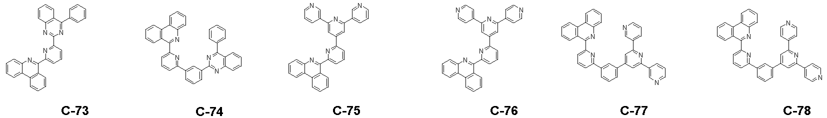

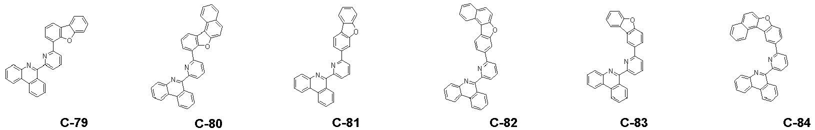

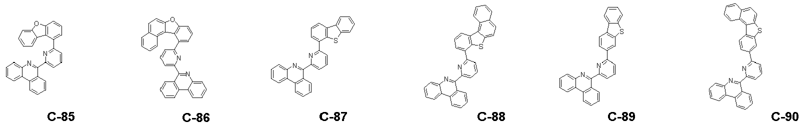

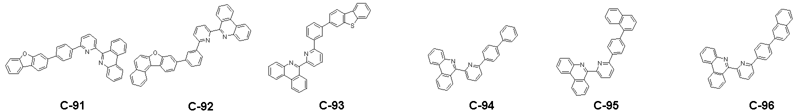

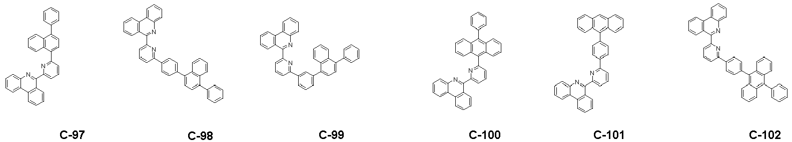

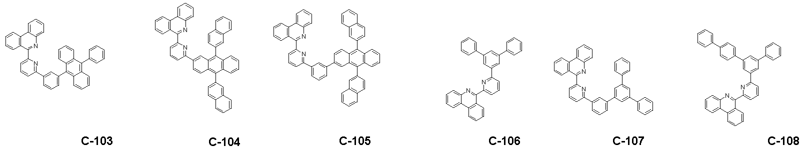

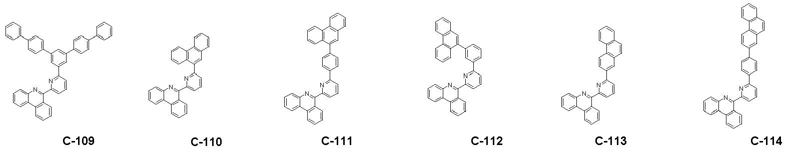

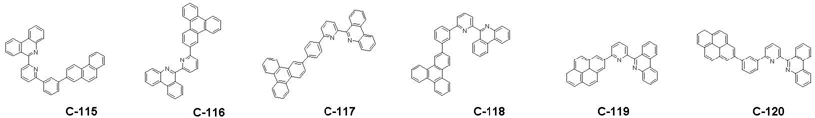

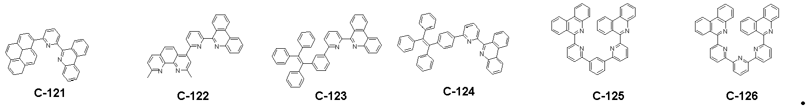

8. An organic electroluminescence device, comprising an anode; a cathode; one or more organic layer interposed between the anode and the cathode,
wherein at least one of the one or more organic layers comprises the organic compound of any one of claims 1 to 7.

9. The organic electroluminescence device of claim 8,
wherein the organic layer comprising the organic compound is an electron transport layer.

10. An organic electroluminescence device, comprising:
an anode and a cathode that are arranged at a distance;
a plurality of lighting units interposed between the anode and the cathode; and
a charge generation layer, interposed between the adjacent lighting units, comprising an N-type charge generation layer and a P-type charge generation layer
wherein each of the lighting units comprises a hole transport layer, a light-emitting layer, and an electron transport layer, and
the N-type charge generation layer comprises the organic compound of any one of claims 1 to 7.

11. The organic electroluminescence device of claim 10,
wherein the N-type charge generation layer comprises one host exhibiting an electron transport property, and the host is the organic compound of any one of claims 1 to 7.

12. The organic electroluminescence device of claim 11, wherein the N-type charge generation layer further comprises an N-type dopant.
